(19) Europäisches Patentamt / European Patent Office / Office européen des brevets

(11) **EP 4 129 318 A1**

(12) **EUROPEAN PATENT APPLICATION**
published in accordance with Art. 153(4) EPC

(43) Date of publication:
**08.02.2023 Bulletin 2023/06**

(21) Application number: **20904540.0**

(22) Date of filing: **23.11.2020**

(51) International Patent Classification (IPC):
*A61K 38/00* (1995.01)   *A61P 25/00* (2000.01)
*A61P 31/00* (2000.01)   *A61P 37/00* (2000.01)

(52) Cooperative Patent Classification (CPC):
**A61K 38/00; A61P 25/00; A61P 31/00; A61P 37/00**

(86) International application number:
**PCT/CU2020/050006**

(87) International publication number:
**WO 2021/129897 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2019 CU 20190113**

(71) Applicant: **Centro Nacional de Biopreparados
Bejucal 32600 (CU)**

(72) Inventors:
 • **RODRÍGUEZ MARTÍNEZ, Claudio
  Bejucal 32600 (CU)**
 • **GARCÍA HERNÁNDEZ, Yenela
  Quivicán 33500 (CU)**
 • **REYES ZAMORA, Mary Carmen
  Bejucal 32600 (CU)**
 • **ESQUIVEL CRESPO, Nashelly
  Cerro La Habana 10600 (CU)**
 • **VIERA ORAMAS, Diana Rosa
  Bejucal 32600 (CU)**
 • **GOVÍN RAVEIRO, Yanary
  La Salud Quivicán 33500 (CU)**
 • **RÁBAGO CÁPIRO, Rachel
  Marianao La Habana 11500 (CU)**
 • **ODELÍN VERDECIA, Mayelín
  Quivicán 33500 (CU)**
 • **PEREA MARTÍNEZ, Yoel
  Bejucal 32600 (CU)**

(74) Representative: **Capitán García, Maria Nuria
Felipe IV no. 10, bajo iz.
28014 Madrid (ES)**

Remarks:
The complete document including Reference Table(s) and the Sequence Listing(s) can be downloaded from the EPO website

(54) **PROTEIN-BASED PHARMACEUTICAL COMPOSITION WITH NEUROPROTECTIVE, IMMUNOMODULATING, ANTI-INFLAMMATORY AND ANTIMICROBIAL ACTIVITY**

(57)   The present invention consists of a pharmaceutical composition for therapeutic purposes based on proteins and their component peptides with a total protein concentration of 0.2 to 3 mg/mL or mg/g. The proteins and peptides are combined by selecting one or more belonging to at least two groups classified by their activity on the human organism, including antioxidant activity, activity on the ubiquitin-proteosome system, anti-inflammatory and immune system modulating activity, antimicrobial activity, and activity on protein transcription and synthesis. From the combination of proteins and peptides, a pharmaceutical composition is formed for intranasal, intramuscular, intravenous, oral or sublingual administration for the treatment of diseases of the central and peripheral nervous system, autoimmune and inflammatory, infectious, primary and secondary immunodeficiencies and cancer.

**Description**

**TECHNICAL SECTOR**

The present invention relates to the field of the formulation of pharmaceutical products for the therapy of diseases and disorders in humans and more specifically relates to pharmaceutical combinations and compositions for therapeutic use based on proteins or their component peptides to prevent or treat neurological, immunological, inflammatory and microbial disorders or diseases, among others.

**PRIOR ART**

Spanish patent application document ES2479815 (A1) discloses a neuroprotective peptide. International patent application WO2011051535 (A1) discloses bis(aralkyl)amino- and (hetero)aryl-derived chemical compounds for the treatment of cognitive disorders including Alzheimer's Disease (AD).

New applications of the 5-amino acid thymus peptide are claimed in Chinese patent application CN101693104 (A). With the peptide, it is achieved to significantly increase carbohydrate tolerance in animal model by maintaining glycometabolism, protecting islet cell structures and preventing diabetes.

International patent application WO 2010/011315 A2 describes a composition of thymus-derived peptides directed to the treatment of infectious, autoimmune, inflammatory diseases and includes the treatment of AD. The peptides are obtained by synthesis or by extraction and purification. The composition includes from 2 to 100 of other proteins of the thymic hormone family, although none are specified, nor is the use of any of them exemplified. The use of adjuvants is included. The sequences of the peptides described in the invention only encompass peptides closely related to each other, possessing only deletions, insertions or mutations in amino acid sequence. The effectiveness of the thymic peptides by themselves or in combination with other mentioned proteins and compounds is not sufficient to reverse the effects caused by AD, cognitive deficit and other neurodegenerative diseases, for this reason no concrete results of such an application are shown. For many diseases, as in the case of AD, not only the action of modulating the immune response or the anti-inflammatory effect is sufficient, but it is necessary to intervene in several metabolic pathways or physiological processes.

The same patent describes that in certain cases the composition is free of Cystatin A and histones. In other cases it is presented as a mixture of peptides with 2 to 100 other peptides from the thymus. The peptides can be obtained by synthesis or from natural sources. In addition, they are combined with adjuvants for oral, parenteral, subcutaneous, intravenous, intranasal, pulmonary, intramuscular and mucosal administration. The patent does not clearly describe any of the 2 to 100 peptides with which the thymus peptides of sequences 1 to 265 can be combined, so it is not obvious that they can be effective by themselves to combat all the diseases described in the claims, this is reinforced by the authors' statement that they include the combined use of antimicrobials, anticancer, among other compounds. The described mechanisms on which the composition and the invention acts only relate to the immune system and do not relate to lipid metabolism, antioxidant activity, beta amyloid protein aggregation and other mechanisms that relate to neurological diseases.

Thymosin beta 10 or its salt is claimed in the patent of the People's Republic of China CN1814276 as protective of the immune system and neuronal damage. It is known that the role of this hormone is related only to anti-inflammatory effect.

International patent application WO201303048226 A1 claims an immunomodulatory metallopeptide characterized because its formula is Xn-Asp-Gly-Pro-Lys-Phe-Leu-Xm, with n having a value from 0 to 4 and m from 0 to 2. The ions may be Zn, Cu and Mg or combinations thereof. The peptide is selected from: Leu-Glu-Asp-Gly-Pro-Lys-Phe-Leu, Glu-Asp-Gly-Pro-Lys-Phe-Leu, Asp-Gly-Pro-Lys-Phe-Leu, X-Leu-Glu-Asp-Gly-Pro- Lys-Phe-Leu, X-X- Leu-Glu-Asp-Gly-Pro-Lys-Phe-Leu, Leu-Glu- Asp-Gly-Pro-Lys-Phe-Leu-X, Leu-Glu-Asp-Gly-Pro-Lys-Phe-Leu-X-X and X-Leu-Glu-Asp-Gly-Pro-Lys-Phe-Leu-X. The peptide can be used as an anti-inflammatory agent or as an immunomodulatory or immunostimulatory agent, or to increase the number of T-lymphocytes in the blood, both helper (CD4+) and regulatory (CD4+ CD25+) in humans and animals. Also as an immunomodulator, to treat acute, chronic or relapsing infectious diseases, to treat fibrosis secondary to chronic inflammation, to treat hepatitis, cirrhosis, cancer and metastasis, plateletopenia or thrombocytopenia, and for the treatment of autoimmune or immune hypersensitivity diseases.

Japan patent application JPH06107553 (A) discloses a combination of mammalian thymus gland that causes an analgesic effect on various diseases. The extract obtained is combined with other medicinal plant extracts rich in vitamins B and E, acts on the immune system and is used to treat diseases such as colds, kidney and liver disorders.

Thymus-derived peptides with at least three amino acids derived from the thymic hormones Thymulin and Thymopoietin in the form of D, L or DL were conjugated with at least one compound selected from monocarboxylic acids, with alcohols, aldehydes or amide derivatives (US patent US6211155 B1) to achieve a pharmaceutical formulation.

Russian patent RU2017115294 describes an Acetyl-X-ARG-ARG-amide peptide where X can be - (D-ARG)-ARG-; -(D-LYS)-LYS- or -(D-MET)-MET with antidepressant activity and for the cure of AD. Peptides of this nature and molecular magnitude have not been sufficiently demonstrated to possess activities important for the neuroendocrine system and in general for many processes linked to aging, such as oxidative stress, anti-inflammatory, nor are they able to intervene in the mechanisms that regulate transcription, protein folding processes so important for the ubiquitin-proteosome system, vital to prevent or correct the formation of hyperphosphorylated tau protein that cause neurological disorders.

As can be deduced from the application of thymus peptides, their positive action on the immune system has been demonstrated, as an immunoregulator, with anti-inflammatory activity, protective against bacterial and viral infections. However, they alone do not intervene in a series of pathways and mechanisms present in complex diseases such as neurodegenerative diseases and especially AD.

Jacobsen *et al.* in 2005 demonstrated the results of the use of a novel nonapeptide in the activation of neurotrophins (Jacobsen J.S., Reinhart P., Pangalos MN. Current concepts in therapeutic strategies targeting cognitive decline and disease modification in Alzhheimer's disease. NeuroRx 2(4), 612-626 (2005)) and in US patent US9192654 B, the use of the nonapeptide to prevent or treat neurodegenerative diseases such as AD is described. Subcutaneous, intraperitoneal, intravenous or intranasal routes of administration are used. The sequence of the nonapeptide is EAKSQGGSD. The authors claim cognitive improvement in two experimental animal models based on coadministration of beta-amyloid peptide with the nonapeptide.

In the international patent WO2019018445 other authors describe the use of a polytherapy to modulate the expression of the Cathelicidin gene for the treatment of AD. The regulation of this gene to guarantee the action of Cathelicidin does not guarantee that all the causes that can trigger AD are controlled or eliminated. The most relevant role of this protein is the antibacterial activity.

The document describing the international invention application WO2019129315 (A1) shows a composition comprising an APL peptide with a phosphate buffer and at least one stabilizing sugar. The peptide is in a concentration between 0.5 mg/mL and 10 mg/mL and is directed to combat inflammatory diseases, including AD and hepatic or pulmonary fibrosis. It includes the use of cytosine agonists and other anti-inflammatory drugs. APL peptide has a field of activity limited to the immune system and more specifically to its anti-inflammatory activity. As is known inflammation is only one of the processes accompanying AD that occurs as a result of the body's response to the accumulation of beta-amyloid peptide, the presence of viruses or bacteria and has not been shown to be the original cause of the triggering of AD, although it does play a key role, especially in more advanced stages of the disease.

Inhibition of the binding mechanism of amyloid beta peptide to $\alpha$-7 nicotinic acetylcholine receptors is disclosed in US patent US7018797. This action inhibits pathological changes in cholinergic neurons and only has implication in the mechanisms associated with the impact of beta-amyloid peptide formation and not with the appearance of neurofibrillary tangles of hyperphosphorylated tau protein.

Russian patent RU2588143 C2 of 2017 discloses the composition of a Ra-R1-R2-R3-R3-R4-Rb or Ra-R4-R3-R2-R2-R1-Rb type peptide possessing in their sequence the amino acid combinations HADD, KADD, DDAK, RADD, DDAR, KAED, DEAK, RAED, DEAR, HADE, EDAH, KADE, EDAK, RADE, EDAR, HAEE, EEAH, KAEE, EEAK, RAEE and EEAR. These peptides bind to amyloid beta peptides to inhibit their polymerization and as an effect combat neurodegenerative diseases, in particular AD. As with the previous invention, the fundamental therapeutic target is the formation of amyloid beta peptide polymers.

Other peptides with sequences YEKLLDTEI, LDTEI, especially LDTEL, LDTEV, LDTDI, LDTDL, LDTDV, LDSEI, LDSEL, LDSEV, LDSDI, LDSDL, LDSDV, LETEI, LETEL, LETEV, LETDI, LE TDL, LETDV, VDTEI, VDTEL, VDTEV, VDTDI, VDTDL, VDTDV, IDTEI, IDTEL, IDTEV, ID TDI, IDTDL, IDTDV, IETEI, IETEL, IETEV, IETDI, IETDL, IETDV; YGRKKRRQRRR and YGRKKKRRRRQRRRYEKLLDTEI are employed according to People's Republic of China patent application CN109718363 (A).

Combination therapies for the treatment of AD have also been the subject of patents, as is the case with US patent application US2015283113 (A1). It describes the combination of compounds that inhibit beta-amyloid peptide aggregation, compounds that improve cognitive function, mood or social behavior. The anti-inflammatory compound is combined with others associated with Tau protein or alpha-synuclein. The beta-amyloid aggregation inhibitor is a metal ionophore, a statin or an andocanabinoid. The anti-inflammatory compound may consist of chromoline compounds, a chromoline derivative, or its analog, eugenol, nedocromil, pemirolast, olopatadine, alphatoxin G1 alphatoxin B1, alphatoxin M1 deoxynivalenol, zearalenone, ochratoxin A, fumonisin B1 or its hydrolyzed form or patulin, ergotamine and others such as indomethacin, droxicam, isoxicam. Not all the mechanisms involved in diseases of the immune and neuroendocrine systems are subject to activity by the compounds described.

Analyzing the set of documents cited, it can be summarized that peptides and proteins of different sizes and amino acid sequences have been used for the treatment of diseases and disorders linked to the immune, neuronal, endocrine and defense systems against microorganisms. Effective therapeutic combinations against these diseases are scarce and, at most, they are based on the combination of proteins and peptides with compounds of a nature different from peptides and with activity on one or two of the causes of the disease. The combination of proteins and peptides acting on different metabolic pathways or systems is more rarely described.

As an example, none of the combinations and compositions described is capable by itself of acting on all the mechanisms to which the triggering of neurological diseases is attributed and more specifically on AD, more specifically with simultaneous activity on oxidative stress, transcription, the correct synthesis of proteins involved in the ubiquitin-proteosome system, the synthesis, folding and elongation of other proteins, essential for the elimination of hyperphosphorylated Tau protein and the non-accumulation of beta-amyloid peptide polymers; with action on the immune system, specifically with regulation of the CD4+ and CD8+ cell population, on inflammation, on the defense system against infections by fungi, bacteria and viruses and therefore the regulation of the intestinal microbiome, and on lipid metabolism, which helps to eliminate or reduce insulin resistance with the consequent loss of the capacity to assimilate carbohydrates.

In order to provide a solution to the limitations of the existing inventions so far, the objective of achieving a pharmaceutical composition based on the combination of proteins with a molecular weight lower than 50 000 Da, preferably lower than 20 000 Da with different therapeutic actions on several metabolic pathways and physiological mechanisms that will make it possible to correct and restore the natural functions of the organism, especially for those complex pathologies that involve different therapeutic targets.

According to the present invention, a new and original pharmaceutical composition based on the combination of proteins for therapeutic purposes is provided, which is characterized by containing proteins and their component peptides which have different activities among them, antioxidant activity, activity on the ubiquitin-proteosome system, anti-polymerization of beta amyloid, with anti-inflammatory and immune system modulating activity; and includes proteins and peptides with antimicrobial activity and activity on lipid metabolism.

The peptides and proteins of the present invention belong to at least two of the above mentioned groups of proteins with different activities in proportions that guarantee an adequate effectiveness on the different systems. The selected proteins and peptides that make up the combination are originally mixed with other compounds that serve as vehicles and adjuvants for their better administration by intranasal, intramuscular, parenteral or oral route in effective doses and frequency for the treatment of neurological diseases and disorders such as AD, Parkinson, epilepsy; of the immune system, such as rheumatoid and reactive arthritis; acute or recurrent respiratory diseases, acute infectious diseases and in primary and secondary immunodeficiencies, among others.

The pharmaceutical combination, based on the mixture of molecules of protein origin described in the invention are used as a complement to therapies with other proteins such as calmodulin, erythropoietin, histones, DNA replication factors, keratin, syntaxin, granulocyte colony stimulating factor, forming an original pharmaceutical composition for the treatment of neurodegenerative diseases, cancer, HIV-AIDS, autoimmune, viral, among others.

The term "for therapeutic purposes" in the present invention includes prevention of disease, reduction of symptoms, attenuation of symptoms, reduction of the severity of symptoms or of the disease itself, arrest of disease progression, reversal of disease severity, avoidance of relapses, total eradication of symptoms or cure of disease.

The term pharmaceutical combination in the present invention refers to the mixture of proteins or their component peptides which is employed for therapeutic purposes.

The term pharmaceutical composition in the present invention refers to the conjunction of the pharmaceutical combination described in the preceding paragraph with other proteins and/or vehicles for administration to humans by various routes for therapeutic purposes.

The term infection or infectious diseases refers, for the purposes of the present invention to diseases or to the presence in the human body of cells, the whole organism, metabolites or parts of microorganisms such as bacteria, fungi, viruses and parasites.

The evidence and substantiation of the therapeutic actions described in the paragraph of the present invention describing the concept "for therapeutic purposes" is demonstrated in *in vivo* animal models, in *in vitro* experiments in cell culture and in the treatment of subjects and patients.

## DETAILED DESCRIPTION OF THE INVENTION

According to the present invention a pharmaceutical combination of a protein nature is formed based on proteins and peptides with a molecular weight lower than 50 000 Da, preferably equal to or lower than 20 000 Da which in certain embodiments are extracted or semi-purified, purified or isolated, by appropriate chromatographic purification methods, or obtained with other separation or purification techniques such as ultrafiltration, precipitation with salts, diafiltration, centrifugation. In other embodiments the pharmaceutical combination is formed from total, semi-purified or purified extracts containing the selected proteins. It can be made from mammalian organs and tissues, preferably, but not limited to bovine. The organs and tissues are preferably, but not limited to thymus, brain, spleen and guts.

In other embodiments, the pharmaceutical combination is obtained from purified protein derivatives, products of mammalian organ harvesting or from polypeptides obtained by chemical synthesis. In other embodiments, the pharmaceutical combination is formed from proteins obtained by recombinant routes in bacteria and yeasts, more specifically in *E. coli* or in *Saccharomyces cerevisae.*

Finally, in certain embodiments, the pharmaceutical combination is obtained from combinations of proteins and their component peptides obtained by the combination of two or more of the above-mentioned methods.

To form the pharmaceutical combination that forms part of the pharmaceutical composition, the proteins or their component peptides are mixed by selecting them from the following groups:

**a)** One or more proteins or peptides with antioxidant activity selected from among Thioredoxin or its component peptides containing in their molecule the sequences EKLEAT (SEC ID NO 1), YAFQEALNSAGEK (SEC ID NO 2), MIKPFFHSLSEK (SEC ID NO 3); Cytochrome C or its component peptides containing in their molecule the sequences EDLIAYLK (SEC ID NO 4), TGPNLHGLFGR (SEC ID NO 5), TGQAPGFSYTDAN (SEC ID NO 6) and GITWGEETLMEYLENPK (SEC ID NO 7) and NDUFA4 subunit of Cytochrome C oxidase or its component peptides containing in their molecule the sequences FIGAGGTGAALY (SEC ID NO 8) and VNVDYSKLKKKEGP (SEC ID NO 9);

**b)** One or more proteins or peptides with activity on the ubiquitin-proteosome system selected from Ubiquitin, Ubiquitin-derived proteins with arginine or leucine deletions at the N- or C-termini or methylated, or their component peptides containing in their molecule the sequences MQIFVKTLTG (SEC ID NO 10), KTITLEVEPS (SEC ID NO 11), DTIENVKAKI (SEC ID NO 12), QDKEGIPPDQ (SEC ID NO 13), QRLIFAGKQL (SEC ID NO 14), EDGRTLSDYN (SEC ID NO 15) and IQKESTLHLVLR (SEC ID NO 16); small Ubiquitin-fold modifier Ufm1 or its component peptides containing in their molecule the sequences MSKVSFKKITL (SEC ID NO 17), AVLKFAAEEF (SEC ID NO 18); small Ubiquitin-fold modifier Ufm2 or its component peptides containing in their molecule the sequences VAGQDGSVVVQFK (SEC ID NO 19) and MADEKPKKEGVK (SEC ID NO 20); Peptidyl-prolyl-cis-trans isomerase H or its component peptides containing in their molecule the sequences GVQVETISPGDGR (SEC ID NO 21); GWEEGVAQMSVGQR (SEC ID NO 22); Cystatin B or its component peptides containing in their molecule the sequences VQVDEDDFVHIR (SEC ID NO 23) and VFESLPHENKPVALTSYQT (SEC ID NO 24); P47 NSFL1 cofactor or its component peptides containing in their molecule the sequences EFVAVVTGAEEDR (SEC ID NO 25) and SYQDPSNAQFLESIR (SEC ID NO 26) and Homolog B of the UV-excision repair protein RAD23 or its component peptides containing in their molecule the sequences IDIDPDETVR (SEC ID NO 27) and NFVVVMVTKPK (SEC ID NO 28);

**c)** One or more proteins or peptides with anti-inflammatory and immune modulating activity selected among:

(i) proteins belonging to the Beta Thymosins family or their component peptides containing in their molecule 3 sequences of 4 amino acids: DKPD (SEC ID NO 29), LKKT (SEC ID NO 30) and KETI (SEC ID NO 31).

(ii) proteins belonging to the alpha-thymosins family or their component peptides containing in their molecule two sequences of 7 to 14 amino acids: SDAAVDTSSEITTK (SEC ID NO 32) and VVEZAEN (SEC ID NO 33)

(iii) proteins belonging to the thymopoietin family or their component peptides containing in their molecule three 13 amino acid sequences: FLEDPSVLTKEKL (SEC ID NO 34), KSELVANNVTLPA (SEC ID NO 35), GEQRKDVYVELYL (SEC ID NO 36)

(iv) other thymic proteins containing the sequences RKNVW (SEC ID NO 37), RKDVY (SEC ID NO 38), EAKSQGSN (SEC ID NO 39), EASQGGSD (SEC ID NO 40), PYR-AKSQGGN (SEC ID NO 41);

**d)** One or more proteins or peptides with antimicrobial activity, selected from among the Cathelicidin-4 precursor or its component peptides containing in their molecule the sequences LLELDPPPK (SEC ID NO 42), AVDQLNELSSEANLYR (SEC ID NO 43) and LLELDPPPKDNEDLGTR (SEC ID NO 44) and Cathelicidin-1 and its component peptides containing in their molecule the sequences AVDQLNEQSSEPNIYR (SEC ID NO 45) and LLELDQPPQDDDEDPDSPK (SEC ID NO 46);

**e)** A protein with activity on lipid metabolism, specifically ATOX1 Copper Transporter Protein and its component peptides containing in its molecule the sequences MPKHEFSVDM (SEC ID NO 47) and FDIDLPNKKV (SEC ID NO 48);

**f)** One or more component proteins or peptides with activity on protein synthesis and transcription, selected among Transcription elongation factor A protein 1 (TCEA1) or its component peptides containing in their molecule the sequence NIPMTLELLQSTR (SEC ID NO 49) and Serine/arginine-rich splicing factor 2 or its component peptides containing in their molecule the sequences DAEDAMDAMDGAVLDGR (SEC ID NO 50) and SYGRPPPDVEGMTSLK (SEC ID NO 51).

For the purposes of the present invention, in addition to the peptides with the sequences indicated in groups **a** to **f,** the following are defined as peptides composing the aforementioned proteins:

- derivatives originating from chemical modifications in the amino, hydroxyl and methyl end groups, or derivatives of the side groups.

- derivatives from acetylation, phosphorylation, glycosylation, amidation and derivatization
- derivatives from proteolytic enzymatic action including, but not limited to, those obtained under the action of trypsin, chymotrypsin, pepsin, papain, bromelain or from acid or alkaline hydrolysis of proteins
- derivatives originating from the substitution of L-amino acids by D-amino acids
- conjugated to other molecules or cell ligands or monoclonal antibodies
- chimeric proteins fused from the combination of the proteins and their peptides described in groups **a** to **f** with other proteins or fragments obtained in certain cases by recombinant way in bacteria or yeast or in other cases by chemical synthesis.

For the conformation of the pharmaceutical combination, combinations of proteins of group b are preferably chosen, but not limited to, most preferably Ubiquitin, Ubiquitin-derived proteins with arginine or leucine deletions at the N- or C-termini, or its component peptides containing in their molecule the sequences MQIFVKTLTG (SEC ID NO 10), KTITLEVEPS (SEC ID NO 11), DTIENVKAKI (SEC ID NO 12), QDKEGIPPDQ (SEC ID NO 13), QRLIFAGKQL (SEC ID NO 14), EDGRTLSDYN (SEC ID NO 15) and IQKESTLHLVLR (SEC ID NO 16) with other proteins belonging to other groups such as **a, c, d, e,** and **f.**

In other embodiments, the proteins and peptides mentioned in the preceding paragraph from group b are combined with each other and with other proteins from the rest of groups **a, c, d, e** and **f.**

In certain embodiments the proteins or peptides of group **b** are combined with those of group **c,** preferably those described in subgroup i belonging to the Beta Thymosins family or its component peptides which contain in their molecule 6 sequences of 4 amino acids: DKPD (SEC ID NO 29), LKKT (SEC ID NO 30) and KETI (SEC ID NO 31). In other embodiments, the proteins of group **b** are combined with those described in subgroup ii proteins belonging to the alpha-thymosin family or its component peptides that have 2 sequences of 7 to 14 amino acids in their molecule: SDAAVDTSSEITTK (SEC ID NO 32) and VVEZAEN (SEC ID NO 33).

In other embodiments the proteins or peptides of group **b** are combined with the two aforementioned subgroups i and ii. In still other embodiments, proteins and peptides of group **b** are combined with proteins and peptides of subgroups iii and iv. In still other embodiments the proteins or peptides of group b are combined with proteins or peptides selected from subgroups i, ii, iii and iv. In other embodiments, proteins or peptides from groups **b** and **c** are combined with proteins or peptides from group **a,** preferably Thioredoxin or its component peptides containing in their molecule the sequences EKLEAT (SEC ID NO 1), YAFQEALNSAGEK (SEC ID NO 2), MIKPFFHSLSEK (SEC ID NO 3); Cytochrome C or its component peptides containing in their molecule the sequences EDLIAYLK (SEC ID NO 4), TGPNLHGLFGR (SEC ID NO 5), TGQAPGFSYTDAN (SEC ID NO 6) and GITWGEETLMEYLENPK (SEC ID NO 7).

In certain embodiments, the proteins or peptides of groups a, b and c are combined with the proteins or peptides of group **d:** Cathelicidin-4 precursor or their component peptides containing in their molecule the sequences LLELDPPPK (SEC ID NO 42), AVDQLNELSSEANLYR (SEC ID NO 43) and LLELDPPPKDNEDLGTR (SEC ID NO 44) and Cathelicidin-1 and its component peptides containing in their molecule the sequences AVDQLNEQSSEPNIYR (SEC ID NO 45) and LLELDQPPQDDDEDPDSPK (SEC ID NO 46). In other embodiments, the proteins or peptides of groups **a, b, c** and **d** are combined with the proteins or peptides of group **e:** copper transporter protein ATOX 1 and their component peptides containing in their molecule the sequences MPKHEFSVDM (SEC ID NO 47) and FDIDLPNKKKV (SEC ID NO 48).

In certain embodiments, proteins from groups **a, b, c, d** and **e** are combined with its component protein or peptides from group f: Transcription elongation factor A protein 1 (TCEA1), preferably its component peptides containing in their molecule the sequence NIPMTLELLQSTR (SEC ID NO 49) and Serine/arginine-rich splicing factor 2, preferably its component peptides containing in their molecule the sequences DAEDAMDAMDGAVLDGR (SEC ID NO 50) and SYGRPPPDVEGMTSLK (SEC ID NO 51).

To make up the pharmaceutical composition, the selected proteins or peptides in the pharmaceutical combination are in concentration of 0.2 to 3 mg/g or 0.2 to 3 mg/mL with respect to the total mass or volume of said pharmaceutical composition.

In the pharmaceutical composition, the proteins or peptides of groups **b** and **c** described above as part of the pharmaceutical combination are in individual concentrations of 0.02 to 2.7 mg/mL or 0.02 to 2.7 mg/g with respect to the total volume or mass of said composition (in individual concentrations of 10 % to 90 % with respect to the total of all proteins in the composition), preferably from 0.08 to 2.4 mg/mL or from 0.08 to 2.4 mg/mg, more preferably from 0.04 to 0.9 mg/mL or from 0.04 to 0.9 mg/mg with respect to the total volume or mass of the pharmaceutical composition (preferably between 40 % and 80 %, more preferably between 20 % and 30 %). The total protein content of the combination in the pharmaceutical composition is always maintained in the range from 0.2 mg/g to 3 mg/g or from 0.2 mg/mL to 3 mg/mL.

In the pharmaceutical composition of the present invention, the individual proteins or peptides selected from groups **a, d, e** and **f** making up the pharmaceutical combination are in the range from 0.000002 to 0.3 mg/mL or from 0.000002 to 0.3 mg/mg with respect to the total volume or mass of the pharmaceutical composition (from 0, 001 % to 10 % with respect to the total of all proteins in the composition), preferably from 0.000002 to 0.15 mg/mL or from 0.000002 to 0.15 mg/mg (preferably from 0.001 % to 5 %) and more preferably from 0.000002 to 0.015 mg/mL or from 0.000002 to 0.015 mg/mg (from 0.001 % to 0.5 %).

It should be clarified for the purposes of the present invention and in order to make more understandable the description of the concentrations in which each protein or peptide is found in the combination, that some concentrations are identified in each example with an asterisk (*), whose actual content is the difference between the total mg of all the proteins and the weight in mg of the rest of the proteins per mL.

For certain purposes, e.g. for the treatment of neurodegenerative diseases and primarily AD, stroke, Parkinson's disease, cancer and HIV, the combination of the proteins and their component peptides selected from the groups **a** to **f**, are combined with other proteins, such as Calmodulin, Erythropoietin, histones, DNA replication factors, Keratin, Syntaxin, Granulocyte Colony Stimulating Factor (GCSF). Said proteins are combined in such a way that the total protein content in the pharmaceutical composition remains in the range of 0.2 to 3 mg/mL or 0.2 to 3 mg/g. For this purpose, the final content of the proteins or peptides to be added or combined are in amounts of 0.001 to 1.8 mg/mL or 0.001 to 1.8 mg/g with respect to the total volume or mass of the pharmaceutical composition (not exceeding 60% with respect to the total proteins belonging to groups **a** to **f**). Particularly Calmodulin, Erythropoietin, histones, DNA replication factors, Keratin, Syntaxin, Granulocyte Colony Stimulating Factor (GCSF) individually are in concentration from 0.00001 to 1.5 mg/mL or from 0.00001 to 1.5 mg/g (not exceeding 5%), preferably from 0.000017 to 1.5 mg/mL or from 0.000017 to 1.5 mg/g with respect to the total proteins belonging to groups **a** to **f** (not exceeding 0.5%), more preferably from 0.000034 to 0.85 mg/mL or from 0.000034 to 0.85 mg/g (not exceeding 0.005%).

In other embodiments selected proteins or peptides from groups **a** to **f** are combined with other selected proteins containing in their molecule the amino acid sequences MAGSSFLSP (SEC. ID NO. 52) and LEIRFNAPF (SEC. ID NO. 53) preferably Ghrelin, obtained from the intestine or by synthesis. In such cases these proteins are combined with those of groups **a** to **f** at concentrations of 0.00001 to 1.5 mg/mL or mg/g with respect to the total proteins in the combination (not exceeding 10%), more preferably 0.000017 to 0.3 mg/mL or mg/g (not exceeding 1%).

In cases where the extraction method is employed from sources of natural origin, for example and preferably from mammalian organs, preferably brain, thymus, spleen, a 0.9% concentration NaCl solution will be used as a first choice as primary solvent, to obtain a final extract that maintains the total protein content of the pharmaceutical composition from 0.2 mg/mL to 3 mg/mL.

In certain embodiments in which proteins or peptides obtained synthetically, by recombination techniques or by purification methods are used, the proteins are combined by adding them to a solvent solution selected from 0.9% NaCl or monobasic potassium phosphate and dibasic potassium phosphate buffer solution at a concentration of 0.9%, to a concentration of 0.2 mg/mL to 3.0 mg/mL. The pharmaceutical composition thus formulated is used for intranasal, intramuscular or sublingual administration.

In certain embodiments for intranasal administration, other substances may be employed separately or in combination, for mixing with those mentioned in the preceding paragraph, such as polyvinyl butyl ether or polyvinyl alcohol in concentrations between 1% and 50%, preferably from 1% to 30%, more preferably from 10% to 20%, always ensuring that the final protein concentration is maintained in the range of 0.2 mg/mL to 3 mg/mL. In other embodiments for intranasal administration, other substances can be used separately or in combination with the NaCl or phosphate solutions, such as chitosan, agarose, pectin, sodium carrageenan, cyclodextrin, sodium alginate and Carbacol, always ensuring that the final protein concentration is maintained in the range of 0.2 mg/g to 3 mg/g.

In certain embodiments, the proteins or peptides of the pharmaceutical combination, regardless of the source of procurement, are combined only with polyvinyl butyl ether or polyvinyl alcohol in a ratio of 0.2 mg to 3 mg protein/mL for intranasal, oral or sublingual administration. In other embodiments, the pharmaceutical combination of the present invention, after solubilization in a solvent, preferably 0.9% NaCl solution, 0.9% phosphate buffer or distilled or deionized water, is subjected to drying, preferably by lyophilization or by spray drying to a moisture content of less than 7% and forms a pharmaceutical composition with a total protein content of 0.2 mg/g to 3 mg/g with respect to the mass of the total pharmaceutical composition. The combination of proteins or peptides with low moisture content is preferably employed for oral or sublingual administration and may be employed in conjunction with other vehicles, for example, but not limited to starches, preferably corn and more preferably pregelatinized corn, cellulose, carboxymethylcellulose, microcrystalline cellulose, methylcellulose, silicon dioxide, silica, sodium glycolate, lactose, mannitol, xylitol, polydextrose, hydrogenated vegetable oil, distilled glyceryl monostearate, glyceryl palmitate stearate.

In other embodiments, for oral or sublingual administration, the pharmaceutical combination is emulsified with oils selected from oils obtained from Plukenetia volubilis, arachis, soybean, castor, sesame, preferably Plukenetia volubilis. The emulsion, preferably is of the oil-in-water type and is formed by employing agitation at speeds between 500 and 10 000 rpm, or by applying high pressures, ultrasound, sonication, or combination of various methods to obtain droplet sizes from 5 nm up to 200 nm, more preferably from 50 nm to 200 nm and more preferably from 50 to 100 nm.

The pharmaceutical combination for intramuscular administration is combined in certain embodiments with one of the adjuvants selected from aluminum sulfate or aluminum hydroxide, with a maximum aluminum concentration of 0.3 mg/dose to 1.5 mg/dose. In other embodiments, for intravenous administration, the pharmaceutical combination is combined with 0.9% NaCl solution.

The pharmaceutical composition for therapeutic purposes of the present invention based on proteins and peptides is applied in the therapy of diseases of the central and peripheral nervous system selected from a group consisting of AD, Parkinson's disease, Leigh syndrome, epilepsy, cerebral ischemia, cranial trauma, multiple sclerosis, amyotrophic lateral sclerosis, Wilson's disease and Menkes' disease, retrobulbar neuritis, encephalopathies, polyneuropathies.

The pharmaceutical composition is also applied in the therapy of diseases with autoimmune and inflammatory component, selected from a group including systemic lupus erythematosus, rheumatoid arthritis, juvenile idiopathic arthritis, reactive arthritis, fibromyalgia, ankylopoietic spondylitis, generalized osteoarthrosis, rheumatic fever, myasthenia gravis, sarcoidosis, Sjogren's syndrome, Behcet's disease, anterior uveitis, psoriasis, dermatitis, chronic urticaria, colitis, Crohn's disease, irritable bowel syndrome.

The pharmaceutical composition of the invention is employed in adjuvant therapy in acute, chronic or recurrent infectious diseases, selected from a group including septic shock, HIV, Hepatitis B, Hepatitis C, systemic or deep fungal infections, respiratory or recurrent infections, recurrent herpetic or mucocutaneous herpetic or fungal infection.

The pharmaceutical composition is used, by itself, or in combination with other drugs in therapy as an immunomodulator in states of primary or secondary immunodeficiencies such as aplasia, decreased function and size of the thymus, severe combined immunodeficiency, common variable immunodeficiency, IgA deficiency, ataxia-telangectasia, Wiskott-Aldrich syndrome, immunodeficiencies secondary to infections, immunosenescence.

The invention has application as an immunomodulator in the treatment of immune or inflammatory diseases, rhinosinusitis and atopic dermatitis. The use of the present pharmaceutical composition extends to the treatment as adjunctive therapy of cancer.

Intranasal administration in certain embodiments is executed by administering one or more doses of 0.1 mg to 3 mg of the pharmaceutical combination, in volumes of 0.5 mL to 1 mL to form the pharmaceutical composition, applying it through the nostril, preferably 0.2 mL to 0.3 mL of the pharmaceutical composition, with a few minutes between doses; with frequencies of 1, 2, 3 times per week, on alternate days or daily for a period of between 4 weeks to 1 year. In other embodiments, the intramuscular administration is in doses of 1.5 mg to 15 mg of the pharmaceutical combination, as part of the pharmaceutical composition at frequencies of 1, 2, 3 times per week, every other day or daily, in one or more cycles with rest periods between cycles of 4 to 24 weeks for a period of duration of each cycle of 4 to 12 weeks.

In other embodiments, for intravenous administration, doses of 3 mg to 80 mg of the pharmaceutical combination diluted in 0.9% NaCl solution (pharmaceutical composition) are applied at frequencies of 1, 2, 3 times per week, every other day or daily for a period of 4 weeks to 1 year. In other embodiments, for oral administration the pharmaceutical combination is supplied in doses of 60 mg to 120 mg with daily frequency, or every other day for 4 weeks to 1 year. Finally, in other embodiments sublingual administration of the pharmaceutical combination is executed with doses of 0.5 mg to 3 mg in volumes of 0.1 mL to 0.5 mL in the pharmaceutical composition at a frequency of twice daily, daily or every other day for a period of 6 months to 1 year.

The proteins included in the pharmaceutical composition and their role in the pharmaceutical composition are listed below, which allows a better understanding of the essence of the invention and its advantages over the prior art.

Thioredoxin (Trx)

Protein oxidation processes essentially lead to the appearance of damage at different levels and to the appearance of immunological, neuronal and cancer diseases, among others.

Trx is a small protein with antioxidant enzymatic activity (oxireductase), that is, it responds to reactive oxygen species by facilitating the reduction of other proteins by exchanging dithiol-disulfide residues. Very relevant substrates have been identified such as insulin, glucocorticoid receptor, ribonucleases of great significance in diseases of different nature, such as AD. Trx plays a very important role in cell-cell communication.

Thioredoxin 1 (Trx1) as a protein with antioxidant activity protects neurons from the effect of oxidative stress. The level of this protein in AD patients is reduced in their brain. The presence of ApoE4 causes the reduction in Trx1 levels, *in vivo* in the hippocampus of mice genetically modified for this gene, and *in vitro* in primary cortical neurons of humans and in neuroblastoma cells in the presence of Cathepsin D. Trx1 is truncated by an $\alpha$-secretase originating the peptide Thioredoxin-80 which is present in neurons and its level is found to be reduced in AD patients and inhibits A$\beta$-peptide aggregation (Persson P. Thioredoxin-1 in Alzheimer disease. Thesis. 2015-11-27. Accessed 21.09.2019 at https://openarchive.ki.se/xmlui/handle/10616/44887).

There is strong evidence for the role of oxidative stress in AD pathogenesis and neuronal apoptosis. To investigate the protective effect of Trx1 on A$\beta$-peptide induced toxicity, hippocampal cells were cultured and treated with Trx and Thioredoxin reductase (TR). Trx was shown to possess a protective role against cytotoxicity mediated by reactive oxygen species (ROS) generation. We also demonstrated depressed levels of Trx in AD patients and concluded that these depressed levels may contribute to the oxidative stress and consequent neurodegeneration observed in the brain of AD patients (Lovell MA, Xie C, Gabbita SP, Markesbery WR. Decreased thioredoxin and increased thioredoxin reductase levels in Alzheimer's disease brain. Free Radic Biol Med. 2000 Feb 1;28(3):418-27). Other authors demonstrate the protective role of Trx against oxidative stress damage in the brain (Akterin S., Cowburn R. F., Miranda-Vizuete A., Jimenez A., Bogdanovic N., Winblad B. and Cedazo-Minguez A. Involvement of glutaredoxin-1 and thioredoxin-1 in β-amyloid toxicity and Alzheimer's disease. Cell Death & Differentiation volume 13, pages1454-1465 (2006)).

Therefore, oxidative stress in general and Trx1 in particular play a key role in the pathogenesis of AD. By including it in the pharmaceutical composition of the present invention, it is possible to achieve its interaction with the substrate insulin which, as is known in AD patients, a resistance to this substrate and the non-assimilation of carbohydrates is observed. In addition, it favors the interaction between neurons, exerts a role as an antioxidant and prevents the aggregation of Aβ-peptide in the brain. The present paper describing the new invention shows evidence of the ultimate effect in decreasing oxidative stress *in vitro* and *in vivo.* The therapeutic application extends to other pathologies caused by oxidative stress in different systems.

Other oxidative processes that occur in diseases such as cancer and autoimmune diseases are prevented or at least mitigated by increasing the concentration and activity of Trx1.

Cytochrome C

Cytochrome C is a small hemoprotein associated with the inner membrane in mitochondria and is essential for ion transport as it transports an electron. It can be oxidized or reduced by the passage of iron from its ferrous to its ferric form and vice versa without the need to bind oxygen.

In patients with neurological diseases, for example with AD, Aβ peptide accumulates in mitochondria causing their dysfunction. Hernandez-Zimbron *et al.* in 2019 demonstrated that Aβ peptide 1-42 binds to a peptide comprising the amino-terminal region of Cytochrome oxidase subunit 1 and may explain, in part, the decreased enzymatic activity in the respiratory chain of complex IV and the consequent metabolic dysfunction observed in AD (Hernandez-Zimbron LF, Luna-Muñoz J, Mena R, Vazquez-Ramirez R, Kubli-Garfias C, et al. (2012) Amyloid-b Peptide Binds to Cytochrome C Oxidase Subunit 1. PLoS ONE 7(8): e42344. Accessed 19.09.2019 at https://www.ncbi.nlm.nih.gov/pmc/articles/PMC3424232/).

Being part of the pharmaceutical composition of the present invention, Cytochrome C by itself or in combination with the other proteins with antioxidant effect, protects cells and tissues in general from damage by oxidative stress, whose effect is demonstrated *in vitro* and *in vivo* in the running examples and results in cognitive enhancement and immune response.

NDUFA4 Subunit of Cytochrome C oxidase

The NDUFA4 subunit of Cytochrome C oxidase is considered a subunit of the NADH Complex 1 dehydrogenase enzyme that possesses redox centers and the NADH binding site. It possesses hydrophobic and hydrophilic domains that are suggested to be critical for protein metabolism and its hydrophobic domain functions as an anchor for the NADH:ubiquinone oxireductase complex within the mitochondrial membrane. Its low levels are associated with deficiency of Cytochrome oxidase C. This protein is involved in stabilizing the membrane-matrix linkage arms of Complex 1 which, when deregulated and decreased, characterizes the advanced stage of AD (Adav S.S., Park J.E., Sze S.K. Quantitative profiling brain proteomes revealed mitochondrial dysfunction in Alzheimer's disease. Molecular Brain. volume 12, Article number: 8 (2019)). Mutations in the gene encoding the protein lead to Leigh syndrome and other neurological disorders.

In the new pharmaceutical composition proposed in the present invention NDUFA4 Subunit of Cytochrome C oxidase plays a complementary role to the antioxidant effect and its mechanism of action involves a positive intervention in the NADH:ubiquinone oxireductase complex that ultimately results in the Ubiquitin-proteosome system responsible for the elimination of hyperphosphorylated Tau protein from the interior of neurons with implications in neurological disorders. Never before have these proteins been combined for the treatment of neurological, immune system and other diseases included in the scope of the invention.

Ubiquitin

Free radicals generated by pro-oxidant compounds damage the macromolecules of the human organism, especially proteins and other components of its cellular structure. Numerous diseases have their origin in oxidative processes, and in aging it is an irreversible and common phenomenon. The human organism relies on a complex system called Proteosomal to maintain homeostasis and eliminate damaged or aberrant proteins from cells and avoid the toxic effects of their accumulation. The proteosome is a complex of proteases that degrade ubiquitinated proteins. The ubiquitin system is the main route for the regulated degradation of cytosol, nucleus and membranes.

Proteosome activity can be regulated by different pathways and levels, such as the synthesis of its subunits;

the rate of its assembly and disassembly; its post-translational regulation that includes the regulation of its proteolytic activity, that is, the recognition of substrates; by subsequent conformational changes of the proteosome, deubiquitination, unfolding of the structure, translocation in the catalytic chamber; the subcellular localization of the proteosome and its recruitment into specific organelles and the destruction of the proteosome itself. Deviations in these mechanisms impact the onset of diseases such as Lateral Amyotrophic Sclerosis, Huntinton's Disease and AD (Livneh I, Cohen-Kaplan V., Cohen-Rosenzweig C, Avni N., Ciechanover A. The life cycle of the 26S proteasome: from birth, through regulation and function, and onto its death. Cell Research (2016) 26:869-885).

Ubiquitin protein plays a pivotal role in the Proteosomal system by binding and polymerizing to misshaped proteins resulting from oxidative stress and initiates the process of elimination of these damaged proteins in the Proteosome (Tramutola A., Di Domenico F.,Barone E.,Perluigi M.,Butterfield, D.A.Oxid Med Cell Longev. It Is All about (U)biquitin: Role of Altered Ubiquitin-Proteasome System and UCHL1 in Alzheimer Disease2016; 2016: 2756068).

As can be seen, different proteins are involved in these processes and can regulate them or correct the negative effects of oxidative processes, defects in spatial conformation or DNA construction, among others, constituting significant therapeutic tools.

Ubiquitin administered as part of the proposed pharmaceutical composition, can restore its normal levels in the organism before the effects of oxidative stress that degrades it in subjects already suffering from a disease mediated by oxidative stress or in subjects with damage and mutations in the genes that generate Ubiquitin with specific deletions of terminal amino acids that disable it for Ubiquitination, or in individuals where the proteins that guarantee the special conformation processes of the proteins prevent the correct folding of the "own" Ubiquitin. Neither in the relevant scientific literature, nor in the patent databases, were solutions found involving the combined use of this protein with the set of the other groups described as part of the pharmaceutical composition of the present invention, nor such marked effects on the *in vitro* and *in vivo* performance parameters of the composition.

Small Ubiquitin-fold modifier Ufm1

The Small Ubiquitin-fold modifier Ufml, with a molecular mass of 9.1 kDa, a spatial conformation similar to Ubiquitin, is characterized because, when processed by its C-terminus, it exposes a glycine residue that would enable its binding to other proteins via E1 (activating), E2 (conjugating), and E3 (binding) enzymes that participate in the Ubiquitination process in tissues such as lung, liver, brain, kidney and heart. It is a post translational modifier. It is presupposed to play a role in the stress response in the endoplasmic reticulum (Herrmann J., Lerman L.O., Lerman A. Ubiquitin and Ubiquitin-Like Proteins in Protein Regulation. Retrieved 21.09.2019 from https://doi.org/10.1161/01.RES.0000264500.11888.f0. Circulation Research. 2007; 100:1276-1291; Komatsu M, Chiba T, Tatsumi K, Iemura S, Tanida I, Okazaki N, Ueno T, Kominami E, Natsume T, Tanaka K. A novel protein-conjugating system for Ufml, a ubiquitin-fold modifier. EMBO J. 2004 May 5;23(9):1977-86. Epub 2004 Apr 8).

Small Ubiquitin-fold Modifier Ufm2

The Small Ubiquitin-folding Modifier Ufm2 is a protein of approximately 100 amino acids, with a spatial conformation also similar to that of Ubiquitin and molecular mass of 12 kDa, which modifies other proteins in cells (sumoylation). Ufm2 acts similarly to Ubiquitin in that it binds to target proteins as part of the post-translational system. In addition to performing a protein degradation function similar to Ubiquitin, it is involved in cellular processes such as apoptosis, protein stability, transcriptional regulation, all of which may have an impact on the treatment of various diseases, especially neurodegenerative diseases. Amyloid Precursor Protein (APP) sumoylation has been linked to reduced levels of Aβ aggregates. Overexpression of SUMO-specific E2 enzymes was associated with lower levels of Aβ aggregation (IMyung-Jin et al. Polymorphisms of small ubiquitin-related modifier genes are associated with risk of Alzheimer's disease in Korean: A case-control study. Journal of the Neurological Sciences. Volume 364, 15 May 2016, Pages 122-127). SUMO is also linked to gene transcription, cell cycle and apoptosis, protein stability, and intracellular and intranuclear trafficking, all of which are of vital importance for the therapeutic treatment of various diseases and aging.

It was recently reported that the coupling of Ufm1 and Ufm2 to proteins is a post-translational modification required for normal long-term synaptic plasticity, the cellular correlate for learning and memory and cognitive processes. It is also referred in the publication that other authors demonstrated that this regulatory process is disrupted in AD patients, confirming that sumoylation plays a primary role in phosphorylation. Arancio, O. asserted in 2019 (Arancio O. Regulation of Synaptic Plasticity and Cognition by SUMO in Normal Physiology and Alzheimer's Disease. Accessed 20.09.2019 at http://columbianeuroresearch.org/taub/res-taub-connect-13-2014.html) that increased sumoylation via transduction of conjugation enzymes recovers synaptic plasticity deficit and memory.

The addition or combination of both protein folding modifiers contributes to the role of Ubiquitin, may lead to a lower appearance of hyperphosphorylated Tau protein and to the beta amyloid protein aggregation and, in general, to a better protein folding in organisms where natural mechanisms do not guarantee its synthesis at adequate levels. In the bibliography consulted, no references were found on the combination of the mentioned proteins with the rest of the proteins included in the pharmaceutical composition of the present application.

Peptidyl-prolyl cis-trans isomerase H

Peptidyl-prolyl-prolyl cis-trans isomerase H protein catalyzes the cis-trans isomerization of proline peptide imide bonds into oligopeptides and accelerates the spatial conformation of proteins. It may act as a chaperone mediating protein interactions. This protein has been shown to restore misfolded Tau protein, such that it is able to bind to microtubules leading to isomerization of cis-Tau to the trans-Tau form. Blair *et al.* (Blair L.J., Baker J.D., Sabbagh J.J., and Dickey C.A. The emerging role of peptidyl-prolyl isomerase chaperones in tau oligomerization, amyloid processing and Alzheimer's disease. J Neurochem. 2015 Apr; 133(1): 1-13) conclude that these proteins regulate A$\beta$ pathology, through isomerization of amyloid precursor protein (APP) or through protection from associated toxicities, and that this regulation may alter the accumulation of A$\beta$ oligomers and plaques. Moreover, they argue that they may regulate Tau protein phosphorylation and alter its structure ultimately protecting from the toxic effects of Tau protein multimerization (Blair L.J., Baker J.D., Sabbagh J.J., and Dickey C.A. The emerging role of peptidyl-prolyl isomerase chaperones in tau oligomerization, amyloid processing and Alzheimer's disease. J Neurochem. 2015 Apr; 133(1): 1-13).

Loss of activity of this protein may alter Ubiquitin-mediated modification of postsynaptic protein density leading to the formation of aberrant synaptic structures. In addition, oxidative stress can lead to increased susceptibility of neurons to A$\beta$ oligomer toxicity (LXu L., Ren Z., Chow F.E., Tsai R., Liu T., Rizzolio F., Boffo S., Xu Y., Huang S., Lippa C.F., and Gong Y. Pathological Role of Peptidyl-Prolyl Isomerase Pin1 in the Disruption of Synaptic Plasticity in Alzheimer's Disease. J Neurochem. 2015 Apr; 133(1): 1-13). The aim of the inclusion of this protein in the pharmaceutical composition precisely consists in achieving a synergy in the natural mechanism of protein spatial conformation in two "circuits" vital for neurotransmission: preventing the formation of hyperphosphorylated tau protein and preventing the formation of A$\beta$-peptide oligomers. No references were found to the therapeutic use of this protein in combination with the others described, nor evidence of the final effect in diseases of the Central Nervous System (CNS) caused by a drug containing it.

Cystatin B

Cystatin B is a very important regulatory protein that maintains a balance between protease and antiprotease activity. The neurotransmitter acetylcholine is associated with normal brain function; if its levels in the cortex of the brain are reduced, AD is triggered. Patients with this condition show reduced levels of acetylcholine and develop pathological changes in cholinergic neurons. One way to combat this phenomenon may be to block the action of the enzyme acetylcholinesterase which cleaves acetylcholine. Cystatin B may play this role, since cystatins in general regulate cathepsins, which are endogenous cysteine proteases, which in turn are permeable to lysosomes or dead cells. Cystatin B, as well as other Cystatins, are natural inhibitors of cysteine proteinases. Dysregulation of Cathepsins leads to the formation of senile plaques, beta-amyloid deposits, tangles of hyperphosphorylated Tau protein in AD (Amin F. and Bano B. Putative Involvement of Thiol Protease Inhibitor in the Function of Alzheimer Drug. Accessed 21.09.2019 at https://www.intechopen.com/online-first/putative-involvement-of-thiol-protease-inhibitor-in-the-function-of-alzheimer-drug). On the other hand, studies in wild type mice reveal the high association between periodontitis and accelerated cognitive decline in AD, demonstrating that Cathepsin B plays a critical role in initiating the process of microglia-mediated neuroinflammation and neural dysfunction, all due to exposure of lipopolysaccharide from Porphyromonas gingivalis. The accumulation of intracellular A$\beta$ in neurons is also demonstrated, it is concluded that Cathepsin B is a therapeutic target to treat neuroinflammation, impaired learning and memory functions (Wu Z., Ni J, Liu Y., Teeling JL., Takayama F., Collcutt A., Ibbett P., Nakanishi H. Cathepsin B plays a critical role in inducing Alzheimer's disease-like phenotypes following chronic systemic exposure to lipopolysaccharide from Porphyromonas gingivalis in mice. Brain Behav. Immun. 2017. Oct; 65:350-361).

Cystatin B is also implicated in epilepsy by inhibiting proteases of the Cathepsin family, both proteins coincide in the nucleus and cytoplasm of proliferating cells (Riccio M.Di Giaimo R, Pianetti S.Palmieri P.P, Melli M. Santi S.Nuclear Localization of Cystatin B, the Cathepsin Inhibitor Implicated in Myoclonus Epilepsy (EPM1). Experimental Cell Research. Volume 262, Issue 2, 15 January 2001, Pages 84-94).

As can be seen, it is essential to have therapeutic tools that prevent or reduce damage from harmful enzymatic activities. These can be avoided by the proper balance of protease and antiprotease activity that is called to inhibit the action of the enzyme acetylcholinesterase that cleaves acetylcholine and, on the other hand, acts as a regulator of cathepsin B, whose level influences the formation of beta-amyloid plaques and the production of hyperphosphorylated Tau protein.

NSFL1 Cofactor P47

Among the diseases associated with aging, AD stands out. It is well known that the disruption of neuronal synapses is caused by hyperphosphorylation of the Tau protein. To prevent this, dendritic branching and maintenance of neuronal connections is essential. The NSFL1 cofactor P47 is linked to transport and fusion into vesicles and membranes and between membrane compartments, thus playing a critical role in protein transport within cells (Kent Z. Q. Wang, Erin Steer, P. Anthony Otero, Nicholas W. Bateman, Mary Hongying Cheng, Ana Ligia Scott, Christine Wu, Ivet Bahar, Yu-Tzu Shih, Yi-Ping Hsueh and Charleen T. Chu. PINK1 Interacts with VCP/p97 and Activates PKA to Promote NSFL1C/p47 Phosphorylation and Dendritic Arborization in Neurons. eNeuro 19 December 2018, 5 (6) ENEURO.0466-18.2018. Retrieved 19.09.2019 from https://www.eneuro.org/content/5/6/ENEURO.0466-18.2018).

The novel inclusion of NSFL1 Cofactor P47 in the pharmaceutical composition and its combination with the rest of the proteins guarantees an adequate balance of the ubiquitin-proteosome system, ensuring the intracellular transport towards the proteosome of the hyperphosphorylated tau protein that could not be achieved by the routes previously described in previous inventions.

UV excision repair protein RAD23 homolog B

Other proteins are essential in the mechanism of degradation of oxidatively damaged proteins due to the aging process, such as the UV excision repair protein RAD23 homolog B, which binds to the proteosome and assists in the recruitment of ubiquitinated proteins to proteosomes (Vaites L.P. and Harper W. Protein aggregates caught stalling. DOI: 10.1038/d41586-018-03000-2. Accessed 20.09.2019 at https://www.savalnet.cl/cienciaymedicina/progresosmedicos/ agregados-proteicos-en-la-neurodegeneracion.html: Dantuma N.P., Heinen C., Hoogstraten D. The ubiquitin receptor Rad23: At the crossroads of nucleotide excision repair and proteasomal degradation. DNA Repair. 2009. 8(4):449-460). RAD23 is inhibited by excess phosphorylation, so it is essential to avoid the process of hyperphosphorylation, which can be achieved by employing other protein- and peptide-mediated mechanisms.

The pharmaceutical composition also includes this important protein that acts in the final stages of the whole process that takes place in the ubiquitin-proteosome system and ensures the excision of the proteins in the complex, especially hyperphosphorylated Tau.

DNA damage, as an expression of stress and cellular damage also caused by oxidation, accompanies different neurological disorders such as Down syndrome, Parkinson's disease, AD, cerebral ischemia and cranial trauma (Martin L.J. DNA Damage and Repair: Relevance to Mechanisms of Neurodegeneration J Neuropathol Exp Neurol. 2008. Vol. 67, No. 5). UV excision repair protein RAD23 homolog B may play a pivotal role in the correction of DNA damage.

Any failure in the ubiquitination mechanism implies the non-recognition of the damaged (oxidized) proteins, and therefore, their toxic accumulation and non-degradation in the proteosome and the emergence of malignant, autoimmune and neurodegenerative diseases, among others.

In general, no effective combinations of the proteins of this group with effect on the ubiquitin-proteosome system have been described, acting in combination with each other or in combination with the other groups of proteins comprising the pharmaceutical combination described in the invention and which have caused the beneficial effects on the cognitive aspects such as those achieved with the proposed protein-based pharmaceutical composition described herein.

Thymus proteins and peptides with hormonal properties

Inflammatory diseases are characterized by severe imbalances of the immune system leading to massive damage to organs and systems. Lunin and Novoselova published a detailed review of scientific publications of the last two decades on the regulation of the immune system by thymus hormones. (Lunin S.M. and Novoselova E.G. Thymus hormones as prospective anti-inflammatory agents. Expert Opinion on Therapeutic Targets. 2010. 14(8):775-86). Special attention is given to three of the hormones: Thymulin, Thymosin alpha and Thymopoietin. It is proposed that the regulation may be mediated by the effect of thymic hormones on the activities of peripheral immune cells and the bidirectional coupling between thymic hormones and the hypothalamic-pituitary-adrenal axis. Thymulin exerts mostly inhibitory effect on immune responses and stimulates the endocrine system and reveals its role in recovery from stress conditions. Thymosin alpha stimulates the immune response, especially the Th-1 response and indicates its theoretical beneficial effect for the treatment of viral and oncological diseases. This protein has been successfully used for the treatment of Hepatitis B. Thymopoietin stimulates the immune response and inhibits it under stress conditions.

The site http://es.peptidesstore.com/blogs/articles/9236639-peptide-regulation-of-ageingse describes the role of peptides obtained from thymus, liver and pineal gland in the induction of protein synthesis and restoration of functions in the organism. The interaction of the studied peptides with DNA binding sites in gene promoter segments with induction of double-stranded helical disjoining, and also activation of RNA polymerase is demonstrated. The article points out the potentiality of employing not only complete proteins, but also derived peptides for the activation of natural mechanisms of restoration of deviations of "natural" functions of proteins in the human organism.

Cathelicidin-4

Within the hypotheses explaining the emergence of AD, the Antimicrobial Protection hypothesis is distinguished. In this model, the deposition of Aβ is a consequence of an innate immune response to the genuine or mistaken perception of an immune challenge. It is assumed that, first, Aβ traps and neutralizes invading pathogens on β-amyloid. Fibril formation regulates neuroinflammatory pathways to fight infection, but in the case of AD this process is dysregulated leading to chronic inflammation and neurodegeneration (D.Moir R., Lathe R., TanziR.T. The antimicrobial protection hypothesis of Alzheimer's disease. Alzheimer's & Dementia. Volume 14, Issue 12, December 2018, Pages 1602-1614, 2018; Soscia SJ, Kirby JE, Washicosky KJ, Tucker SM, Ingelsson M, et al. (2010) The Alzheimer's Disease-Associated Amyloid b-Protein Is an Antimicrobial Peptide. PLoS ONE 5(3): e9505. doi:10.1371/journal.pone.0009505). Some of the microorganisms triggering AD-associated processes may include spirochetes, viruses, fungi and Chlamydia pneumoniae, among others (Fülöp T, Itzhaki RF, Balin BJ, Miklossy J and Barron AE (2018) Role of Microbes in the Development of Alzheimer's Disease: State of the Art - An International Symposium Presented at the 2017 IAGG Congress in San Francisco. Front. Genet., 10 September 2018. https://doi.org/10.3389/fgene.2018.00362).

Cathelicidin-4 has a structure that maintains separate hydrophobic and cationic amino acids that facilitates interaction with microbial membranes. The electrostatic interaction facilitates the binding of the positively charged peptides to the negatively charged bacterial membrane. This makes it possible for the protein to integrate into the lipid bilayer of pathogenic bacteria causing cell rupture and death (BioSystems NCBI. Consulted on 20.09.2019 at https://www.ncbi.nlm.nih.gov/biosystems/1457777?Sel=geneid:820#show=genes2007).

Cathelicidin 1 belongs to the group of antimicrobial peptides present in the lysosomes of macrophages and polymorph-nuclear leukocytes. Its role is relevant in the innate immune defense against pathogenic bacteria. These proteins present in the therapeutic pharmaceutical composition of the present invention guarantee to cover all possible triggering mechanisms of neurological diseases such as AD, diseases caused by microorganisms which in many cases are concomitant diseases with the main ones, especially in immunocompromised subjects, elderly or in vulnerable health situations. In this way, the organism's natural arsenal for defense against pathogen invasion is strengthened, an approach that has not been found in patent documents or scientific dissemination in which these molecules are efficiently combined for therapeutic purposes.

Copper transport protein ATOX1

The copper transport protein ATOX1 (Antioxidant protein 1) is a metalloprotein involved in copper homeostasis by releasing copper from the cytosol to ATPase transporters and is localized in the cytoplasm. It binds free metals and protects cells from reactive oxygen species and metal misbinding on metalloproteins. This protein is primarily expressed in neurons and is found in high concentrations in different subtypes of neurons with high levels of metals such as copper, iron and zinc.

In AD, hypercholesterolemia and homeostasis are risk factors as they participate in the generation of beta-amyloid plaques and neurofibrillary tangles. Aβ formation occurs in brains with high cholesterol and copper levels that enable the formation of Aβ:copper complexes that can catalytically oxidize cholesterol and generate $H_2O_2$, oxysterols and other products of lipid peroxidation. Tau protein is sensitive to the interaction of copper with cholesterol as it is involved in the hyperphosphorylation and aggregation cascade that precedes the formation of hyperphosphorylated Tau tangles. ATOX 1 may thus play a pivotal role in the prevention of oxidative stress damage (Hung Y.H., Bush A.I., and La Fontaine S. Links between copper and cholesterol in Alzheimer's disease. Review ARTICLE. Front. Physiol., 16 May 2013 https://doi.org/10.3389/fphys.2013.00111). Retrieved 27.09.2019 from https://www.ncbi.nlm.nih.gov/pmc/articles/PMC3655288/).

In mice transfected with the gene encoding ATOX 1 it has been shown that, under conditions of oxidative damage, the transfected neurons were protected against oxidative damage and this protection is similar to that achieved with copper/zinc superoxide dismutase. The role of ATOX 1 has relevance not only in AD but in amyotrophic lateral sclerosis, Wilson's disease (decreased enzymatic activity of copper-dependent enzymes in the brain) and Menkes disease (oversaturation to toxic levels of copper leading to neurodegeneration) (Kelne G.S., Lee M., Clark M.E., Maciejewski D., McGrath D., Rabizadeh S., Lyons T., Bredesen D., Jenner P., Maki R.A. The Copper Transport Protein Atox1 Promotes Neuronal Survival. The Journal of Biological Chemistry. 2000. 275, 580-584). ATOX1 is involved not only in the activation of copper-dependent enzymes involved in neurotransmitter biosynthesis, iron efflux, neurovascularization, wound healing, and blood pressure regulation, but also in modulating responses to cancer therapies, inflammation response, and oxidative stress. In short, it acts not only as a copper chaperone but also as an antioxidant (Hatori Y and Lutsenko S. The Role of Copper Chaperone Atox1 in Coupling Redox Homeostasis to Intracellular Copper Distribution. Antioxidants (Basel). 2016 Sep; 5(3): 25. Published online 2016 Jul 27).

In addition to the aforementioned diseases, copper disorders are associated with Parkinson's disease and amyotrophic lateral sclerosis (Greenough M. A., A. Ramirez Munoz, Busha A. I. and Opazo C. M. Metallo-pathways to Alzheimer's disease: lessons from genetic disorders of copper trafficking. Metallomics, 2016,8, 831-839). It was considered relevant and novel to include this protein in the pharmaceutical composition because of the diversity of biological and physiological mechanisms in which it intervenes, which include effects on homeostasis, binding of free metals such as copper, iron and zinc, critical for the process of pereoxidation; inhibition of beta-amyloid lattice formation, protection against hypercholesterolemia and the damage it causes by pereoxidation, the avoidance of Tau protein hyperphosphorylation, and its participation in neurotransmitter synthesis, neurovascularization and healing. In this way, it not only acts in a beneficial way, but also potentiates the action of the rest of the proteins with which it is combined.

Transcription elongation factor A1 (TCEA1)

Synaptic dysfunction characterizes several neuronal disorders, including AD, in which there is a link between deficits in synaptic plasticity and impaired capacity for new protein synthesis. Transcription elongation factor A1 (TCEA1) is strongly involved in synaptic plasticity. Beckelman *et al.* in 2016 demonstrated in transgenic mice and in human brains analyzed post mortem that in AD the levels of this protein were significantly decreased, especially in the hippocampus. The authors suggest a high correlation between dysregulation of protein synthesis and neuronal synapse breakdown in AD. It interacts selectively with zinc via non-covalent bonds.

The also called transcription elongation factor SII is responsible for the elimination of polymerase pauses or stalls and specifically acts by preventing RNA-polymerase from stopping prematurely (Carlos M.G. Elongation. Retrieved 27.09.2019 from https://www.sebbm.es/ BioROM/contenido/av_bma/apuntes/T11/elonga.htm. CETEX: Scientific Histopathology Report of the Trial Evaluating the Effect of Biocen-128 in Naturally Aged Male BALB/c Mice. 2018). The translation process has an estimated error rate of 1 amino acid per 103 - 104 due to selection of incorrect tRNAs during the chain elongation process (Juarez P.R. Quality control failures in protein synthesis: origin of rare diseases. Faculty of Biology. University of Salamanca. Thesis. 2016). On the other hand, it is known that Tau protein aggregation is linked to several neurological disorders including AD. These disorders induce post-translational modifications and alter the structure of molecular chaperones (Fontaine, S.N., et al., (2015). Cellular factors modulating the mechanism of tau protein aggregation. Cell. Mol. LifeSci. 72, 1863-1879).

Transcription elongation factor A2 (TCEA2)

Transcription elongation factor A2 (TCEA2) possesses in its structure a Zinc-containing motif present in proteins that interact with nucleic acids. It catalyzes the acetylation or deacetylation of histone-like proteins (Wind M and Reines D. Transcription elongation factor SII. Bioessays. 2000 Apr; 22(4): 327-336). Both factors were considered to form the pharmaceutical composition because their activity differs from the activity of the rest of the proteins included.

Serine/arginine-rich splicing factor 2

Serine/arginine-rich splicing factor 2 belongs to a family of cofactors containing one or two RNA recognition motifs and a serine/arginine-rich domain. It plays an important role in post-translational modifications. Relevant is its role in the splicing of phosphorylated proteins such as those involved in ubiquitin-proteosome mechanisms (Tan W., Wang W. and Ma Q. Physiological and Pathological Function of Serine/Arginine-Rich Splicing Factor 4 and Related Diseases. Biomed Res Int. 2018; 2018: 3819719. Published online 2018 Feb 12. doi: 10.1155/2018/3819719).

In Down syndrome phosphorylated tyrosine (Dyrk1A) and regulated kinase 1A interact with one of the serine/arginine-rich splicing factors (SRp55) and phosphorylates its proline-rich domain, which suppresses the ability to promote Tau exon inclusion. Thus upregulation of Dyrk1A can lead to neurofibrillary degeneration as in Down syndrome (Xiaomin Yin X., Jin N., Gu J., Shi J., Zhou J., Zhou J., Gong C-X., Iqbal K., Grundke-Iqbal I. and Liu F. Phosphorylation-regulated Kinase 1A (Dyrk1A) Modulates Serine/Arginine-rich Protein 55 (SRp55)-promoted Tau Exon 10 Inclusion. 2012 The Journal of Biological Chemistry 287, 30497-30506).

The following is a synthesis of examples, although not limited, of the therapeutic applications of the proteins that make up the pharmaceutical composition of the present invention, indicating the activity that one or more of them present and the diseases in which they are applied:

- improvement or eradication of cognitive deficit: AD, Leigh's disease, Epilepsy, ischemia, multiple sclerosis, encephalopathies;
- anti-inflammatory activity: AD, multiple sclerosis, amyotrophic lateral sclerosis, retrobulbar neuritis, polyneuropathies, systemic lupus erythematosus, rheumatoid arthritis, juvenile idiopathic arthritis, ankylosing spondylitis, rheumatic fever, Behcet's syndrome, iridocyclitis, psoriasis, ulcerative colitis, Crohn's disease, irritable bowel syndrome, chronosinusitis, atopic dermatitis, asthma;
- immune system regulation: AD, lupus erythematosus, multiple sclerosis, rheumatoid arthritis, fibromyalgia, osteoarthritis, myasthenia gravis, sarcoidosis, Sjögren's syndrome, Behcet's syndrome, urticaria, Crohn's disease, Louis-Barr's syndrome, atopic dermatitis, aplasia, thymic hypoplasia, severe combined immunodeficiency, common variable immunodeficiency, IgA deficiency, ataxia-telangectasia, Wiskott-Aldrich Syndrome, immunodeficiencies secondary to infections;
- regulation of oxidative phosphorylation (antioxidant activity): AD, Leigh's disease, multiple sclerosis, amyotrophic lateral sclerosis;
- correction of copper metabolism disorders: Wilson's disease;
- lowering of cholesterol levels: AD, rheumatoid arthritis;
- restoration of neuronal plasticity;
- antimicrobial activity and infections in general, both as disease triggers or as a consequence and regulation of the intestinal microbiome: AD, multiple sclerosis, reactive arthritis, rheumatic fever, psoriasis, Wiskoft-Aldrich syndrome, HIV-AIDS.

By way of summary the most significant advantages of the present invention mentioned above are disclosed, adding some others that were not explicitly described:

- None of the above solutions describes the treatment of such diverse neurological, autoimmune, immunological and other diseases with the same protein pharmaceutical composition. Understanding by treatment, the prevention of the disease, its cure or reversal or arrest, especially in the early stages, the partial or total decrease of the symptoms, their attenuation.
- None of the above solutions describes one or more compositions that intervene at the same time in the possible mechanisms described in the scientific literature that lead to the onset of AD and, in general, in neurological disorders, including oxidative stress; hyperphosphorylation; inflammation, including neuroinflammation; dysregulation of the immune response; disorders of lipid metabolism (hypercholesterolemia) and of metals such as copper, iron and zinc (highly toxic and oxidizing in excess); reduced levels of acetylcholine; disorders of ubiquitination, sumoylation and incorrect protein folding; loss of intracellular protein stability; formation of beta-amyloid peptide oligomers; loss of plasticity and neuronal synapses; the action of pathogenic microorganisms and incorrect protein synthesis and transcription.
- All the proteins and their component peptides in the pharmaceutical composition and their different variants are present in the organism, therefore, they cannot cause any type of rejection in the patients who receive the treatment, by restoring the physiological levels of the same in the organism, especially when the levels are affected as a result of a pathology or caused by it.
- It was demonstrated in *in vivo* and *in vitro* experiments and in patients and subjects the compatibility between all the proteins and peptides that make up the pharmaceutical composition, an aspect that was neither expected nor obvious. No toxicity, tissue damage, damage to organs or to the human organism, nor immunogenicity was detected, since the pharmaceutical composition uses proteins obtained by different routes present in humans or peptides derived from proteins or that compose them of up to 50,000 Da and those coming from mammals, especially bovids with molecular weights lower than 20,000 Da and with sequences equal or similar to those of humans.
- In the case of using proteins and their component peptides described in the invention obtained from species other than humans, such as bovids, no incompatibility is presented, given the high degree of homology and, in addition, the absence of toxicity was experimentally demonstrated in these cases, even using all the proteins or their component peptides described in groups a, b, d, e and f with some of group c, by different routes and in the concentrations proposed.
- A non-obvious synergistic effect is achieved between the proteins and peptides of the pharmaceutical composition that even leads to unexpected effects, as is the case of the decrease of cholesterol and triglyceride content in blood and the better balance in the population of B lymphocytes in target organs.
- The effects of the pharmaceutical composition of the present invention demonstrated in experiments with naturally aged animal models surpass the effects achieved in transgenic models described in previous solutions in the state of the art, in which pathologies are manifested that occur differently from aging as a natural process, even demonstrating in examples of the present invention, a cognitive behavior similar to young animals without any treatment. This result has not been observed in other solutions.
- The proteins and their component peptides used in the pharmaceutical composition of the present invention are compatible with other proteins used in the treatment of other diseases such as cancer, and can also be combined in the treatment with chemo- or radio-therapy, since as already mentioned they are all found in the organism fulfilling the functions that are deficient in these patients.
- The range of concentrations of the proteins and their component peptides described in the present invention, both for the individual proteins or peptides, their groups or subgroups, as well as for the total of the same, makes it possible to better direct the treatment to specific diseases or disorders and even apply them to different phases of the treatment or stage of the disease and to personalize the treatments.

- For the first time, a single pharmaceutical composition is described with which it is possible to treat patients with multiple pathologies of different origins in different phases typical of the elderly and especially the elderly, for example, patients suffering from a neurological disease such as AD, with hypercholesterolemia and diabetes, arthritis of various kinds, immunosenescence, infection and even cancer.

- As the proteins and their component peptides that make up the pharmaceutical composition are relatively small in size, less than 50 000 Da and preferably less than 20 000 Da, very different formulations can be achieved, with different vehicles for their safe administration by the most commonly used routes in therapy (intranasal, intramuscular, intravenous, oral and sublingual).

- Unexpected results are achieved, not only in the treatment of diseases, but also in anatomy, such as the development of greater brain mass *in vivo* in experimental animals; this finding, also linked to the *in vitro* demonstration of a neuronal cell proliferation effect and the absence of cytotoxicity, provides a novel effect for the set of proteins and their component peptides included in the composition.

- Surprising results of total absence of toxic effect as described in the extended acute toxicity test in two animal species are achieved by employing such a significant number of proteins and peptides with different functions.

**BRIEF DESCRIPTION OF THE FIGURES**

Figure 1 shows the percentage of alternation in the Y-maze test of 12- to 13-month-old male BALB/c mice treated with the C4 variant pharmaceutical composition relative to young and aged control animals (2 to 3 months of age and 12 to 13 months of age).

Figure 2 presents a graph showing the number of entries of experimental animals into the Y-maze of 12- to 13-month-old male BALB/c mice treated with the pharmaceutical composition of the C4 variant relative to young and aged control animals (2 to 3 months of age and 12 to 13 months of age).

Figure 3 shows the results of the Morris water maze test with 14- to 15-month-old female C57BL/6 mice. Group G1 represents young controls (2-3 months of age) receiving 0.9% NaCl (placebo). Group G2 includes aged controls receiving 0.9% NaCl (placebo). Groups G3 and G4 receive treatment with the pharmaceutical compositions of variants C7 and C8 respectively for 6 weeks. A one-way ANOVA and a Tukey post-hoc test ($p<0.05$) were applied. Significant differences for each group with respect to the time the animals remain in the platform quadrant (start of treatment) are marked with the sign *.

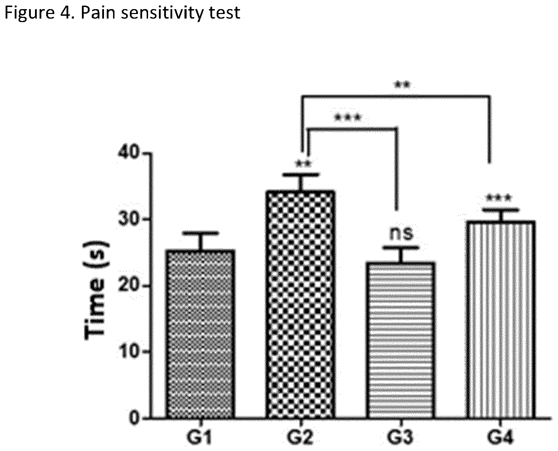

With the help of Figure 4, the results of the pain sensitivity test of 14- to 15-month-old female C57BL/6 mice are presented. Group G1 includes the young controls (2-3 months old) receiving 0.9% NaCl (placebo). Group G2 to aged controls receiving 0.9% NaCl (placebo). Groups G3 and G4, receive treatment with the pharmaceutical compositions of variants C7 and C8 respectively for 6 weeks. A one-way ANOVA and a Tukey post-hoc test ($p < 0.05$) were applied. Significant differences for each group with respect to the group of young control animals are distinguished with the symbol *.

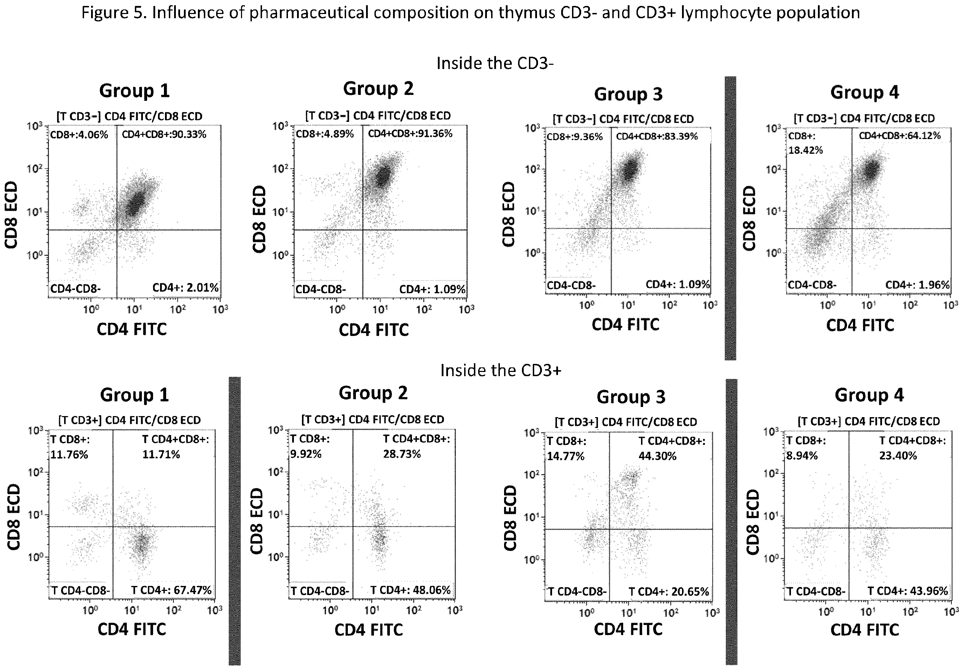

Figure 5 shows the bar graph of the influence of the pharmaceutical composition on the CD3- and CD3+ lymphocyte population determined by flow cytometry of thymus homogenate from experimental mice.

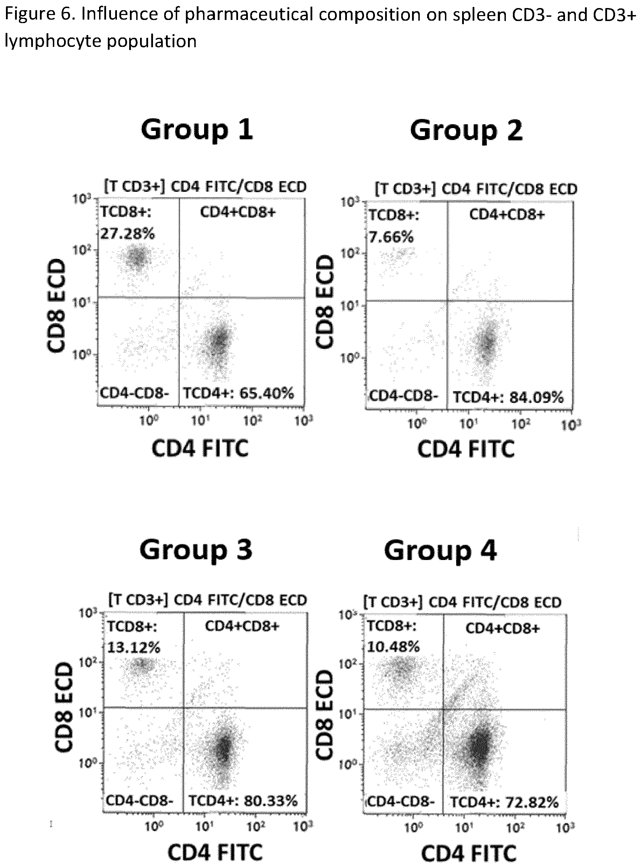

Figure 6 shows the influence of the experimental pharmaceutical composition of the present invention on the CD3- and CD3+ lymphocyte population of spleen homogenate assayed by flow cytometry.

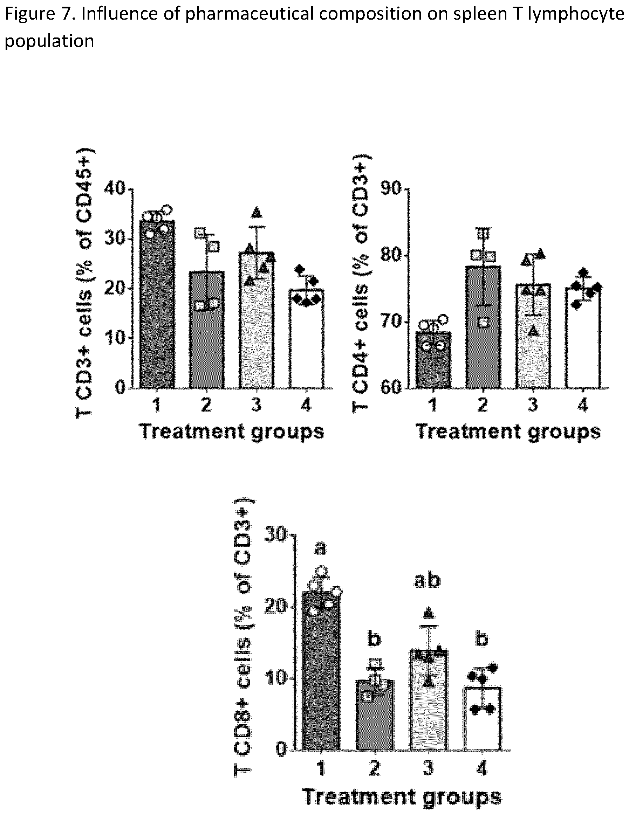

Figure 7 presents a graph showing the results of the study of the influence of the experimental pharmaceutical composition on the T-lymphocyte population of spleen homogenate measured by flow cytometry.

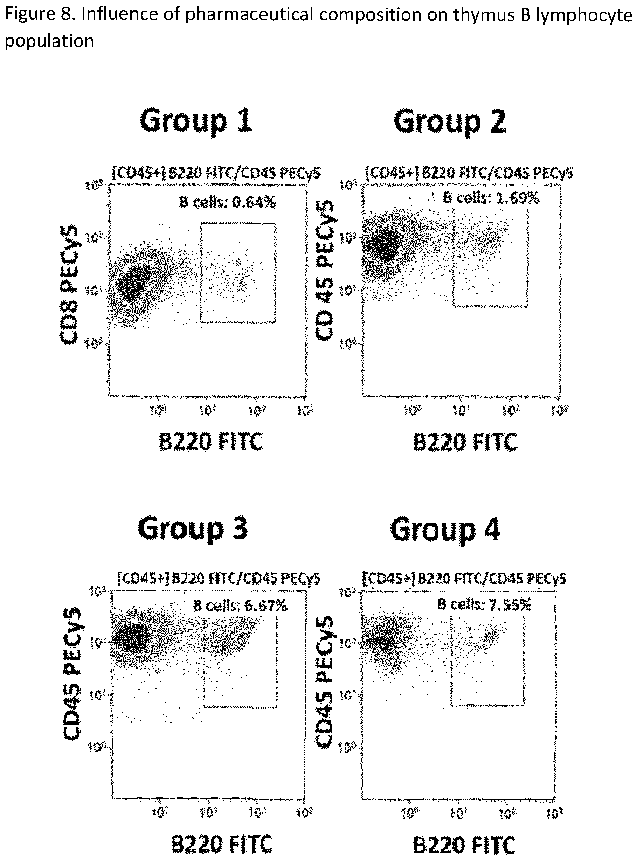

Figure 8 details the influence of the experimental pharmaceutical composition on the thymus homogenate B-lymphocyte population of the experimental animals determined with the aid of the flow cytometry technique.

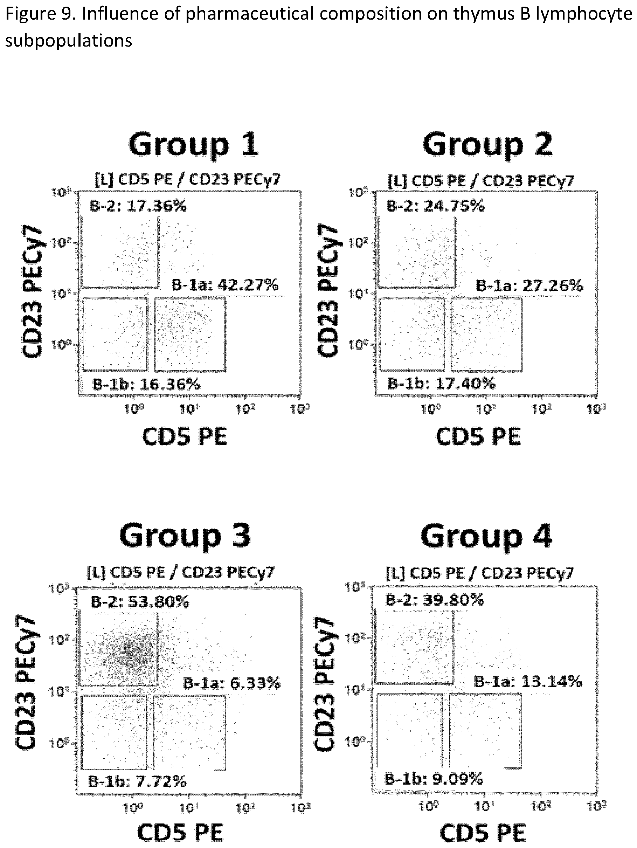

Figure 9 gives a graphical view of the influence of the pharmaceutical composition on the thymus homogenate B-lymphocyte subpopulations obtained in the group of animals that was treated with the pharmaceutical composition of the present invention.

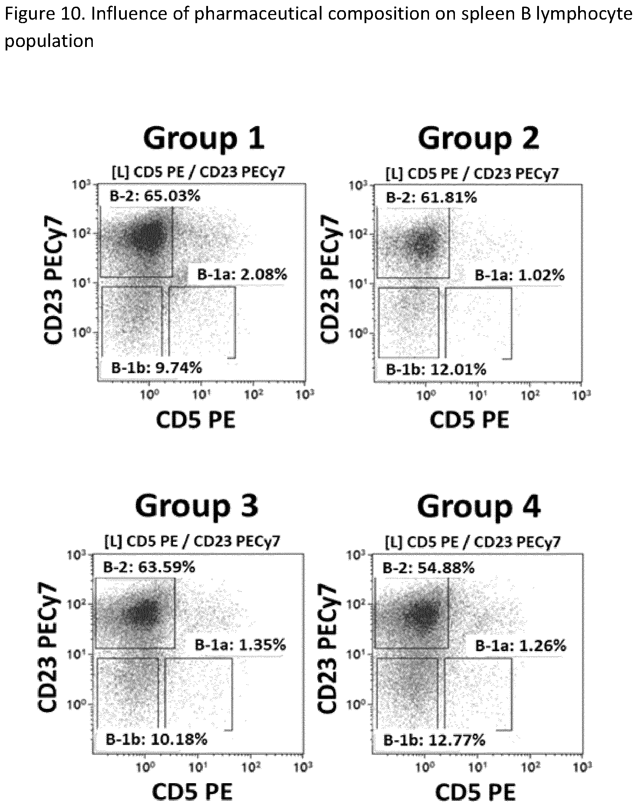

Figure 10 shows results evidencing the influence of the pharmaceutical composition on the spleen B lymphocyte population.

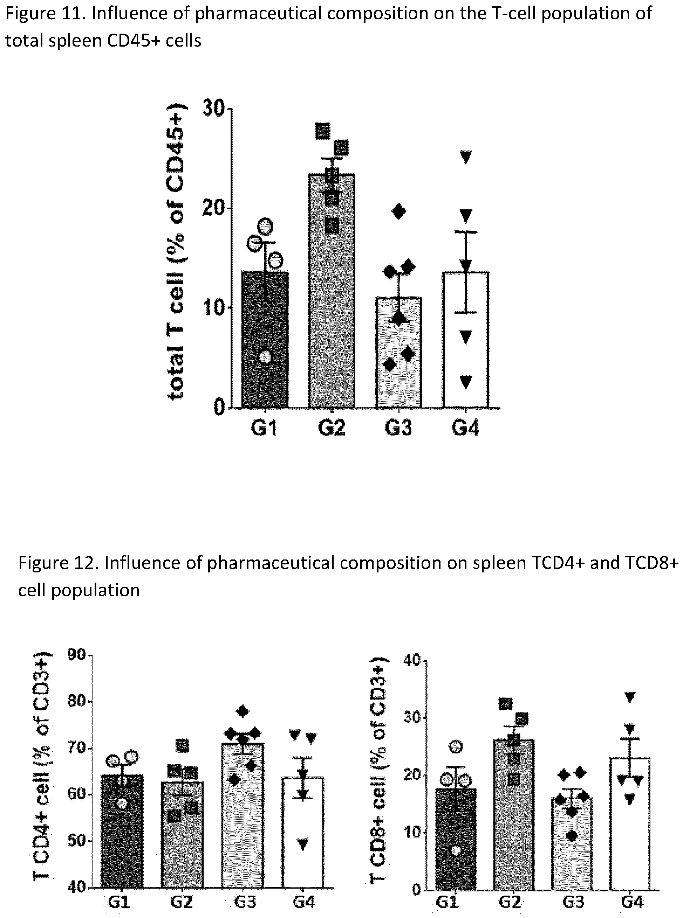

Figure 11 demonstrates the influence of the pharmaceutical composition of the present invention on the T cell population of the total CD45+ cells in spleen homogenates of experimental mice.

Figure 12 plots Influence of the pharmaceutical composition of the present invention on the TCD4+ and CD8+ T cell population of spleen homogenate.

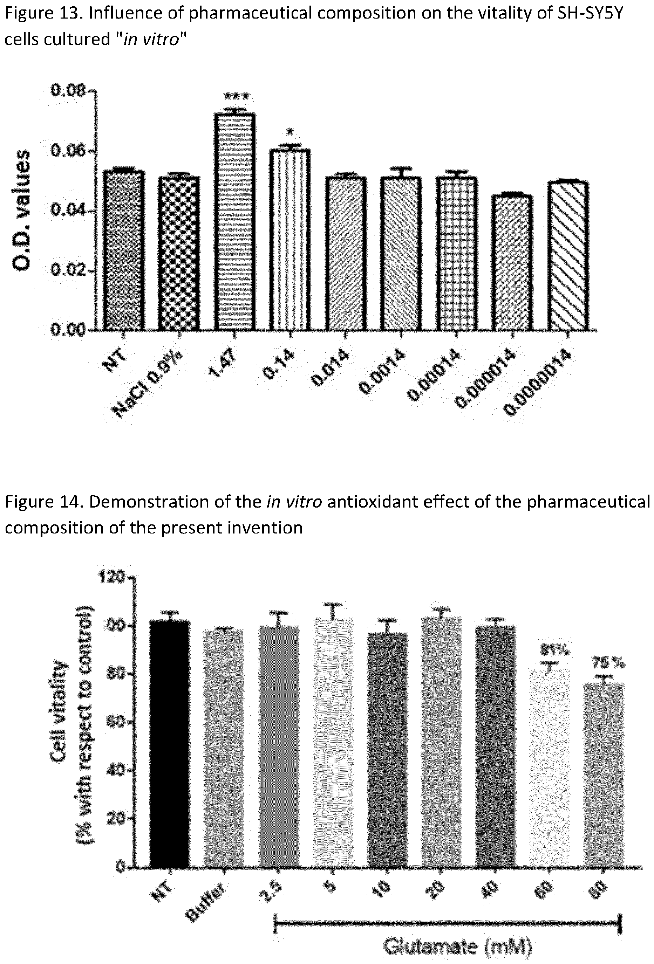

Figure 13 shows the influence of the pharmaceutical composition on the vitality of SH-SY5Y cells cultured *"in vitro"* and the absence of cytotoxic effect.

Figure 14 is a reflection of the experiment for the demonstration of the antioxidant effect on neuronal cells *in vitro* of the pharmaceutical composition of the present invention.

**EMBODIMENT EXAMPLES**

**Example 1.** Effect of Pharmaceutical Compositions C1, C2 and C3 on cognitive improvement in aged BALB/c mice Compositions C1, C2 and C3 -Include pharmaceutical combinations that are diluted with different amounts of 0.9% NaCl solution composed of all proteins of group **a**- Thioredoxin* (0.000004 mg/mL for C1, 0, 000034 mg/mL for C2 and 0.00006 mg/mL for C3), Cytochrome C (0.000004 mg/mL for C1, 0.000034 mg/mL for C2 and 0.00006 mg/mL for C3), Cytochrome C oxidase subunit NDUFA4 (0.000004 mg/mL for C1, 0.000034 mg/mL for C2 and 0.00006 mg/mL for C3); all group **b** proteins - Ubiquitin (0.06 mg/mL for C1, 0.51 mg/mL for C2 and 0.9 mg/mL for C3), the Small Ubiquitin-Fold Modifier Ufm1 (0.048 mg/mL for C1, 0.408 mg/mL for C2 and 0.72 mg/mL for C3), Small Ubiquitin-fold modifier Ufm2 (0.048 mg/mL for C1, 0.408 mg/mL for C2 and 0.72 mg/mL for C3), Peptidyl-prolyl-cis-trans isomerase H (0.000004 mg/mL for C1, 0.000034 mg/mL for C2 and 0.00006 mg/mL for C3), Cystatin B (0, 000004 mg/mL for C1, 0.000034 mg/mL for C2 and 0.00006 mg/mL for C3), a peptide composing the NSFL1 Cofactor P47 containing the sequences SEC ID NO 25 and SEC ID NO 26 (0.000004 mg/mL for C1, 0.000034 mg/mL for C2 and 0, 00006 mg/mL for C3) and a peptide composing UV excision repair protein RAD23 homolog B containing in its molecule the sequences SEC ID NO 27 and SEC ID NO 28 (0.000004 mg/mL for C1, 0.000034 mg/mL for C2 and 0.00006 mg/mL for C3); the group c protein subgroup i with the sequences in its molecule: SEC ID NO 29, SEC. ID NO. 30 and SEC ID NO 31, identified by mass spectrometry and with the help of the Swiss Prot database as belonging to Thymosin Beta 4 (0.044 mg/mL for C1, 0.372 mg/mL for C2 and 0.657 mg/mL for C3); the group **d** proteins Cathelicidin-4 precursor (0.000004 mg/mL for C1, 0.000034 mg/mL for C2 and 0.00006 mg/mL for C3) and Cathelicidin-1 (0.000004 mg/mL for C1, 0.000034 mg/mL for C2 and 0.00006 mg/mL for C3); the copper transport protein ATOX1 (0.000004 mg/mL for C1, 0.000034 mg/mL for C2 and 0.00006 mg/mL for C3) from group **e** and from group **f** a peptide composing the transcription elongation factor A protein 1 (TCEA1) containing in its molecule the sequence SEC ID NO 49 (0.000004 mg/mL for C2 and 0.00006 mg/mL for C3), 000004 mg/mL for C1, 0.000034 mg/mL for C2 and 0.00006 mg/mL for C3), a peptide composing serine/arginine-rich splicing factor 2 containing in its molecule the sequences SEC ID NO 50 and SEC ID NO 51 (0.000004 mg/mL for C1, 0.000034 mg/mL for C2 and 0.00006 mg/mL for C3).

Pharmaceutical composition C1 is formulated by diluting the components of the pharmaceutical combinations in 0.9% NaCl solution, so that the total concentration of total proteins is 0.2 mg/mL determined by the Lowry method. Pharmaceutical composition C2 contains 1.7 mg/mL of proteins and pharmaceutical composition C3 contains 3.0 mg/mL. The pH of compositions C1 and C2 is 6.28 and that of pharmaceutical composition C3 is 6.3.

Experimental protocol

Aged male BALB/c mice, 12 to 13 months old and weighing 32 g to 34 g, are used to verify the cognitive deficiency in comparison with young animals of 2 to 3 months of age, based on the application of different behavioral tests. During the whole experimental procedure, the animals are kept in a conventional room with a controlled environment (minimum security barrier type IV), with a temperature of 21 $\pm$ 3 °C and relative humidity from 40% to 70%, with 18 air changes per hour, 100% injection of outside air with 85% filtration. In addition, 12 hours of light and 12 hours of darkness automatically controlled and with free access to water and food.

Treatment:

Aged animals are distributed in 6 groups of 6 animals each (from G2 to G7) and young animals are grouped in one group (G1). The animals in group G2 receive the pharmaceutical composition C1 (with 0.2 mg/mL of proteins) with a daily frequency for 6 weeks; those in group 3 (G3) receive the pharmaceutical composition C2 (1.7 mg/mL) with a daily frequency for 6 weeks; those in group 4 (G4) receive the pharmaceutical composition C2 (1.7 mg/mL) but with an alternate day frequency for 6 weeks; group 5 (G5) is given the same pharmaceutical composition C2 (1.7 mg/mL) but with a frequency of 2 times per week for 6 weeks, and in the case of group 6 (G6) pharmaceutical composition C3 (3.0 mg/mL) is used with a frequency of 2 times per week for 6 weeks.

Regardless of the composition, the dose is administered in a volume of 30 μL intranasally. A 0.9% NaCl solution is used as a vehicle. The response to treatment is analyzed by the object recognition test.

Groups G1 and G3 of this experiment, plus a group G7 (aged control, receiving 0.9% NaCl solution) are used to analyze brain weight. The animals are sacrificed by cervical dislocation and five animals from each group have their brains removed. Subsequently, the brain is weighed on a 0.0001 g precision analytical balance (Sartorius, Germany) and the weight of the organ relative to the body weight of the animal is calculated.

Parameters to be evaluated:

Object recognition test: it is performed inside an acrylic box with dimensions of 22 × 22 × 25 cm. In the first phase of the test, the animal is placed inside the empty box for 3 min to adapt to the test conditions. Subsequently, two objects identical in shape, size and texture are placed at the diagonal ends of the box and the animal is allowed to explore both objects for 7 min. The animals are then removed from the test box and after 1 h they return to the same box, but one of the objects is replaced by another one of different shape, but with similar texture and size. In this phase the animal is also left to explore for 7 min and the execution of the test is recorded with a digital camera. The exploration time is considered as that in which the animal bites the object, touches it with the forelimbs, nose or vibrissae, or places the nose at a distance of less than 1.5 cm to 2.0 cm from the object. In addition, in this segment of the test, those animals that in the first 3 min do not show interest in exploring the objects are rejected. In this experiment, the preference index (PI) per object is calculated from the exploration time of each object and according to the expression:

$$\text{PI for FO (\%)} = \frac{FO\ Exp.Time}{FO\ Exp.Time + NO\ Exp.Time}\ X\ 100$$

$$\text{PI for NO (\%)} = \frac{NO\ Tiempo\ Exp.}{FO\ Exp.Time + NO\ Tiempo\ Exp.}\ X\ 100$$

where:

    FO: Familiar object
    NO: Novel object
    PI: Preference index
    FO Exp. Time: exploration time of the familiar object
    NO Exp. Time: exploration time of the novel object

Results:

Table 1 shows that formulations C1, C2 and C3, when administered daily, every other day or 2 times per week to the experimental animals, succeed in improving cognitive function in those with impairment, since the scanning time for NO is significantly longer than that of FO ($p < 0.01$).

This experiment demonstrates the usefulness of the compositions of the present invention in dementing diseases or in diseases leading to other cognitive disorders such as AD or to palliate the effects of cognitive impairment associated with aging.

On the other hand, when comparing the results of the groups with compositions C1, C2 and C3 in the three study frequencies with respect to the animals of group G7 (aged animals without treatment), it is observed that the IP index for the known object decreases drastically and the IP of the new object increases significantly with the treatment. These findings confirm that there is a combined action of the proteins that make up the pharmaceutical composition on memory in the experimental animals, which leads to affirm that in humans the cognitive functions typical of the effects of senescence are recovered and that the pharmaceutical composition has application in the treatment of diseases such as AD, Leigh's disease, epilepsy, multiple sclerosis, amyotrophic lateral sclerosis, among others.

The results in this experiment surpass those referenced in the scientific literature and patent sources, such as those of patent document US9192654 B2, since that work uses an animal model obtained by induction caused by intracerebroventricular administration of the amyloid-β25-35aa peptide to demonstrate the effect of the nonpeptide PAT compound on cognitive improvement. That model has the disadvantage that it only reproduces the acute phase of the disease (Charleine Z, Anthony B, Brice D, Stephane M, Emeline K, Guy I, Gaelle N, Johann M, Nathalie C, Tangui M, Laurent G. 2011. Time-Course and Regional Analyses of the Physiopathological Changes Induced after Cerebral Injection of an Amyloid _ Fragment in Rats The American Journal of Pathology, 179(1): 315-334). However, the course of the disease is known to run gradually over several years (Tal D.R.; Rüb U; Orantes M; Braak H. 2002 Phases of A_-deposition in the human brain and its relevance for the development of AD. NEUROLOGY. 58:1791-1800) and therefore, the use of the naturally aged animal (mouse) model as shown in the example of the present invention has the advantage that it reproduces the pathological events that characterize Alzheimer's type dementia that develop gradually as they do in humans (Neha, Rupinder K. Sodhi, Amteshwar S. Jaggi, Nirmal Singh. 2014 Animal models of dementia and cognitive dysfunction. http://dx.doi.org/10.1016/j.lfs.2014.05.017. Okawa, O., N. Ohishi, and K. Yagi (1979): Assay of lipid peroxides in animal tissues by the thiobarbituric acid reaction. Anal Biochem. 95 (2): 351 - 58).

It is considered that the statistically significant increase in brain weight of the G3 group with respect to G7 (Table 2) was an unexpected and highly positive result.

Although in the literature it is found that, when treatments whose therapeutic target is the brain are applied, an increase in brain functionality occurs, that may be mediated by an increase in neurogenesis (Kodali *et al.*, 2015), which is not always demonstrable. Therefore, this unexpected finding in one of the compositions corresponds with the cognitive improvement found in the behavioral test.

After the histological analysis performed on the brains of the mice, it was concluded that in 100% of the animals this organ presented a correct morphology in the absence of any tumor mass (CETEX: Scientific Histopathological Report of the Trial Evaluation of the effect of Biocen-128 in naturally aged male BALB/c mice. 2018). Emphasis was placed on the observation of tumor mass, since in the *in vitro* neuronal cell experiments to be described in Example 9, unexpectedly significant cell proliferation was observed, an aspect that was not previously described in previous inventions for some of the proteins and peptides included in the present composition. Therefore, this result corresponds with the cognitive improvement found in the object recognition behavioral test.

These results evidencing cognitive improvement show information that is not evidenced in the international invention document WO 2010/011315 of 2010 because in it, although a composition of thymic peptides is used for the treatment of AD, no concrete results of the application of this compound to eliminate the cognitive deficit are shown.

In summary, the results shown in this experiment demonstrate the possibilities of successful application in humans, where Alzheimer's type dementia occurs in different phases (mild, moderate or acute) (Tal D.R.; Rüb U; Orantes M; Braak H. 2002 Phases of A_-deposition in the human brain and its relevance for the development of AD. NEUROLOGY. 58:1791-1800).

The body weight of 12- to 13-month-old male BALB/c animals both controls and treated with C1 for 6 weeks had significant differences with respect to young control animals (2 to 3 months of age) (p <0.0001). In the group of young animals at the experimental time of 6 weeks, body weight differed significantly from that shown by the animals at the beginning of the experiment. In the rest of the groups, no significant differences were found between the body weight at the beginning of the experiment and that obtained after 6 weeks of treatment (Table 3). In laboratory animals, weight is correlated with age and the results obtained in this experiment correspond to those reported by Animal Resource Center (available at http://www.arc.wa.gov.au/page). In this reference it is stated that young male BALB/c strain mice should gain weight up to 8 months and aged mice should remain stable in the weight range of 28 to 34 g as long as they are healthy. Therefore, the results obtained show that the housing and feeding conditions used were adequate and that the treatment did not affect this parameter.

Table 1. Effect of compositions C1, C2 and C3 administered intranasally for 6 weeks with different frequency of administration on object preference index.

| Group and treatment conditions | Object recognition test results | |
|---|---|---|
| | PI × FO | PI × NO |
| G1 (vehicle, 0.9% NaCl)<br>Daily frequency × 6 weeks | 45.89 ± 6.34 | 54.11 ± 6.34*** |
| G2 (C1: 0.2 mg/mL protein)<br>Daily frequency × 6 weeks | 42.75 ± 6.54 | 57.25 ± 6.54*** |
| G3 (C2: 1.7 mg/mL protein)<br>Daily frequency × 6 weeks<br>Effective quantity: 0.051 mg/30 g<br>(total: 1.5 mg) | 37.16 ± 7.05 | 62.84 ± 7.05*** |

(continued)

| Group and treatment conditions | Object recognition test results | |
| --- | --- | --- |
| | PI $\times$ FO | PI $\times$ NO |
| **G4** (C2: 1.7 mg/mL protein) Alternating frequency $\times$ 6 weeks Effective quantity: 0.051 mg/30 g (total: 1.5 mg) | 41.11 $\pm$ 4.40 | 58.89 $\pm$ 4.40*** |
| **G5** (C2: 1.7 mg/mL protein) Frequency: 2 times per week $\times$ 6 weeks Effective quantity: 0.051 mg/30 g (total: 1.5 mg) | 21.31 $\pm$ 4.46 | 78.69 $\pm$ 4.46*** |
| **G6** (C3: 3.0 mg/mL protein) Frequency: 2 times per week $\times$ 6 weeks Effective quantity: 0.090 mg/30 g (total: 2.7 mg) | 37.06 $\pm$ 3.99 | 62.94 $\pm$ 3.99*** |
| **G7** (vehicle, NaCl al 0.9%) aged mice Daily frequency $\times$ 6 weeks | 61.91 $\pm$ 7.62 | 38.09 $\pm$ 7.62 *** |
| *Significant differences for each group between KO and NO for $p<0.05$ | | |

Table 2. Effect of pharmaceutical composition C1 administered intranasally for 6 weeks on brain weight.

| | **G1** (young mice, vehicle 0.9% NaCl) Daily frequency x 6 weeks | **G7** (aged mice, vehicle 0.9% NaCl) Daily frequency x 6 weeks | **G3** (C3) (1.7 mg/mL protein) Daily frequency x 6 weeks |
| --- | --- | --- | --- |
| Brain weight (%) | 1.765 $\pm$ 0.048 | 1.415 $\pm$ 0.074 | 1.528 $\pm$ 0.061 |

Table 3. Effect of pharmaceutical composition C1, C2 and C3 administered intranasally for 6 weeks with different frequency of administration on body weight.

| Group and treatment conditions | Body weight results | |
| --- | --- | --- |
| | Time 0 | Time 6 weeks |
| **G1** (vehicle, 0.9% NaCl) young mice Daily frequency $\times$ 6 weeks | 21.61 $\pm$ 0.72 | 29.52 $\pm$ 1.83*** |
| **G2** (C1: 0.2 mg/mL protein) Daily frequency $\times$ 6 weeks Effective quantity: 0.006 mg (total: 0.18 mg) | 32.82 $\pm$ 1.35 | 32.94 $\pm$ 1.30 ns |
| **G3** (C2) 1.8 mg/mL protein Daily frequency $\times$ 6 weeks Effective quantity: 0.051 mg (total: 1.53 mg) | 32.41 $\pm$ 1.87 | 31.28 $\pm$ 1.54 ns |
| **G4** (C2) 1.8 mg/mL protein Alternating frequency $\times$ 6 weeks Effective quantity: 0.051 mg (total: 1.53 mg) | 31.72 $\pm$ 1.52 | 33.51 $\pm$ 1.64 ns |

(continued)

| Group and treatment conditions | Body weight results | |
|---|---|---|
| | Time 0 | Time 6 weeks |
| **G5** (C2) 1.8 mg/mL protein Frequency: 2 times per week x 6 weeks Effective quantity: 0.051 mg (total: 1.53 mg) | 31.06 $\pm$ 1.69 | 32.54 $\pm$ 1.64 [ns] |
| **G6** (C3) 3.0 mg/mL protein Frequency: 2 times per week x 6 weeks Effective quantity: 0.090 mg (total: 2.7 mg) | 32.86 $\pm$ 1.31 | 33.06 $\pm$ 1.52[ns] |
| **G7** (vehicloe 0.9% NaCl) aged mice Daily frequency x 6 weeks | 30.86 $\pm$ 2.07 | 31.46 $\pm$1.84 [ns] |
| ns - no significant differences | | |

**Example 2.** Effect of the pharmaceutical composition of the present invention by different routes of administration in aged BALB/c mice

Experimental protocol:

Pharmaceutical composition C4: similar to pharmaceutical composition C2 of Example 1 but different in that the pharmaceutical combination contains group c protein, subsection i (Thymosin Beta 4 at different concentration (0.17 mg/mL)) and is combined with a protein of the same subgroup i with the sequences described: SEC ID NO 29, SEC ID NO 30, and SEC ID NO 31, but methylated at one of its termini, corresponding to Thymosin beta 10 of bovine origin (0.2 mg/mL); in addition, it contains Calmodulin* (0.000004 mg/mL), Keratin (0.00004 mg/mL) and Syntaxin (0.00004 mg/mL).

The effects of the pharmaceutical formulation C4 by intranasal (i.n.), intraperitoneal (i.p.), sublingual and oral routes are compared. For each route a single level of protein concentration in the pharmaceutical composition is used for which the pharmaceutical combination is adjusted to a protein concentration of 1.7 mg/mL in the appropriate vehicle taking into account the formulations. Doses vary according to the route of administration (i.n: 1.5 mg/kg body weight in phosphate buffer vehicle; i.p: 10 mg/kg body weight in 0.9% NaCl; oral: 50 mg/kg body weight in polyvinylbutyl ether and sublingual: 10 mg/kg body weight emulsified with Plukenetia volubilis oil). In this experiment a similar design to experiment 1 is used but 6 treatment groups are formed, two of the groups (young control-G1 and aged control-G2) receive 0.9% NaCl and the remaining four groups are treated with the pharmaceutical composition C4 every other day for 6 weeks. The response to treatment is analyzed by the spontaneous alternation test.

Parameters to be evaluated:

Y Maze: An attachment of three arms of equal length (30 cm), interconnected with each other at an angle of 120°, is used. Each arm is identified with a different letter (A, B, C). The test is run for 7 min and at the beginning of the test the animal is placed in the center of the maze. An entry is considered when all four legs are inside one of the arms. An alternation is considered when the animal visits the three arms of the maze consecutively, without repeating the entry to the arm it visited previously. In addition, the number of arm entries is used as an indicator of locomotor activity. From the number of alternations, the variable alternation percentage is calculated by the following expression:

$$Alternation\ (\%) = \frac{Alternations}{Total\ of\ posible\ alternations}\ X\ 100$$

where: Total of posible alternations = Total of entries - 2

Results:

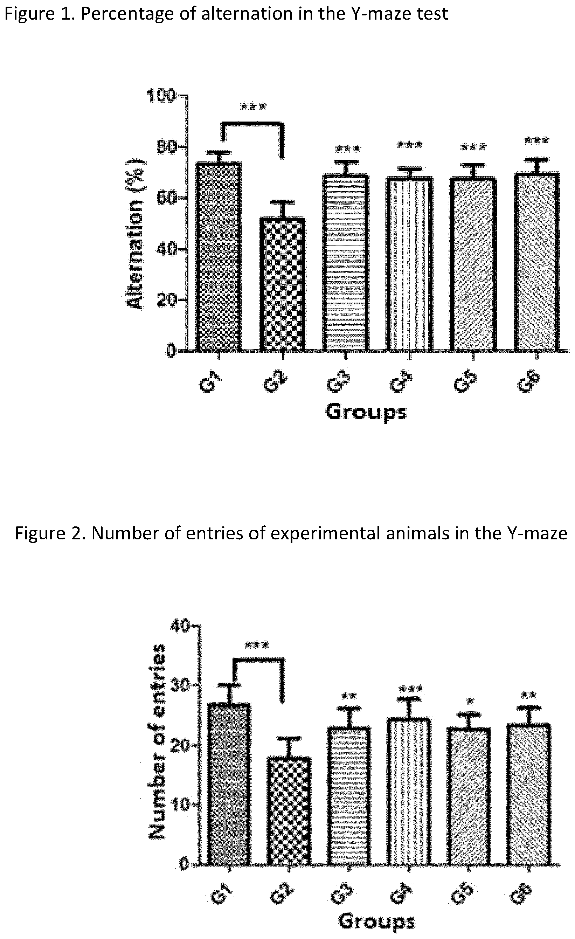

Figures 1 and 2 show that the C4 formulation by the four routes of administration (i.n., i.p., sublingual and oral) when administered daily, every other day or 2 times per week, achieves improvement of cognitive function in mice presenting deficiency. The results obtained in the Y maze test in this experimental procedure demonstrate the superiority of the treated groups (G3 to G6) significantly in the percentage of alternation (Figure 1) and the number of alternations (Figure 2) with respect to the aged animals (G2), in addition to presenting a similar behavior to the young controls (G1) without showing significant differences. These results suggest that the mice treated with the pharmaceutical composition of the present invention remember better the last arm visited than the aged controls and, in addition, suggest that the treatment manages to improve the ability to orient in space, a parameter that deteriorates in mice as they age and that is satisfactory in young animals without being submitted to any drug or treatment (Hiramitsu, M., O. Takiguchi, A. Nishiyama, and M. Hiromasa (2010): Cilostazol prevents amyloid β peptide induced memory impairment and oxidative stress in mice. BJP. 161: 1899-1912). Therefore, based on the memory mechanism assessed in the test, i.e., spatial short-term working memory, it can be stated that a cognitive improvement is obtained with the treatment.

This demonstrates the versatility of the formulation, which can be administered by different routes.

**Example 3.** Effect of pharmaceutical composition on antioxidant activity

Experimental protocol:

Pharmaceutical composition C5: Similar to pharmaceutical composition C2 of Example 1, with the difference that within the pharmaceutical combination the concentration of the proteins dissolving in the selected delivery vehicle are: from group b: Ubiquitin (0.51 mg/mL), small Ubiquitin fold-modifier Ufm1* (0.34 mg/mL), small Ubiquitin fold-modifier Ufm2 (0.34 mg/mL), Peptidyl-prolyl-cis-trans isomerase H (0.000017 mg/mL), Cystatin B (0.000017 mg/mL), a peptide comprising the NSFL1 Cofactor P47 containing in its molecule the sequences SEC ID NO 25 and SEC ID NO 26 (0.000017 mg/mL) and a peptide comprising the UV excision repair protein RAD23 homolog B containing the sequences SEC ID NO 27 and SEC ID NO 28 (0.000017 mg/mL); group **c** protein (identified as Thymosin beta 4) has a concentration of 0.493 mg/volume. The concentration of the rest of the proteins is 0.000034 mg/mL. The total protein concentration of the combination in the appropriate vehicle is adjusted to obtain the pharmaceutical composition with 1.7 mg/mL of total proteins.

The effects of the i.n. formulation C5 are compared for the pharmaceutical composition protein concentration of 1.7 mg/mL, administered at alternating frequency for 6 weeks. In this experiment a similar design to experiment 1 is used, but 3 treatment groups are formed, two of the groups (young control and aged control) receive 0.9% NaCl and the remaining group is treated with the pharmaceutical composition C5 every other day for 6 weeks. The response to treatment is analyzed using the lipid damage indicator Thiobarbituric Acid Reactive Substances (TBARS) and protein carbonylation as an indicator of protein damage.

Parameters evaluated:

Brain homogenates are prepared in Tris buffer at pH 7.6 containing 0.32 M of sucrose, 10 mM of Tris and 1 mM of EDTA, and differential centrifugation is performed until the postmitochondrial fraction is obtained, according to the method described by Uwe and Von Hagen (Uwe, M. and J. Von Hagen (2009): Isolation of subcellular organelles and structures. Methods Enz. 463: 305-326). The supernatant is dispensed into 1 mL aliquots, which are stored at -80 °C. Oxidative damage to lipids and proteins is measured in these homogenates.

Quantification of oxidative damage to lipids is performed using the TBARS quantification method according to Okawa *et al* (Okawa, O., N. Ohishi and K. Yagi (1979): Assay of lipid peroxides in animal tissues by the thiobarbituric acid reaction. Yagi (1979): Assay of lipid peroxides in animal tissues by the thiobarbituric acid reaction. Anal Biochem. 95 (2): 351 - 58). The method consists of the spectrophotometric measurement of substances reacting with thiobarbituric acid (TBA), which is based on the stoichiometric reaction of two molecules of TBA with one molecule of malondialdehyde (MDA), which is one of the products of the chain reaction called lipid peroxidation. The reaction of MDA with TBA forms a pink compound which has its absorbance maximum (ABS) at a wavelength of 532 nm in acidic solution. TBARS are determined from the molar extinction coefficient of MDA ($^\wedge=155$ mM$^{-1}$cm$^{-1}$) and the protein concentration in the tissue homogenate which is determined by the modified Lowry method for biological tissues (Upreti *et al*., 1988).

Quantification of oxidative damage to proteins is performed using the method described by Resnick and Parker (Resnick, A.Z. and L. Packer (1994): Oxidative damage to proteins: Spectrophometric method for carbonyl assay. Methods Enz. 233: 357 - 63). The method is based on the reaction of 2,4 dinitrophenyl hydrazine with the carbonyl groups of damaged proteins. The amount of proteins with carbonyl groups is determined from the maximum abosrbance (ABS) measured at 360 nm; and the molar extinction coefficient of hydrazine ($\xi=22\,000$ nM cm$^{-1}$mL$^{-1}$). On the other hand, the protein concentration in the samples is determined by extrapolating the ABS at 280 nm on a Bovine Serum Albumin (BSA) curve, at concentrations of 0.2 to 2.0 mg/mL. Finally, the result is expressed as the ratio between the molar concentration of carbonyl groups and the concentration of proteins present in the sample.

Results:

Table 4 shows that the C5 formulation i.n. for the extract concentration of 1.7 mg/mL, when administered every other day for 6 weeks, achieves a decrease in oxidative damage appearing in the brain of aged mice aged 12 to 13 months. In the G2 group, a significant increase in damage to both lipids and proteins in the brain was obtained with respect to the G1 group. In the case of the treated group, there was a significant decrease in damage with respect to the aged controls and no differences were found with respect to the young control animals. The decrease in oxidative damage to proteins in the brain homogenate detected in the G3 group can be considered as a very positive and unexpected effect of the treatment and, according to the literature, this damage is described as a physiological event associated with the aging process (Myhre, O., H. Utkilen, N. Duale, G. Brunborg and T. Hofer (2013): Metal dyshomeostasis and Inflammation in Alzheimer's and Parkinson's diseases: Possible impact of environmental exposure. http://dx.doi.org/10.1155/2013/726954 (Accessed 8-07-2016) and therefore decreasing it constitutes a benefit. In addition, oxidative damage to proteins may be a crucial factor in aging, since oxidized proteins lose structural and catalytic integrity, and are therefore more susceptible to hydrolyzation (Ramirez-Ramirez, D., J. Valenti-Perez, M. Garcia, Z. Batista and J. Estrada (2013): Oxidative stress in aged rats. Finlay Journal. 3(4)).

The elements present in the pharmaceutical composition claimed separately cannot achieve this effect, only a combination of the described proteins can achieve it; for example, combining especially the protein of group **e** (ATOX 1) which intervenes in the transport of copper (pro-oxidant) with Thioredoxin (being an oxireductase), with cytochrome C and with the Cytochrome C oxidase subunit NDUFA4, all belonging to group **a,** could explain this effect by combining them with those of group **b,** which contributes to the protective effect in the elimination of damaged proteins and other toxic substances derived from oxidation inside the cells.

Thus, the beneficial effect of the pharmaceutical composition in the treatment of diseases such as, but not limited to AD, Leigh's disease, multiple sclerosis and amyotrophic lateral sclerosis is demonstrated.

Table 4. Effect of the pharmaceutical composition C5 on antioxidant activity

| Oxidative damage | G1 (vehicle, 0.9% NaCl) young mice | G2 (vehicle, 0.9% NaCl) aged mice | G3 (C5) Dose 1.5 mg/kg via i.n. Effective amount 0.025 mg |
|---|---|---|---|
| Carbonylated proteins (nmoles/mg protein) | $1.006 \pm 0.17$ | $2.742 \pm 0.456$*** | $1.359 \pm 0.1994\Delta\Delta$ |
| TBARS (nmoles MDA/mg protein | $5.525 \pm 1.210$ | $9.538 \pm 1.041$*** | $6.726 \pm 1.021\Delta\Delta\Delta$ |
| * Significant differences with respect to G1. $\Delta$ Significant differences with respect to G2 | | | |

**Example 4.** Effect of the pharmaceutical composition on lipid metabolism

A pharmaceutical composition C6 similar to pharmaceutical composition C2 of Example 1 is tested, but different in that in the pharmaceutical combination the group **c** protein, subgroup i, identified as Thymosin beta 4 (0, 476 mg/mL) is combined with the group **c** protein, subgroup ii with 2 sequences of 7 and 14 amino acids (SEC ID NO 32) and (SEC ID NO 33), identified by mass spectrometry and with the aid of the Swiss Prot sequence database as Thymosin alpha 7* (0.017 mg/mL).

Experimental protocol:

The effects of the pharmaceutical combination C6 via i.n. for the pharmaceutical composition concentration of 1.8 mg/mL, alternating frequency for 6 weeks, are compared. In this experiment a similar design to experiment 1 was used but 3 treatment groups were formed, two of the groups (young control and aged control) received 0.9% NaCl and the remaining group was treated by C6 every other day for 6 weeks. The response to treatment was analyzed by quantification of triglycerides and cholesterol.

Parameters evaluated:

Triglyceride concentration is determined according to the methodology described by Morejón and Triana (2015), in which the Monotriglitest reagent set (HELFA® Diagnósticos, Cuba) is used. Cholesterol concentration is also determined using the Colestest reagent set (HELFA® Diagnósticos, Cuba).

Results:

The result obtained with the analysis of serum triglyceride concentration unexpectedly shows that the treatment has a positive effect in the G3 group in which a significant decrease of this parameter is obtained with respect to G2 (Table 5). According to Araki *et al.* (Araki, S., M. Okazaki and S. Goto (2004): Impaired lipid metabolism in aged mice as revealed by fasting-induced expression of apolipoprotein mRNAs in the liver and changes in serum lipids. Gerontology. 50(4): 206-215) who compared young mice aged 6 to 8 months and aged mice aged 24 to 28 months, in the aged animals lipid mobilization is impaired with age due to decreased ApoE gene expression. This leads in the long term to excessive lipid accumulation in tissues such as liver, adipose tissues and blood vessels and results in an increased incidence of age-related diseases.

After consulting the patent sources by the authors of the present invention, it did not turn out that this finding of decreased triglyceride content was obvious, since in the present pharmaceutical composition no known active principle of any drug described for this purpose is employed, nor were reports found of a combination of proteins and peptides such as those claimed in the present invention with such beneficial effect.

As previously stated, in neurological disorders and especially in AD, hypercholesterolemia constitutes a risk factor causing the appearance of beta amyloid plaques and neurofibrillary tangles.

In this way, the combination of the protein of group e, ATOX 1, with those of the other groups, especially those of group b, prevents the formation of Aβ: copper complexes that can catalytically oxidize cholesterol and generate $H_2O_2$, oxysterols and other products of lipid peroxidation.

By way of summary it can be stated that the pharmaceutical composition is suitable for the treatment of diseases originating from or leading to imbalances in lipid metabolism, especially excess cholesterol and increased triglycerides, such as but not limited to AD and rheumatoid arthritis.

Table 5. Effect of pharmaceutical composition C6 on lipid metabolism

| Concentration in serum (mmol/L) | G1 (vehicle, 0.9% NaCl) young mice | G2 (vehicle, 0.9% NaCl) aged mice | G3 (C6) Dose 1.5 mg/kg via i.n. Effective amount 0.025 mg (total: 0.75 mg) |
|---|---|---|---|
| Triglycerides | 1.372 ± 0.271 | 2.679 ± 0.297*** | 1.671 ± 0.114 Δ Δ Δ |
| Cholesterol | 2.115 ± 0.267 | 3.129 ± 0.307*** | 2.583 ± 0.256 Δ Δ |
| * Significant differences with respect to G1. Δ Significant differences with respect to G2 | | | |

**Example 5**

In this example, the effect of pharmaceutical combinations is studied in aged C57BL/6 mice, for which the pharmaceutical combination of pharmaceutical composition C2 of Example 1 is modified by employing only 0.17 mg/mL of Ubiquitin, 0.153 mg/mL of the small Ubiquitin fold-modifier Ufml, 0.152 mg/mL of the Small Ubiquitin fold-modifier Ufm2, combining it with Erythropoietin* (0.85 mg/mL) to form pharmaceutical composition C7 and adjusting the total protein concentration in the vehicle to 1.7 mg/mL and combining it with Calmodulin (0.000017 mg/mL), Replication Factor (0.000017 mg/mL), Keratin (0.000017 mg/mL) and Syntaxin (0.000017 mg/mL) to form the pharmaceutical composition C8 and adjusting the total protein concentration in the pharmaceutical formulation to 1.7 mg/mL of the vehicle).

Experimental protocol

Female C57BL/6 mice aged 14 to 15 months (weighing 24 to 32 g), as well as young animals aged 3 to 4 months (20 to 24 g) are used. The animals are kept in a conventional room with a controlled environment (type IV minimum security barrier), with a temperature of 21 ± 3 °C and relative humidity between 40% to 70%, with 18 air changes per hour, 100% injection of outside air with 85% filtration and 12 hours of light and 12 hours of darkness, which is automatically controlled. The mice are fed with EAO1004cenp diet for rodents ad libitum and drinking water.

Treatment

The aged animals are distributed in 3 groups of 10 animals each (G2 to G4) and the young animals are grouped in G1. In group G3 the C7 formulation composed of the pharmaceutical composition C2 with Erythropoietin is evaluated and in group G4 the C8 formulation composed of a combination of the pharmaceutical composition C2 with Calmodulin, Replication Factor, Keratin and Syntaxin is evaluated. In both groups the treatment is administered in a volume of 30 μL, i.n., for an extract concentration of 1.8 mg/mL, alternating frequency for 6 weeks. For groups G1 (young) and G2 (aged) 0.9% NaCl solution is used as a vehicle. The response to treatment is analyzed by the Morris water maze test and the pain sensitivity test.

Parameters evaluated

Morris water maze test: it was performed in a plastic tank of 120 cm in diameter, inside which a plastic platform is placed, whose upper base is square. The test consists of 2 phases: a training phase lasting three days with 4 trials from the same position per day per animal and a holding phase on the fourth day without a platform. The platform is kept in a fixed location during the 3 training days and the animal is placed with its back to the platform, i.e. with its face facing the tank wall. In each training section the animal is allowed to swim for 90 s until it finds the platform. The animal is allowed to stand on the platform for a period between 10 and 30 s. On the fourth day of the test the platform is removed from the tank and all animals are placed in the same position to swim in the tank for 60 s. The time that each animal remains in each of the 4 quadrants and the time it takes to go for the first time to quadrant 1, where the platform is located (latency period), is counted with the help of the video player.

Pain sensitivity test by hot iron: A rectangular stainless steel base box with transparent plastic walls and roof was used so that the animal's behavior can be observed inside. The box contains an electronic circuit that allows the base of the box to be heated to a temperature of 50 °C to 55 °C as selected. The base of the box was heated to a temperature between 50 °C and 51 °C. Subsequently, the animal was placed inside the box and the timer was set to stop at the moment the animal jumped or licked its hind leg for the first time. The period of time elapsed between the start of the test and that behavior is considered the test response variable (latency).

Results

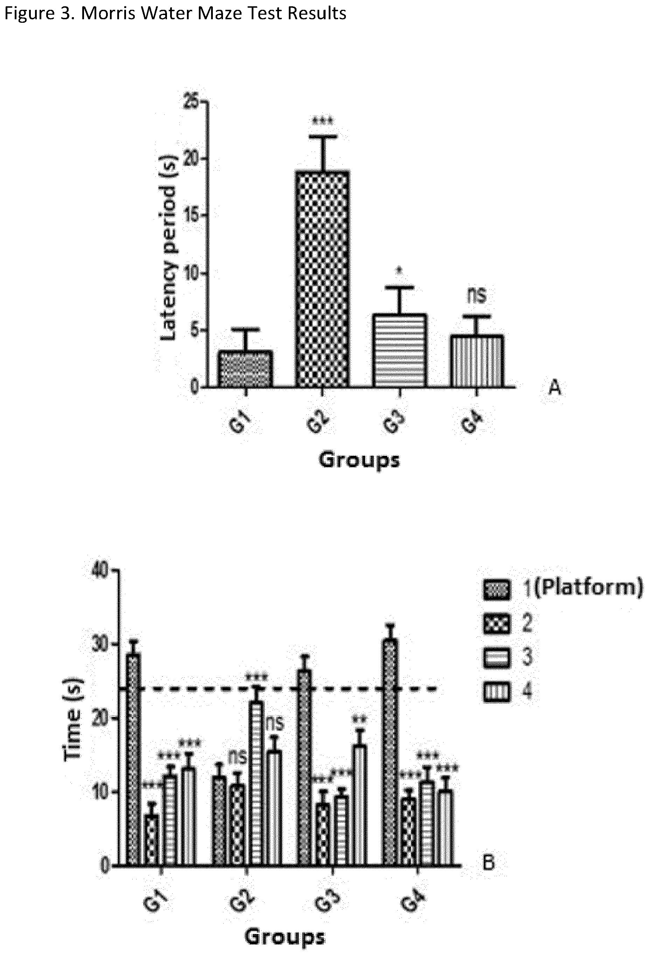

At the end of 6 weeks of treatment, the pain sensitivity test and the Morris water maze test, which evaluates cognitive improvement, are applied. In the latter, the aged control mice (G2) take longer to find the quadrant of the platform than the rest of the animals (Figure 3A). These results demonstrate the cognitive improvement in the treated groups in terms of the latency period during the acquisition phase. These animals take less time to visit the quadrant of the platform for the first time, even in the G4 group treated with C8 there are no significant differences with respect to the young animals, and in the G3 group, although this variable does not behave as in the young animals, it is significantly less than in the aged control group. The fact that the aged animals show the worst performance with respect to this variable has been described in the literature and D'Hooge R. and De Deyn P.P. (D'Hooge R., De Deyn P.P. Applications of the Morris water maze in the study of learning and memory. Brain Research Reviews 36 (2001) 60 -90. Fontaine, S.N., et al., (2015). Cellular factors modulating the mechanism of tau protein aggregation. Cell. Mol. LifeSci. 72, 1863-1879) raised that, as animals age, they show a decline in cognitive functions and have a decrease in the performance efficiency of this behavioral test. This finding is due to the fact that these animals change their swimming, locomotion and exploration skills. It has been proposed that these facts coincide with structural and physiological modifications in some brain structures, specifically in the hippocampus, related to spatial learning.

As a result of the analysis of the time spent in each quadrant (Fig. 3B), the presence of cognitive impairment in the aged mice and the improvement appearing in the animals treated with the pharmaceutical composition according to the present invention in both combinations C7 and C8 are confirmed. Animals in groups G3 and G4 behave similarly to young controls. The longer stay in the target quadrant by animals in the treated groups shows that these animals perform better during the retention phase and that treatment with both formulations has a positive effect. This is a spatial preference test in which, if the animal has learned in the final phase of the test, it will swim longer in the target quadrant, i.e., where the platform was previously located, because the memory mechanisms associated with retention will have been correctly activated (D'Hooge R., De Deyn P.P. Applications of the Morris water maze in the study of learning and memory. Brain Research Reviews 36 (2001) 60 -90. Fontaine, S.N., et al., (2015). Cellular factors modulating the mechanism of tau protein aggregation. Cell. Mol. LifeSci. 72, 1863-1879).

In the case of the hot plate test to evaluate pain sensitivity (nociception) due to the effect of heat, it was observed that the untreated aged control animals (G2) responded in a significantly longer time to heat than the young control animals (G1) (Fig. 4). In the case of animals treated with C7 and C8 formulations on alternate days (G3 and G4), the response time to heat was significantly shorter than in the G2 group, while G3 showed no difference with respect to the G1 group.

The results in the heat pain sensitivity test (Fig. 4) for the G2 group can be explained by the fact that during the natural aging process that accompanies AD, a systemic inflammatory process develops that decreases antinociception in aged animals and it is assumed that this process is mediated by the distortion of the cortisol-mediated hormonal system (King-Himmelreich TS., Möser CV, Wolters MC, Olbrich K, Geisslinger G, Niederberger E. Age-Dependent Changes in the Inflammatory Nociceptive Behavior of Mice. Int. J. Mol. Sci. 2015, 16, 27508-27519; doi:10.3390/ijms161126041). The result for groups G3 and G4 indicates that treatment with the pharmaceutical composition of the present invention, both combining the pharmaceutical composition C2 with Erythropoietin and when C2 is combined with Calmodulin, Replication Factor, Keratin and Syntaxin, has a favorable effect in terms of decreasing the inflammatory process, which allows recovering the animal's response to pain similarly to the young control group.

It is thus reaffirmed the systemic anti-inflammatory activity of the pharmaceutical composition and the very significant cognitive improvement, both beneficial effects for the treatment of dissimilar diseases, especially those described as therapeutic targets in this invention. Among those that occur with cognitive impairment as an example, but not exclusively, AD, Leigh's Disease, epilepsy, ischemia, sclerosis and encephalopathy and among those that occur with inflammatory processes, as an example but not limitedly, AD, multiple sclerosis, amyotrophic lateral sclerosis, retrobulbar neuritis, polyneuropathies, systemic lupus erythematosus, rheumatoid arthritis, juvenile idiopathic arthritis, ankylosing spondylitis, rheumatic fever, Behcet's syndrome, iridocyclitis, psoriasis, ulcerative colitis, Crohn's disease, irritable bowel syndrome, rhonosinusitis, atopic dermatitis and asthma.

**Example 6**

Effect of pharmaceutical composition C8 on inflammation in OF1 mice by the chronic inflammation model of cotton-pellet induced granuloma test.

Pharmaceutical composition C8 is similar to C2, with the difference that the total protein content is 1.0 mg/mL, and in it are combined all proteins of groups a, b, d, e, f, that of subgroup i of group c and protein subgroup iii Thymopoietin, with amino acid sequences in its molecule SEC ID NO 34, SEC ID NO 35 and SEC ID N. 36 (0.00034 mg/mL).

Experimental protocol

Male mice of the OF-1 line, from CENPALAB (Havana, Cuba), with an average weight of 20.2 g $\pm$ 1.5 g at the beginning of the experiment, are used. The animals are housed under the conditions described in example 1. The implantation of the cotton pellet is performed according to the methodology described by Bertollo *et al.* in 2006. (Bertollo CM, Oliveira AC, Rocha LT, Costa KA, Nascimento EB, Jr., Coelho MM: Characterization of the antinociceptive and anti-inflammatory activities of riboflavin in different experimental models. Eur J Pharmacol 2006, 547(1-3):184-191.BioSystems NCBI. Consulted 20.09.2019 in https://www.ncbi.nlm.nih. gov/biosystems/1457777?Sel=geneid:820#show=genes2007). Three groups of 10 animals each were formed: G1 Vehicle (administered with 0.85% sterile saline solution i.p.); G2 Positive control, administered with dexamethasone orally at a dose of 3 mg/kg body weight and G3, with the pharmaceutical composition C8 at a dose of 10 mg/kg i.p. Subsequently, the animals were anesthetized by i.p. administration of sodium thiopental (40 mg/kg, 0.01 mL/g). Each animal underwent an incision in the lateral dorsal half and a 10 mg cotton pellet, previously sterilized, was implanted subcutaneously in the dorsal region under aseptic conditions. At the end of the operation, the animals are placed in their boxes. After 24 h, i.p. administration is started, at the rate of one daily administration in the morning, during 2 days after the implantation of the cotton pellet. Meanwhile, group G2 is administered dexamethasone during the 7 days of the experiment.

Parameters to be evaluated

On the eighth day of the experiment the animals are anesthetized under the same conditions as described above and the granulomas are carefully removed together with the connective tissue formed. The weight of the wet and dry granuloma is calculated and expressed in mg $\times$ 100.

Results

The administration of the pharmaceutical composition formulation C8 shows a significant decrease in the wet and dry weight of the granuloma (G3) compared to the control group (G1) and shows no difference with the positive control which was administered dexamethasone (G2) (Table 6). The model of granuloma induction by implantation of cotton pellets is considered classic for the study of the transudative (edema) and proliferative (granulomatous tissue) components of the chronic inflammatory process (Swingle KF, Shideman FE: Phases of the inflammatory response to subcutaneous implantation of a cotton pellet and their modification by certain anti-inflammatory agents. J PharmacolExpTher 1972, 183(1):226-234). In this model it is described that granuloma formation is initiated by the presence of the antigen (cotton pellet) which causes stimulation of the immune system; production of antibodies, interleukins and complements, which, under persistent antigenic stimulation, contribute to the proliferation and formation of granulomatous tissue around the cotton pellet (Spector WG, Heesom N: The production of granulomata by antigen-antibody complexes. J Pathol 1969, 98(1):31-39). These results are unsolved elements in the Russian invention document RU2017115294 since it reports a peptide with antidepressant activity and for the cure of AD, but no experiments are shown to justify the anti-inflammatory activity that is characteristic of this disease, as well as of other pathological processes that occur during aging and other neurological, immunological, autoimmune disorders, among others.

Table 6. Effect of pharmaceutical composition C8 on inflammation caused by cotton pellet-induced granuloma.

| Parameters | G1 (vehicle, 0.9% NaCl) | G2 Positive control Dexamethasone (3 mg/kg) | G4 (C8) 10 mg/kg dose via i.p. |
|---|---|---|---|
| Weight of wet granuloma (mg x 100) | $10.40 \pm 0,62$ | $6.32 \pm 0.84$*** | $6.66 \pm 0.89$*** |
| Weight of dry granuloma (mg x 100) | $2.02 \pm 0,29$ | $1.23 \pm 0.26$*** | $1.18 \pm 0.19$*** |
| *- Significant differences with respect to group G1 (vehicle) | | | |

**Example 7**

Effect of the pharmaceutical composition on the modulation of the immune response. Pharmaceutical composition C9 is tested in experimental mice. This pharmaceutical composition is similar to C2 of example 1 with the difference that a total extract of thymus is used containing all the proteins described in group c, these being found as a whole in an amount of 0.37 mg/mL (determined by the Lowry method) in relation to the total proteins of the pharmaceutical composition.

Four groups of animals were formed, three with aged BALB/c mice (16 to 17 months old) and one with young animals (2 to 3 months old). The group of young animals (G1) as well as one of the groups of aged animals (G2) received 0.9% NaCl (placebo). Meanwhile, the remaining two groups of aged animals receive treatment with the experimental pharmaceutical composition, intranasally (i.n) at a dose of 1.69 mg/kg of body weight, at a daily frequency for 6 or 10 weeks (G3 and G4, respectively).

Assay protocol and results of immune response modulation. Spleens and thymuses are surgically removed from at least 5 mice per group. Each spleen is processed independently. Thymus is collected from each animal and a pool is formed from each group. Organs are preserved in phosphate buffered sodium chloride solution (PBS) on ice until processing. The organs are macerated and 2 mL of PBS is added to obtain the cell suspension. Additional 3 mL of PBSA is added to the cell suspension obtained and filtered through a 40 $\mu$m pore diameter filter (BD Falcon) into a 15 mL Corning tube. The macerated and filtered organs are centrifuged at 1700 rpm in the refrigerated centrifuge for 5 min. The supernatants obtained are separated and the cell pellet is resuspended in 1 mL of erythrocyte lysis solution (0.83% NH4Cl). Cells are incubated for 5 min at room temperature; 20 mL of PBS is added to each tube and again centrifuged at 1700 rpm for 5 min. The supernatant is separated and again 20 mL of PBS buffer is added to each tube, again centrifuged at 1700 rpm for 5 min. This cell washing operation is repeated a third time. Subsequently, 2 mL of PBS with 1% bovine serum albumin (BSA) is added to the set of cells obtained in the precipitate in the tubes. The obtained suspension is again filtered through a 40 $\mu$m pore diameter filter (BD Falcon) and the resulting filtrate is kept on ice at 2 °C to 8 °C overnight.

For the identification of the different T and B lymphocyte populations in the different tissues to be analyzed, the previously obtained cell suspensions are used. The cells ($2 \times 10^6$/tube) are incubated for 30 min at 4 °C with 500 $\mu$L of blocking solution, consisting of phosphate-buffered saline solution (NaCl: 0.27 mol/L; KCl: 0.005 mol/L; $Na_2HPO_4$: 0.016 mol/L; $KH_2PO_4$: 0.003 mol/L) (SSTF) supplemented with 1% bovine serum albumin (BSA, Sigma-Aldrich) and mouse serum (1: 20, v:v) to block antibody constant region receptors. Cells are centrifuged at 200 g for five minutes and labeled for 30 minutes at 4 °C with 500 $\mu$L of a mixture of fluorophore-conjugated antibodies diluted in 1% SSTF-ASB. The mixtures used are:

- for T lymphocytes: anti-CD4 FITC (1:200 dilution, v:v), anti-CD3 PE (1:600 dilution, v:v), anti-CD8 ECD (1:200 dilution, v:v) and anti-CD45 PECy5 (1:600 dilution, v:v).
- for B lymphocytes: anti-B220 FITC (1:200 dilution, v:v), anti-CDS PE (1:600 dilution, v:v), anti-CD45 PECy5 (1:600 dilution, v:v) and anti-CD23 PECy7 (1:400 dilution, v:v).

Results

Figure 5 shows the influence of the experimental pharmaceutical composition on the CD3- and CD3+ thymus lymphocyte population. When analyzing the CD3- subpopulations, no major differences between the placebo-treated groups (young and aged) were observed. However, in the aged animals treated with the pharmaceutical composition it is shown that, in a dose-dependent manner, an increase in CD3-CD4-CD8- triple negative (TN) subpopulations, a decrease in CD3-CD4+CD8+ subpopulations and an increase in CD3-CD8+ cells occur.

In the thymus under the influence of the stromal microenvironment, immature TN thymocytes acquire the surface molecules CD3, CD4 and CD8. During the maturation process of T cells, subpopulations with differentiated expression of these molecules are generated (Sen M. A, Lieberman M, Alpert S, Weissman IL, Small M. Differentiation of CD3 - 4 - 8- Thymocytes in Short-Term Thymic Stromal Cell Culture. J. Exp. Med 1992;176:543-55; Galy A, Verma S, Bárcena A, Spits H. Precursors of CD3 + CD4 + CD8 + Cells in the Human Thymus Are Defined by Expression of CD34. Delineation of Early Events in Human Thymic Development. J. Exp. Med 1992; 178:391-401). The findings in the treated animals demonstrate the effects of the pharmaceutical composition on thymocyte maturation, which is favorable considering the physiological involution that occurs in this gland with aging, with the inexorable loss of T-cell subpopulations.

In relation to the CD3+ subpopulations, differences are shown between the groups of young and aged animals, with a decrease in CD3+CD4+ T lymphocytes in the aged animals, which has been reported as part of the changes that occur with aging in mice (Scott Hale J, Boursalian TE, Turk GL, Fink PJ. Thymic output in aged mice. PNAS 2006;103 (2):8447-8452) and also in humans (Weinberger B, Lazuardi L, Weiskirchner I, Keller M, Neuner C, Fischer KH, et al. Healthy aging and latentinfection with CMV lead to distinct changes in CD8+ and CD4+ T-cell subsets in the elderly. Hum Immunol 2007, 68(2):86-90; Garcia B, Saavedra D, Lorenzo-Luaces P, Badia TJ, Leonard I, Mazorra Z, et al. Immunosenescence and gender: a study in healthy Cubans. Immunity & Ageing 2013, 10:16). In addition, when comparing aged animals with young animals, an increase in CD4/CD8 double positive (DP) T cells was observed. The number of CD4/CD8 DP T cells varies between species, being found in lower proportion in humans and mice relative to pigs and chickens. In humans, it has been shown to increase with age, and its increase has been related to thymic involution (Overgaard N.H, Junf JW, Steptoe RJ, Wells JW.CD4+/CD8+ doble-posiive T cells: more than just a development stage.J Leucocyte Biol 2015;97:31-37). In the treated groups, an increase of CD3+CD4-CD8- T lymphocytes is evidenced in relation to the controls, which correspond to thymocytes in initial stages of the maturation process (Abbas AK, Lichtman AH, Pillai S, editors. Desarrollo del linfocito y reordenamiento del gen del receptor para el antigeno. In: Inmunologia Celular y Molecular. 9na ed. Madrid: ELSEVIER; 2018) which shows that the formulation is stimulating the process of generation and maturation of T cells at the level of the thymus.

Figure 6 also shows the influence of the experimental pharmaceutical composition on the CD3+ lymphocyte population, but in the periphery, when analyzed from the spleen homogenate of the animals of the experimental groups. Differences are evident between the groups of young and aged animals, with a decrease in CD3+CD8+ T lymphocytes in the aged animals, while in the animals treated with the experimental pharmaceutical composition there was an increase in both CD4+ and CD8+ T cells with both treatment schemes used in the experiment, although not in a linear fashion.

Figure 7 shows a graph with the results of the study of the influence of the experimental pharmaceutical composition on the population of T lymphocytes in spleen homogenate measured by flow cytometry. A decrease of CD3+CD45+ cells is observed in the aged animals, these cells increase in the animals subjected to the lower dose treatment. CD45 molecules play an important role in the regulation of T lymphocyte proliferation and differentiation. Mouse CD45 (also known as Ly5 and leukocyte common antigen) is a 180 to 220 kDa variable glycosylated member of the class 1 subtype of the protein tyrosine phosphatase family. It is synthesized as a 1291 amino acid (aa) precursor containing a 23 aa signal sequence, a 541 aa extracellular domain (DEC), a 22 aa transmembrane segment and a 705 aa cytoplasmic region. The DEC is encoded by exons 4 to 16 of the CD45 gene. Alternative splicing of exon 4 (or A) (aa 30 to 74), exon 5 (or B) (aa 75 to 123) and exon 6 (or C) (aa 124 to 169) define different lymphocyte populations and functional stages. Virgin T cells express exon 5 (CD45 RB), whereas activated T cells do not express exon 4, 5 or 6 (CD 45 RO) (Nishimura H, Hattori S, Abe M, Ueda G, Okamoto H, Tsurui H, Hirose S, Shirai T. Differential expression of three CD45 alternative structures on murine T cells: exon 6-dependent epitope as a marker for functional heterogeneity of CD4+ T cells. Int Immunol. 1992 Aug;4(8):923-30). In this experiment, cells were not labeled with antibodies specific for the different CD45 isoforms.

It can be seen that the aged animals have a higher proportion of CD3+CD4+ T lymphocytes and a lower proportion of CD3+CD8+ in relation to the young animals, with an increase in the latter occurring with the lower dose treatment scheme. The impairment of CD4+ and CD8+ cell subpopulations with senescence has been widely discussed, with different results in several studies. Age-related changes in CD4 T cells have been less well documented than in CD8 cells (Garcia B, Saavedra D, Lorenzo-Luaces P, Badia TJ, Leonard I, Mazorra Z, et al. Immunosenescence and gender: a study in healthy Cubans. Immunity & Ageing 2013, 10:16). The existence of a more effective homeostatic control mechanism that allows the maintenance of an appropriate number of virgin CD4+ T cells in the elderly, in contrast to the CD8+ compartment, is proposed, with evidence of a decrease in virgin CD8+ cells as early as middle age (Goronzy JJ, Lee WW, Weyand CM.Aging and T-cell diversity. Exp. Gerontol 2007;42 (5), 400-406).

Some authors have observed in the elderly a dramatic decrease in peripheral virgin T cells in both CD4+ and CD8+ subsets (Scott Hale J, Boursalian TE, Turk GL, Fink PJ. Thymic output in aged mice. PNAS 2006;103 (2):8447-8452) while in other cases the evidence has been towards the decrease of "naive" CD4+ T cells (Larbi A, Pawelec G, Witkowski JM, Schipper HM, Derhovanessian E, Goldeck D,et al. Dramatic shifts in circulating CD4 but not CD8 T cell subsets in mild Alzheimer's disease. J. Alzheimers Dis 2009; 17 (1): 91-103) and in other studies the changes have only been significant for CD8+ decline. Most published studies document a decrease in the number and proportion of "naive" CD8 T cells in aged individuals along with a reciprocal increase in CD8+ T-cell memory (Goronzy JJ, Lee WW, Weyand CM.Aging and T-cell diversity. Exp. Gerontol 2007;42 (5), 400-406; Yan J, Greer JM, Hull R, O'Sullivan JD, Henderson RD, Read SJ, McCombe PA: The effect of aging on human lymphocyte subsets: comparison of males and females. Immun Ageing 2010; 7:4; Wikby A, Johansson B, Olsson J, Lofgren S, Nilsson BO, Ferguson F: Expansions of peripheral blood CD8 T-lymphocyte subpopulations and an association with cytomegalovirus seropositivity in the elderly: the Swedish NONA immune study. Exp Gerontol 2002, 37(2-3):445-453), demonstrating in this case, a positive influence of the experimental pharmaceutical composition on CD8+ lymphocytes.

Figure 8 shows the influence of the experimental pharmaceutical composition on the subpopulation of CD45+B220+ cells in the thymus. With treatment there is evidence of an increase in cells with expression of these markers, which are recognized to be expressed in a selective subpopulation (subset) of B cells whose expression pattern is consistent with markers of "naive" cells (Rodig SJ1, Shahsafaei A, Li B, Dorfman DM. The CD45 isoform B220 identifies select subsets of human B cells and B-cell lymphoproliferative disorders. Hum Pathol. 2005 Jan;36(1):51-7). CD45 molecules play an important role in the regulation of T and B lymphocyte proliferation and differentiation. Expression of B220 on mature T cells precedes apoptosis after activation and proliferation of these cells (Oka S, Mori N, Matsuyama S, Takamori Y, Kubo K. Presence of B220 within thymocytes and its expression on the cell surface during apoptosis. Immunology 2000;100:417-423). The influence of the formulation on the processes of cell maturation, proliferation and differentiation at the level of the thymus is thus evident.

Figures 9 and 10 show the influence of the experimental pharmaceutical composition on the proportion of B lymphocyte subpopulations in the thymus and spleen, respectively. In the animals under treatment there is evidence of an increase in the percentage of B2 lymphocytes in the thymus, as well as a decrease in B1a and B1b lymphocytes. All these subpopulations respond to antigens, but have different requirements for T-cell support. B2 cells, which correspond to "traditional" B lymphocytes and mature in the bone marrow, inhabit the secondary lymphoid organs, where they are grouped in "primary follicles"; under antigen stimulation (with active help from T lymphocytes) they generate the germinal centers, which are the sites where these cells proliferate, mature and differentiate into memory B lymphocytes or plasmablasts, which will later be differentiated into plasma cells (Santos-Argumedo L. Phenotypic and functional diversity of B lymphocytes. Revista Alergia Mexico 2015;62:302-311). B1 cells, responsible for the production of serum IgM in the absence of apparent antigenic stimulation, are mature B lymphocytes with particular anatomical location and phenotypic and functional characteristics. B1 lymphocytes are mostly located in the peritoneal and pleural cavity, show characteristics of activated cells and are of larger size and cytoplasmic complexity than conventional B cells. While the latter must differentiate into plasma cells in order to secrete immunoglobulins, B1 lymphocytes spontaneously release antibodies into the extracellular environment, operating under a particular differentiation program. The antibodies produced by B1 lymphocytes would have a protective role, since they are involved in the removal of aged and apoptotic cells, in immunomodulation mechanisms and in resistance to infections; yet, their participation in autoimmune processes has also been suggested (Merino MC, Gruppi A. Origin and development of B1 lymphocytes A cell population involved in defense and autoimmunity. MEDICINA (Buenos Aires) 2006; 66: 165-172). However, in the spleen, a peripheral lymphoid organ where the presence and importance of B lymphocytes is much greater, the same response to treatment is not evident. The results show the greater and more important influence of the experimental pharmaceutical composition on T cells than on B lymphocytes.

The immunomodulatory and immunostimulatory role enables the application of the pharmaceutical composition in the treatment of various diseases and disorders of the immune system such as, but not limited to, aplasia, thymic hypoplasia, severe combined immunodeficiency, common variable immunodeficiency, IgA deficiency, ataxia-telangiectasia, Wiskott-Aldrich syndrome, immunodeficiencies secondary to infections, systemic lupus erythematosus, rheumatoid arthritis, juvenile idiopathic arthritis, reactive arthritis, fibromyalgia, ankylosing spondylitis, generalized osteoarthrosis, rheumatic fever, myasthenia gravis, sarcoidosis, Sjogren's syndrome, Behcet's disease, anterior uveitis, psoriasis, dermatitis, chronic urticaria, colitis, Crohn's disease, irritable bowel syndrome.

**Example 8**

Pharmaceutical composition C10 similar to pharmaceutical composition C2 of Example 1 is used, with the difference that group a proteins are absent.

Animals and housing conditions: 48 female C57BL/6 mice aged 14-15 months (24-32 g weight) are used. The animals are placed in polypropylene boxes, with wood chip bedding. They are kept in a conventional room with a controlled environment (type IV minimum security barrier), with a temperature of 21 ± 3 °C and relative humidity between 40% and 70%, with 18 air changes per hour, 100% injection of outside air with 85% filtration and lighting of 12 hours light and 12 hours darkness, which is automatically controlled. The mice are fed with EAO1004cenp diet for rodents ad libitum and drinking water.

Distribution of treatment groups, frequency of administration and doses: Four groups of animals were formed, a control group with young animals, two control groups with aged animals and one group with aged animals treated with the pharmaceutical composition C10 administered intranasally (i.n.) at a dose of 1.5 mg/kg of body weight. Groups G1 to G2 received treatment daily and G3 to G4 received treatment every other day.

Characteristics of the formulations evaluated: For i.n. administration, an experimental batch of the pharmaceutical composition C10 is used, at a concentration of 3.0 mg/mL of total proteins, dissolved in 0.9% NaCl (SSF). The formulation is stored at 2 to 8 °C, however, prior to administration the product is tempered at 37 oC. The daily dose for each group, both C10 and NaCl, is distributed in 2R bulbs with 0.5 mL volume. Each bulb includes all the doses of placebo or C10 for the same day, and the rest is discarded. Sterile 200 μL tips are used for administration, one for each animal, which is discarded at the end of each administration to avoid contamination of the product as it is a parenteral route. The dose to be administered for the intranasal route is calculated by estimating a weight of 28.03 g per animal in the case of aged animals and 22.50 g in the case of young animals.

The biological samples were obtained and the animals were sacrificed as in the previous example.

The procedure for the identification of the cell populations is the same as in example 7. For the labeling of the cells we proceed to use: for T lymphocytes: anti-CD8 FITC (dilution 1:200, v:v), anti-CD3 PE (dilution 1:600, v:v), anti-CD4 ECD (dilution 1:200, v:v) and anti-CD45 PECy5 (dilution 1:600, v:v); for B lymphocytes: anti-B220 FITC (1:200 dilution, v:v), anti-CD45 PECy5 (1:600 dilution, v:v). Next, 500 μL of phosphate-buffered saline solution with 1% fetal bovine serum (PBS-FBS) is added and the cells are washed by centrifugation at 200 g for five minutes. Subsequently, 200 μL of (PBS-FBS) containing 1% FBS is added and $0.2 \times 10^5$ - $1 \times 10^5$ cells are acquired on a Gallios flow cytometer (Beckman Coulter). The data are analyzed in the Kaluza software and plotted with the GraphPad Prism software.

Results

Figure 11 demonstrates the influence of the pharmaceutical composition of the present invention on the T cell population of total CD45+ cells in spleen homogenates of experimental mice. Differences in CD45 expression were observed between young and aged animals, with significantly higher expression of this marker in T lymphocytes under treatment with the experimental pharmaceutical composition. In this experiment the cells were not labeled with antibodies specific for the different CD45 isoforms.

Figure 12 plots the influence of the pharmaceutical composition of the present invention on the population of TCD4+ and CD8+ T cells of spleen homogenate. A higher proportion of CD3+CD4+ T cells is seen in young animals, which is not significantly influenced by treatment. No differences were found between young and old animals in the proportion of CD3+CD8+ lymphocytes. However, in animals treated with the experimental pharmaceutical composition, an increase of these cells was evidenced.

As we mentioned above, the impairment of CD4+ and CD8+ cell subpopulations with senescence has been widely discussed, with different results in several studies and with more evidence about the changes occurring in CD8+ cells (Garcia B, Saavedra D, Lorenzo-Luaces P, Badia TJ, Leonard I, Mazorra Z, et al. Immunosenescence and gender: a study in healthy Cubans. Immunity & Ageing 2013, 10:16). With senescence, both CD4+ and CD8+ cells are reported to be affected (Weinberger B, Lazuardi L, Weiskirchner I, Keller M, Neuner C, Fischer KH, et al. Healthy aging and latentinfection with CMV lead to distinct changes in CD8+ and CD4+ T-cell subsets in the elderly. Hum Immunol 2007, 68(2):86-90) with the decrease in the number and proportion of naive CD8 T cells in aged individuals being much better documented (Goronzy JJ, Lee WW, Weyand CM.Aging and T-cell diversity. Exp. Gerontol 2007;42 (5), 400-406; Yan J, Greer JM, Hull R, O'Sullivan JD, Henderson RD, Read SJ, McCombe PA: The effect of aging on human lymphocyte subsets: comparison of males and females. Immun Ageing 2010; 7:4; Wikby A, Johansson B, Olsson J, Lofgren S, Nilsson BO, Ferguson F: Expansions of peripheral blood CD8 T-lymphocyte subpopulations and an association with cytomegalovirus seropositivity in the elderly: the Swedish NONA immune study. Exp Gerontol 2002, 37(2-3):445-453) demonstrating, in this case, a positive influence of the experimental pharmaceutical composition on CD8+ lymphocytes.

**Example 9**

The cytotoxicity of a pharmaceutical composition C11 characterized by containing the proteins of the pharmaceutical composition C2 of Example 1 is tested with different protein concentrations starting from 1.7 mg/mL in 0.9 % NaCl and in dilutions.

For the activation of the cell line, RPMI1640 supplemented with 10% Fetal Bovine Serum (FBS), 2 mM L-glutamine, 1 mM sodium pyruvate and 0.1 mM non-essential amino acids, all from SigmaAldrich (Germany), was used as growth medium. The cells used, SH-SY5Y ATCC® CRL-2266™ were kindly donated by the Center for Genetic Engineering and Biotechnology (CIGB), are routinely cultured according to the recommendations of the Culture Collection.

Cell thawing is performed by pouring the medium into a 50 mL Corning tube, then adding RPMI-1640 medium (43.5 mL) with the supplements glutamine 200 nM (500 μL), antibiotic 100x (500 uL), non-essential amino acids (500 μL) and 10% fetal bovine serum (5 mL). Once the medium is prepared, 13 mL is added to the 75 cm culture flask. The cryovial is then removed from the nitrogen tank and quickly placed in a beaker with hot water at 37 °C to thaw in less than 1 min. Once the cell line is thawed, the contents are removed and placed in the previously prepared culture flask. The characteristics of the cells are observed under the microscope and they are incubated at 37 °C, with 5% $CO_2$ for 24 h. The medium is changed 24 h after thawing the cell line and all the supernatant of the flask is transferred to a 50 mL Corning tube, then 13 mL of medium without supplement is added to the culture flask to wash the cell monolayer. As these cells are adherent, the treatment with trypsin is performed with the objective of separating them from the bottom of the flask, for which 4 mL of 10x trypsin solution is added and incubated for 2 min, after which time the trypsin is inactivated with RPMI 1640 medium. Subsequently, the whole content of the culture flask is transferred to a 50 mL Corning tube and centrifuged at 1200 rpm, for 8 min and a temperature of 25 °C. After centrifugation, the entire supernatant is gently poured into the sterile Corning tube. The cell pellet is shaken by giving 5 to 6 strong blows on the bottom of the tube and the cells are detached, then re-suspended in 5 mL of new culture medium and absorbed by pipette 10 to 12 times to disaggregate the cells. When the cells are homogeneously distributed, they are transferred to the culture flask with new medium. The characteristics of the cells are observed under the microscope and they are incubated at 37 °C, with 5% $CO_2$ for 24 h.

After the incubation time, the organoleptic characteristics of the cells, such as contour, shape and viability, were observed under the microscope. The SH-SY5Y cell line was found to be in perfect condition, demonstrating that the cells assimilated the nutrients present in the medium very well. SH-SY5Y is a human-derived cell line used in scientific research. The original cell line, called SK-N-SH, from which it was subcloned, was isolated from a bone marrow biopsy taken from a four-year-old woman with neuroblastoma.

SH-SY5Y cells are seeded in 96-well flat-bottom plates (NuncThermoFisher™, Germany), at a cell density of $1.0 \times 10^4$ cells/well. To achieve cell adhesion to the surface of the wells, the plate is pretreated with Poly D Lysine (PD), dispensing 30 μL/well, and incubated at 37 °C overnight. The next day, the plate is washed with sterile water, to remove excess PD, and kept in the oven (approximately 45 °C) until any remaining water and/or moisture in the wells is completely removed. The cells are centrifuged at 220 g for 5 minutes and the resulting cell pellet is dissolved in 1 mL of culture medium for cell counting. This is performed using the exclusion method with Trypan Blue and Neubauer's chamber, where the number of live cells counted is multiplied by the dilution factor and by Neubauer's constant ($10^4$), to finally adjust the cell density to $1.5 \times 10^6$ cells/mL and seed 0.1 mL of cell suspension in each well. The plates are incubated overnight at 5% $CO_2$ and 37 °C, to allow their adherence and adaptation to the new culture conditions.

SH-SY5Y cells are seeded and cultured in 96-well plates as described above. The concentration of the pharmaceutical composition capable of causing 50% of the cytotoxic damage is determined over a 24-hour period and by evaluating six increasing concentrations of the pharmaceutical composition C11 (1.47 mg/L at 1/10 dilution). Decimal cell dilutions result in 0.147 mg/mL, 0.0147 mg/mL, 0.00147 mg/mL, 0.000147 mg/mL, 0.0000147 mg/mL and 0.00000147 mg/mL.

Cell vitality assays are performed by adding MTT (Thiazolyl Blue Tetrazolium Bromide) (0.5 mg/mL, final concentration/well) dissolved in culture medium to all wells of the plate equally and incubated at 37 °C and 5% $CO_2$ for approximately 3 hours. After removing all the supernatant, the lysis mixture is added: 3% sodium dodecyl sulfate (SDS) followed by 4% isopropanol with hydrochloric acid (1 mol/L). The plate is shaken for 5 minutes manually, using a multichannel pipette (Eppendorf, Germany), and the optical density obtained by the formation of formazan crystals produced by the degradation of mitochondrial dehydrogenases is measured in a spectrophotometer (PolarStar Omega, BMG Labtech, Germany) at 570 nm. To determine the concentration of the pharmaceutical composition C11 capable of decreasing cell vitality by 50%, IC50 (half maximal inhibitory concentration), 6 increasing concentrations (1.47-0.00000147 mg/mL) are evaluated over a period of 24 hours. The results are shown in Figure 13.

As shown in Figure 13 the exposure of SH-SY5Y cells to the pharmaceutical composition C11, during a period of 24 hours, unexpectedly causes an increase in cell vitality, at concentrations 1.47 and 0.147 mg/mL with respect to those cells not treated, presenting statistically significant differences ($p < 0.001$), an effect that is not to be expected, since only the absence of toxicity and not the phenomenon of cell proliferation is supposed to be observed. The vehicle used to dissolve the proteins of the composition, saline solution, shows no effect on cell vitality (second column). The remaining concentrations used in the study do not show significant differences to the untreated cells.

As a conclusion of the experiment, it can be demonstrated that the pharmaceutical composition unexpectedly provoked cell growth and proliferation *in vitro* in the example of SH-SY5Y cells at 24 h, in addition to demonstrating the absence of cytotoxic activity. This finding is undoubtedly due to the inclusion in the pharmaceutical composition of proteins involved in protein synthesis, neurogenesis, transcription, proper protein folding and elongation, transduction, among others.

As a result, the ability of the pharmaceutical composition for the treatment of diseases, for example, but not limited to AD, Parkinson's, schizophrenia, ischemia, fibromyalgia is evidenced.

**Example 10**

Antioxidant activity of a pharmaceutical composition (C12) according to the present invention. A pharmaceutical composition consisting of the pharmaceutical composition C2 of Example 1 combined with Ghrelin* (0.002 mg/mL) is tested. The culture media, the cell line, its activation and preparation are run as described in Example 9.

Glutamate mediates most excitatory synapses in the central nervous system (CNS). It is the main mediator of sensory, motor, cognitive and emotional information and is involved in memory formation and retrieval, being present in 80%-90% of brain synapses.

The concentration of glutamate capable of causing 50% of cytotoxic damage is determined over a 24-hour period and by evaluating seven increasing concentrations of 80; 60; 40; 20; 10; 5; 2.5 mM glutamate. The amounts are dissolved in 4 mL of RPMI 1640 medium and 4 mL of Tris HCl buffer to obtain a ½ dilution.

Cell vitality assays are performed by adding MTT (0.5 mg/mL, final concentration/well) dissolved in the culture medium to all wells of the plate equally and incubated at 37°C and 5% $CO_2$ for approximately 3 hours. After removing all the supernatant, the lysis mixture is added: 3% SDS followed by 4% isopropanol with hydrochloric acid (1 mol/L). The plate is shaken for 5 minutes manually, using a multichannel pipette (Eppendorf, Germany), and the optical density obtained by the formation of formazan crystals produced by the degradation of mitochondrial dehydrogenases is measured in a spectrophotometer (PolarStar Omega, BMG Labtech, Germany) at 570 nm. After the incubation time has elapsed, the organoleptic characteristics of the cells such as outline, shape and viability are observed under the microscope. It can be seen that the SH-SY5Y cell line is in perfect condition, demonstrating that they assimilate the nutrients present in the medium very well.

Exposure of SH-SY5Y cells to glutamate over a period of 24 h in the presence of the pharmaceutical composition causes only a 25% decrease in cell vitality. As shown in Figure 14, the concentration of glutamate causing very slight damage with respect to those cultures not subjected to damage (NT), was 80 mM and can be considered as very high. The vehicle used to dissolve the glutamate has no effect on cell vitality (second column). This demonstrates the protective effect of the tested pharmaceutical composition against oxidative stress, as it does not reach, even at high glutamate concentrations, 50% inhibition of cell growth.

This antioxidant effect evidences its use for therapy caused by oxidative phenomena or processes such as AD, Leigh's disease, multiple sclerosis, amyotrophic lateral sclerosis, among others.

**Example 11**

Acute intranasal extended toxicity of the pharmaceutical composition in BALB/Ccenp mice Forty BALB/Ccenp mice (20/sex) were used, distributed in 2 experimental groups: Control (NaCl 0.9%) and Treated (Pharmaceutical Composition C2). Two applications are performed, spaced approximately 6 h apart, taking into account that the ideal volume for a mouse is 20 μL per nostril. The administrations are performed intranasally, in the morning and in the afternoon. The dose to be administered is approximately 6.8 mg/kg; each animal receives a different dose according to its body weight. Observations are made daily and body weight is determined on days 0, 7 and 14 of the study. At the end of the study, hematology and blood biochemistry examinations are performed on half of the animals in each group. Therefore, 5 animals/sex/group are assigned for hematology and the other 5 animals/sex/group for blood biochemistry; the animals assigned for blood biochemistry undergo a complete necropsy. Post-mortem body weight is determined, selected organs (liver, kidneys, lungs, thymus, spleen and brain) are weighed and the relative weight of the organs is calculated. All organs and tissues are preserved for histopathological examination. Nasal structures are also analyzed.

The study ends with a 100% survival rate. Taking into account the following: the substance does not cause deaths or signs of toxicity, no variations in body weight values, no hematological affectations of interest are detected, low values in albumin are produced in males treated with the test substance, no anatomopathological alterations that could be associated with the test substance are detected, although alterations in the relative weight of kidneys and brain are observed, it can be concluded that the pharmaceutical composition C2 does not produce evident toxicity in BALB/Ccenp mice after a single-dose i.n. administration.

**Example 12**

Acute intranasal extended toxicity of the pharmaceutical composition C9 in CENP: SPRD rats Twenty Cenp: SPRD rats (10/sex) were used, distributed in 2 experimental groups: Control (NaCl 0.9%) and Treated (Pharmaceutical composition C2). Two applications are performed, spaced approximately 6 h apart, taking into account that the ideal volume for a rat is 50 μL per nostril. The administrations are performed intranasally, in the morning and in the afternoon. The dose to be administered is approximately 1.7 mg/kg; each animal receives a different dose according to its body weight. Observations are made daily and body weight is determined on days 0, 7 and 13 of the study. At the end of the study, hematology and blood biochemistry tests are performed on all animals. Complete necropsy is performed on all animals. Post-mortem body weight is determined, selected organs (liver, kidneys, lungs, thymus, spleen and brain) are weighed and relative organ weights are calculated. All organs and tissues are preserved for histopathological examination. Nasal structures are also analyzed.

The study culminated in 100% survival. Under the experimental conditions of the study and taking into account the following: there were no deaths in the animals, there were no manifest signs of toxicity after the administration of the test substance, no hematological affectations were observed, statistical alterations were observed in the values of total proteins and albumin in females, but without biological significance, and no anatomopathological alterations that could be associated to the test substance were detected. It can be concluded that the pharmaceutical composition of the present invention does not produce toxicity in Cenp: SPRD rats after a single-dose intranasal administration.

**Example 13**

Multiple sclerosis therapy with the pharmaceutical composition of the present invention Patient 1 was a 39-year-old female patient diagnosed with multiple sclerosis according to Poser's criteria (Poser CM, 1983) and cranial and spinal cord MRI, of the exacerbating-remitting form, and 4.5 points on Kurtzke's Expanded Disability Status Scale (Kurtzke J.R., 1983) (EDSS), with 8 flares in the year prior to the start of treatment. The composition was administered at a dose of 60 mg intravenously, weekly for one year. At the end of the year of treatment she had only had 2 outbreaks and EDSS was evaluated with a score of 4.0.

**Example 14**

Fibromyalgia therapy with the pharmaceutical composition of the present invention Patient 2, a 48-year-old female who 6 years ago was diagnosed with fibromyalgia according to the criteria of the American College of Rheumatology [Widespread pain index (WPI) ≥ 7 in 19 anatomical areas, Symptom severity score (SS-score) ≥ 5, (which presents a score between 0 and 12, and scores the presence by degree of fatigue, cognitive symptoms, and awakening without rest); symptoms that have been present with the same intensity for at least three months, with no other pathology that can explain them (Wolfe F et al. ,2011; Covarrubias et al. 2016). Before starting treatment: WPI=14, SS-score=8. Pharmaceutical composition C2 combined with GCSF is administered, at a dose of 3 mg intramuscularly, twice a week for 4 weeks and to continue with 3 mg weekly for 8 weeks. Rest period of 12 weeks and the cycle is repeated. One year after initiation of treatment: WPI=7, SS-score= 6.

**Example 15**

Patient 3, male, 56 years old, who approximately 7 months ago began with a clinical picture of sudden onset consisting of pain and swelling in the right knee, denies any traumatic history, but refers that approximately 15 days earlier he had contracted a case of acute gastroenteritis with fever. He was diagnosed with reactive oligoarthritis secondary to gastroenteritis and was prescribed treatment with non-steroidal anti-inflammatory drugs (NSAIDs), with a total duration of 3 weeks. Since then, on 2 other occasions she has had similar symptoms, but with longer duration and worse response to NSAIDs and physiotherapy. Physical examination revealed painful swelling of the right knee and functional limitation. She was prescribed treatment with composition C2, at a dose of 6 mg intravenously every other day for 2 weeks, continuing with 6 mg intramuscular in aluminum hydroxide gel twice a week for 4 weeks. At the end of the intravenous treatment there was total remission of the volume increase and at the end of 6 weeks there was total remission of the functional limitation. Three years after the end of treatment there was no new recurrence.

**Example 16**

Patient 4, male, 42 years old, with HIV diagnosis since 4 years before; treatment with highly effective antiretroviral therapy (HAART) with Nevirapine 400 mg/day, Lamivudine (3TC) 300 mg/day and Zidovudine (AZT) 600 mg/day. Despite antiretroviral therapy, he had presented several respiratory viral infections: viral load of 1700 copies/mL, CD4+ T lymphocyte count of 110 cells/mL. The product was administered in doses of 3 mg, 3 times per week for 4 weeks, a 4-week rest period and a second cycle of 3 mg, 2 times per week for 4 weeks. At 6 months after starting treatment the viral load is 240 copies/mL and the CD4+ T lymphocyte count is 600 cells/mL. At one year the viral load is undetectable and the CD4+ T cell count is maintained at 600 cells/mL. No respiratory infections or other opportunistic infection manifested. HAART remains the same.

**Example 17**

Patient 5, female, 72 years old, inveterate smoker, diagnosed with Chronic Obstructive Pulmonary Disease, with recurrent acute respiratory infections in the form of pneumonia (5 in the last year), three of which were treated with oral antibiotics (amoxicillin, azithromycin and ciprofloxacin) and the other two with intramuscular penicillin and intravenous ceftriaxone. The composition is administered at a dose of 120 mg daily orally for 3 months, continuing with 80 mg every other day to complete 1 year of treatment. During the whole year of treatment and two years after its completion, she did not present any respiratory infection.

**Example 18**

Patient 6, male, 19 months old, with repeated respiratory, digestive and skin infections, with multiple antibiotics and 3 hospital admissions, diagnosed with reduced thymus size by thymic ultrasound (Thymic Area 574 mm$^2$), with associated cellular deficit (lymphocyte subpopulations CD3+/CD4+=21% (VN:25-50%) CD3+/CD8+=13% (VN:11-32%)) and IgA deficiency (0.36 mg/L). The composition is administered at a dose of 3 mg intramuscular 3 times per week for 4 weeks, 4 weeks rest, 3 mg intramuscular twice per week, 4 weeks rest and 3 mg intramuscular weekly for 8 weeks. After 8 weeks of rest from the last cycle (32 weeks after starting treatment) a thymic ultrasound was performed (normal Thymic Area in 1014 mm$^2$), normal lymphocyte subpopulations (CD3+/CD4+=52%; CD3+/CD8+=26%) and normal IgA (1.43 mg/L). Only one respiratory infection occurred in the period in the form of a common cold with satisfactory evolution and only with symptomatic treatment, with no hospital admissions.

**Example 19**

Patient 8, female, 39 years old, diagnosed with breast neoplasia (triple negative ductal carcinoma, stage IIA in the left breast) who underwent quadrantectomy with axillary emptying and subsequent adjuvant chemotherapy for 4 months with docetaxel. At 16 months a recurrence occurred in the right breast that required new surgical treatment and chemotherapy for 6 months. Patient with significant nutritional deterioration, anemia, asthenia and generalized arthralgias. She presents respiratory infectious symptoms with slow evolution; pulmonary or bone metastasis is discarded. The composition was administered at a dose of 3 mg intramuscular in weekly doses for 12 weeks, 4 weeks of rest and a repeat cycle. After one year of treatment and having recovered her nutritional status, there were no other infectious symptoms, no arthralgias were observed, her general condition was excellent and she returned to work.

**Example 20**

Patient 9, female, 61 years of age, with a cyto/histological diagnosis of stage IIIb non-small cell lung carcinoma, with clinical status criteria (ECOG) of 1. Immediately after concluding the first line treatment with chemotherapy, the composition is administered at a dose of 3 mg intramuscular, 3 times per week for 4 weeks, prior to the use of biological therapy, of which it is known that there will be a better response if the patient starts with a better immune status, i.e. lymphocyte subpopulations of CD4+ T cells, an effect that is achieved with the treatment scheme administered.

**Claims**

1. Pharmaceutical combination for therapeutic purposes **characterized by** containing a mixture of proteins belonging to two or more of the following groups:

   **a)** one or more proteins with antioxidant activity selected among Thioredoxin or its component peptides containing in their molecule the sequences EKLEAT (SEC ID NO 1), YAFQEALNSAGEK (SEC ID NO 2) and MIKPFFHSLSEK (SEC ID N. 3); Cytochrome C or its component peptides containing in their molecule the sequences EDLIAYLK (SEC ID NO 4), TGPNLHGLFGR (SEC ID NO 5), TGQAPGFSYTDAN (SEC ID NO 6) and GITWGEETLMEYLENPK (SEC ID NO 7) and NDUFA4 subunit of Cytochrome C oxidase or its component peptides containing in their molecule the sequences FIGAGGTGAALY (SEC ID NO 8) and VNVDYSKLKKKEGP (SEC ID NO 9);
   **b)** one or more proteins with activity on the ubiquitin-proteosome system selected among Ubiquitin, Ubiquitin-derived proteins with arginine or leucine deletions at the N- or C-terminal or methylated, or its component peptides containing in their molecule the sequences MQIFVKTLTG (SEC ID NO 10), KTITLEVEPS (SEC ID NO 11), DTIENVKAKI (SEC ID NO 12), QDKEGIPPDQ (SEC ID NO 13), QRLIFAGKQL (SEC ID NO 14), EDGRTLSDYN (SEC ID NO 15) and IQKESTLHLVLR (SEC ID NO 16); Small Ubiquitin-fold modifier 1 (Ufm1) or its component peptides containing in their molecule the sequences MSKVSFKKITL (SEC ID NO 17), AVLKFAAEEF (SEC ID NO 18); Small Ubiquitin-fold modifier 2 (Ufm2) or its component peptides containing in their molecule the sequences VAGQDGSVVVQFK (SEC ID NO 19) and MADEKPKKEGVK (SEC ID NO 20); Peptidyl-prolyl-cis-trans isomerase H or its component peptides containing in their molecule the sequences GVQVETISPGDGR (SEC ID NO 21), GWEEGVAQMSVGQR (SEC ID NO 22); Cystatin B or its component peptides containing in their molecule the sequences VQVDEDDFVHIR (SEC ID NO 23) and VFESLPHENKPVALTSYQT (SEC ID NO 24); P47 NSFL1 cofactor or its component peptides containing in their molecule the sequences EFVAVTGAEEDR (SEC ID NO 25) and SYQDPSNAQFLESIR (SEC ID NO 26) and Homolog B of the UV excision repair protein RAD23 or its component peptides containing in their molecule the sequences IDIDPDETVR (SEC ID NO 27) and NFVVVMVTKPK (SEC ID NO 28);

**c**) one or more proteins with anti-inflammatory and immune system modulating activity selected among:

i) proteins belonging to the Beta Thymosin family or their component peptides containing in their molecule 3 sequences of 4 amino acids: DKPD (SEC ID NO 29), LKKT (SEC ID NO 30) and KETI (SEC ID NO 31);
ii) proteins belonging to the alpha-thymosin family or its component peptides containing 2 sequences of 7 to 14 amino acids in their molecule: SDAAVDTSSEITTK (SEC ID NO 32) and VVEZAEN (SEC ID NO 33);
iii) proteins belonging to the thymopoeitin family or their component peptides containing in their molecule 3 sequences of 13 amino acids: FLEDPSVLTKEKL (SEC ID NO 34), KSELVANNNVTLPA (SEC ID NO 35), GEQRKDVYVELYL (SEC ID NO 36);
iv) other thymic proteins containing the sequences in their molecule RKNVW (SEC ID NO 37), RKDVY (SEC ID NO 38), EAKSQGSN (SEC ID NO 39), EASQGGSD (SEC ID NO 40), PYRAKSQGGN (SEC ID NO 41);

**d**) one or more proteins with antimicrobial activity selected from among the Cathelicidin-4 precursor or its component peptides containing in its molecule the sequences LELDPPPK (SEC ID NO 42), AVDQLNELSSEANLYR (SEC ID NO 43) and LLELDPPPKDNEDLGTR (SEC ID NO 44) and Cathelicidin-1 and its component peptides containing in its molecule the sequences LELDPPPK (SEC ID NO 44) and Cathelicidin-1 and its component peptides that contain in their molecule the sequences AVDQLNEQSSEPNIYR (SEC ID NO 45) and LLELDQPPQDDDEDPDSPK (SEC ID NO 46);

**e**) a protein with activity on lipid metabolism, specifically the copper transporter protein ATOX1 and its component peptides containing in its molecule the sequences MPKHEFSVDM (SEC ID NO 47) and FDIDLPNKKV (SEC ID NO 48);

**f**) one or more proteins with activity on protein synthesis and transcription, selected among transcription elongation factor A protein 1 (TCEA1) or its component peptides containing in its molecule the sequence NIPMTLELLQSTR (SEC ID NO 49) and serine/arginine-rich splicing factor 2 or its component peptides containing in its molecule the sequences DAEDAMDAMDGAVLDGR (SEC ID NO 50) and SYGRPPPDVEGMTSLK (SEC ID NO 51).

2. Pharmaceutical composition comprising the pharmaceutical combination according to claim 1 characterized because the combination of the proteins and their component peptides is in concentration from 0.2 mg/g to 3 mg/g or from 0.2 mg/mL to 3 mg/mL of the total mass or volume of the composition, the proteins of groups **b** and **c** being found in concentrations per individual from 0, 02 to 2.7 mg/mL or mg/g of the total mass or volume of the pharmaceutical composition and the component proteins or peptides of groups **a**, **d**, **e** and **f** per individual in concentrations of 0.000002 to 0.3 mg/mL or mg/g of the total mass or volume of the composition.

3. Composition according to claim 1, **characterized by** obtaining the proteins and their component peptides by chemical synthesis, by recombinant techniques through their expression in bacteria or yeast and purification of the recombinant protein; by extraction, extraction and semi-purification or extraction and purification until their individual isolation from mammalian organs and tissues, preferably bovine.

4. Pharmaceutical composition **characterized by** containing a mixture of the proteins described in the pharmaceutical combination of claim 1 with other proteins selected among Calmodulin, Erythropoietin, histones, DNA replication factors, Keratin, Syntaxin, Granulocyte Colony Stimulating Factor, which are in concentration from 0, 001 to 1.8 mg/mL or mg/g with respect to the final volume or mass of the pharmaceutical composition, and the proteins per individual are added in amounts of 0.001 to 1.5 mg/mL or mg/g, preferably from 0.001 to 0.15 mg/mL or mg/g, more preferably from 0.001 to 0.015 mg/mL or mg/g and still more preferably from 0.001 to 0.0015 mg/mL or mg/g.

5. Pharmaceutical composition based on proteins and peptides for therapeutic purposes **characterized by** combining the proteins of claim 1 with other selected proteins or polypeptides containing in their molecule the amino acid sequences MAGSSFLSP (SEC ID NO 52) and LEIRFNAPF (SEC ID NO 53), preferably Ghrelin, which is added in concentration from 0.001 to 1.5 mg/mL or mg/g with respect to the total volume or mass of the pharmaceutical combination, more preferably from 0.001 to 0.3 mg/mL or mg/g with respect to the total volume or mass of the pharmaceutical composition.

6. Composition according to claims 1 to 5 **characterized by** its intranasal, intramuscular, intravenous, oral and sublingual administration.

7. Pharmaceutical composition according to claims 1 to 5, **characterized by** incorporating into the selected pharmaceutical protein or peptide combinations a vehicle for intranasal administration to achieve protein concentration in said pharmaceutical composition from 0.2 to 3 mg/g or from 0.2 to 3 mg/mL, selected from:

   - polyvinyl butyl ether, polyvinyl alcohol;
   - NaCl solution, potassium monobasic and potassium dibasic phosphate buffer solution at 0.9% concentration;
   - chitosan, agarose, pectin, sodium carrageenan, cyclodextrin, sodium alginate and
   - carbacol.

8. Pharmaceutical composition according to claims 1 to 5, **characterized by** incorporating to the selected pharmaceutical combinations of proteins or peptides a vehicle for intramuscular administration to achieve protein concentration in said pharmaceutical composition from 0.2 to 3 mg/g or from 0.2 to 3 mg/mL, selected among:

   - 0.9% NaCl solution, potassium monobasic and potassium dibasic phosphate buffer solution at 0.9% concentration, 0.9% sodium phosphate solution;
   - aluminum sulfate or aluminum hydroxide with a maximum aluminum concentration of 0.3 mg/dose to 1.5 mg/dose.

9. Pharmaceutical composition according to claims 1 to 5, **characterized by** incorporating to the pharmaceutical combinations of selected proteins or peptides a vehicle for intravenous administration consisting of a 0.9 % NaCl solution to achieve the concentration of proteins in said pharmaceutical composition from 0.2 mg/mL to 3 mg/mL.

10. Pharmaceutical composition according to claims 1 to 5, **characterized by** incorporating into the selected pharmaceutical protein or peptide combinations a vehicle for oral administration to achieve protein concentration in said pharmaceutical composition of 0.2 to 3 mg/g or 0.2 to 3 mg/mL, selected from:

   - emulsion with oils of *Plukenetia volubilis,* arachis, soybean, castor, sesame or preferably *Plukenetia volubilis* oil;
   - polyvinyl butyl ether or polyvinyl alcohol, in concentrations from 1% to 50%, preferably from 1% to 30%, more preferably from 10% to 20%, with respect to the volume of the pharmaceutical composition (v/v).

11. Pharmaceutical composition according to claims 1 to 5, **characterized by** incorporating into the selected pharmaceutical combinations of proteins or peptides a vehicle for sublingual administration selected from:

   - polyvinyl butyl ether or polyvinyl alcohol;
   - potassium monobasic and potassium dibasic phosphate buffer solution at 0.9% concentration, potassium monobasic and potassium dibasic phosphate 0.9% concentration, 0.9% sodium phosphate solution;
   - emulsion with oils of Plukenetia volubilis, arachis, soybean, castor, sesame or preferably Plukenetia volubilis.

12. Pharmaceutical composition according to claims 9 and 10, characterized because the emulsion of Plukenetia volubilis, arachis, soybean, castor, or sesame, used as a vehicle for oral or sublingual administration, has a droplet size of from 5 to 200 nm, preferably from 50 to 200 nm and more preferably from 50 to 100 nm.

13. Composition based on proteins and their component peptides for therapeutic purposes according to claims 1 to 5, **characterized by** its application in the therapy of diseases of the central and peripheral nervous system selected from a group consisting of AD, Parkinson's disease, Leigh syndrome, epilepsy, cerebral ischemia, head trauma, multiple sclerosis, amyotrophic lateral sclerosis, Wilson's disease, Menkes disease, retrovulbar neuritis, encephalopathies and polyneuropathies.

14. Composition based on proteins and their component peptides for therapeutic purposes, according to claims 1 to 5, **characterized by** its application in the therapy of diseases with autoimmune and inflammatory component selected from a group consisting of systemic lupus erythematosus, rheumatoid arthritis, juvenile idiopathic arthritis, reactive arthritis, fibromyalgia, ankylosing spondylitis, generalized osteoarthrosis, rheumatic fever, myasthenia gravis, sarcoidosis, Sjogren's syndrome, Behcet's disease, anterior uveitis, psoriasis, dermatitis, chronic urticaria, colitis, Crohn's disease, irritable bowel syndrome.

15. Composition based on proteins and their component peptides for therapeutic purposes according to claims 1 to 5, **characterized by** its application in adjuvant therapy in acute, chronic or recurrent infectious diseases, selected from a group consisting of septic shock, HIV, Hepatitis B, Hepatitis C, systemic or deep fungal infections, recurrent respiratory infections, recurrent herpetic or mucocutaneous herpetic or fungal infection.

16. Composition based on proteins and their component peptides for therapeutic purposes according to claims 1 to 5 **characterized by** its application in therapy as immunomodulator in primary or secondary immunodeficiency states such as aplasia, thymic hypoplasia, severe combined immunodeficiency, common variable immunodeficiency, IgA deficiency, ataxia-telangectasia, Wiskott-Aldrich syndrome, immunodeficiencies secondary to infections, immunosenescence.

17. Composition based on proteins and their component peptides for therapeutic purposes according to claims 1 to 5 **characterized by** its application in therapy as an immunomodulator in inflammatory diseases such as rhinosinusitis, atopic dermatitis.

18. Composition based on proteins and their component peptides for therapeutic purposes according to claims 1 to 5 for its application as an adjuvant treatment in cancer therapy.

19. Composition according to claims 1 to 5, **characterized by** its intranasal administration in one or more doses of 0.1 mg to 3 mg, in volumes of 0.5 mL to 1 mL per nostril, preferably 0.2 mL to 0.3 mL, with a few minutes between doses; with a frequency of 1, 2, 3 times per week, alternate days or daily, during a period of 4 weeks to 1 year.

20. Composition according to claims 1 to 5, **characterized by** its intramuscular administration in doses of 1.5 mg to 15 mg, with a frequency of 1, 2, 3 times per week, alternate days or daily, in one or more cycles with rest periods between cycles of 4 to 24 weeks, during a period for each cycle of 4 to 12 weeks.

21. Composition according to claims 1 to 5, **characterized by** its intravenous administration, in doses of 3 mg to 80 mg, with a frequency of 1, 2, 3 times per week, alternate days or daily for a period of 4 weeks to 1 year.

22. Composition according to claims 1 to 5, **characterized by** its oral administration, in doses of 60 mg to 120 mg, daily or every other day, for a period of 4 weeks to 1 year.

23. Composition according to claims 1 to 5, **characterized by** its sublingual administration, in doses of 0.5 mg to 3 mg, in volumes of 0.1 to 0.5 mL, with a frequency of twice a day, daily or every other day, for a period of 6 months to 1 year.

**Amended claims under Art. 19.1 PCT**

1. Pharmaceutical combination for therapeutic purposes **characterized by** containing a mixture of proteins with different activities selected from at least two of the following groups:

a) at least one protein with antioxidant activity selected among Thioredoxin or its component peptides containing in their molecule the sequences EKLEAT (SEC ID NO 1), YAFQEALNSAGEK (SEC ID NO 2) and MIKPFFHSLSEK (SEC ID N. 3); Cytochrome C or its component peptides containing in their molecule the sequences EDLIAYLK (SEC ID NO 4), TGPNLHGLFGR (SEC ID NO 5), TGQAPGFSYTDAN (SEC ID NO 6) and GITWGEETLMEYLENPK (SEC ID NO 7) and NDUFA4 subunit of Cytochrome C oxidase or its component peptides containing in their molecule the sequences FIGAGGTGAALY (SEC ID NO 8) and VNVDYSKLKKKEGP (SEC ID NO 9);
b) at least one protein with activity on the ubiquitin-proteosome system selected among Ubiquitin, Ubiquitin-derived proteins with arginine or leucine deletions at the N- or C-terminal or methylated, or its component peptides containing in their molecule the sequences MQIFVKTLTG (SEC ID NO 10), KTITLEVEPS (SEC ID NO 11), DTIENVKAKI (SEC ID NO 12), QDKEGIPPDQ (SEC ID NO 13), QRLIFAGKQL (SEC ID NO 14), EDGRTLSDYN (SEC ID NO 15) and IQKESTLHLVLR (SEC ID NO 16); Small Ubiquitin-fold modifier 1 (Ufm1) or its component peptides containing in their molecule the sequences MSKVSFKKITL (SEC ID NO 17), AVLKFAAEEF (SEC ID NO 18); Small Ubiquitin-fold modifier 2 (Ufm2) or its component peptides containing in their molecule the sequences VAGQDGSVVVQFK (SEC ID NO 19) and MADEKPKKEGVK (SEC ID NO 20); Peptidyl-prolyl-cis-trans isomerase H or its component peptides containing in their molecule the sequences GVQVETISPGDGR (SEC ID NO 21), GWEEGVAQMSVGQR (SEC ID NO 22); Cystatin B or its component peptides containing in their molecule the sequences VQVDEDDFVHIR (SEC ID NO 23) and VFESLPHENKPVALTSYQT (SEC ID NO 24); P47 NSFL1 cofactor or its component peptides containing in their molecule the sequences EFVAVTGAEEDR (SEC ID NO 25) and SYQDPSNAQFLESIR (SEC ID NO 26) and Homolog B of the UV excision repair protein RAD23 or its component peptides containing in their molecule the sequences IDIDPDETVR (SEC ID NO 27) and NFVVVMVTKPK (SEC ID NO 28);

**c**) at least one protein with anti-inflammatory and immune system modulating activity selected among:

i) proteins belonging to the Beta Thymosin family or their component peptides containing in their molecule 3 sequences of 4 amino acids: DKPD (SEC ID NO 29), LKKT (SEC ID NO 30) and KETI (SEC ID NO 31);
ii) proteins belonging to the alpha-thymosin family or its component peptides containing 2 sequences of 7 to 14 amino acids in their molecule: SDAAVDTSSEITTK (SEC ID NO 32) and VVEZAEN (SEC ID NO 33);
iii) proteins belonging to the thymopoeitin family or their component peptides containing in their molecule 3 sequences of 13 amino acids: FLEDPSVLTKEKL (SEC ID NO 34), KSELVANNNVTLPA (SEC ID NO 35), GEQRKDVYVELYL (SEC ID NO 36);
iv) other thymic proteins containing the sequences in their molecule RKNVW (SEC ID NO 37), RKDVY (SEC ID NO 38), EAKSQGSN (SEC ID NO 39), EASQGGSD (SEC ID NO 40), PYRAKSQGGN (SEC ID NO 41);

**d**) at least one protein with antimicrobial activity selected from among the Cathelicidin-4 precursor or its component peptides containing in its molecule the sequences LELDPPPK (SEC ID NO 42), AVDQLNELSSEANLYR (SEC ID NO 43) and LLELDPPPKDNEDLGTR (SEC ID NO 44) and Cathelicidin-1 and its component peptides containing in its molecule the sequences LELDPPPK (SEC ID NO 44) and Cathelicidin-1 and its component peptides that contain in their molecule the sequences AVDQLNEQSSEPNIYR (SEC ID NO 45) and LLELDQPPQDDDEDPDSPK (SEC ID NO 46);

**e**) a protein with activity on lipid metabolism, specifically the copper transporter protein ATOX1 and its component peptides containing in its molecule the sequences MPKHEFSVDM (SEC ID NO 47) and FDIDLPNKKV (SEC ID NO 48);

**f**) at least one protein with activity on protein synthesis and transcription, selected among transcription elongation factor A protein 1 (TCEA1) or its component peptides containing in its molecule the sequence NIPMTLELLQSTR (SEC ID NO 49) and serine/arginine-rich splicing factor 2 or its component peptides containing in its molecule the sequences DAEDAMDAMDGAVLDGR (SEC ID NO 50) and SYGRPPPDVEGMTSLK (SEC ID NO 51).

2. Pharmaceutical composition comprising the pharmaceutical combination according to claim 1 characterized because the combination of the proteins and their component peptides is in concentration from 0.2 mg/g to 3 mg/g or from 0.2 mg/mL to 3 mg/mL of the total mass or volume of the composition, the proteins of groups **b** and **c** being found in concentrations per individual from 0, 02 to 2.7 mg/mL or mg/g of the total mass or volume of the pharmaceutical composition and the component proteins or peptides of groups **a**, **d**, **e** and **f** per individual in concentrations of 0.000002 to 0.3 mg/mL or mg/g of the total mass or volume of the composition.

3. Composition according to claim 1, **characterized by** obtaining the proteins and their component peptides by chemical synthesis, by recombinant techniques through their expression in bacteria or yeast and purification of the recombinant protein; by extraction, extraction and semi-purification or extraction and purification until their individual isolation from mammalian organs and tissues, preferably bovine.

4. Pharmaceutical composition **characterized by** containing a mixture of the proteins described in the pharmaceutical combination of claim 1 with other proteins selected among Calmodulin, Erythropoietin, histones, DNA replication factors, Keratin, Syntaxin, Granulocyte Colony Stimulating Factor, which are in concentration from 0, 001 to 1.8 mg/mL or mg/g with respect to the final volume or mass of the pharmaceutical composition, and the proteins per individual are added in amounts of 0.001 to 1.5 mg/mL or mg/g, preferably from 0.001 to 0.15 mg/mL or mg/g, more preferably from 0.001 to 0.015 mg/mL or mg/g and still more preferably from 0.001 to 0.0015 mg/mL or mg/g.

5. Pharmaceutical composition based on proteins and peptides for therapeutic purposes **characterized by** combining the proteins of claim 1 with other selected proteins or polypeptides containing in their molecule the amino acid sequences MAGSSFLSP (SEC ID NO 52) and LEIRFNAPF (SEC ID NO 53), preferably Ghrelin, which is added in concentration from 0.001 to 1.5 mg/mL or mg/g with respect to the total volume or mass of the pharmaceutical combination, more preferably from 0.001 to 0.3 mg/mL or mg/g with respect to the total volume or mass of the pharmaceutical composition.

6. Composition according to claims 1 to 5 **characterized by** its intranasal, intramuscular, intravenous, oral and sublingual administration.

7. Pharmaceutical composition according to claims 1 to 5, **characterized by** incorporating into the selected pharmaceutical protein or peptide combinations a vehicle for intranasal administration to achieve protein concentration in said pharmaceutical composition from 0.2 to 3 mg/g or from 0.2 to 3 mg/mL, selected from:

   - polyvinyl butyl ether, polyvinyl alcohol;
   - NaCl solution, potassium monobasic and potassium dibasic phosphate buffer solution at 0.9% concentration;
   - chitosan, agarose, pectin, sodium carrageenan, cyclodextrin, sodium alginate and
   - carbacol.

8. Pharmaceutical composition according to claims 1 to 5, **characterized by** incorporating to the selected pharmaceutical combinations of proteins or peptides a vehicle for intramuscular administration to achieve protein concentration in said pharmaceutical composition from 0.2 to 3 mg/g or from 0.2 to 3 mg/mL, selected among:

   - 0.9% NaCl solution, potassium monobasic and potassium dibasic phosphate buffer solution at 0.9% concentration, 0.9% sodium phosphate solution;
   - aluminum sulfate or aluminum hydroxide with a maximum aluminum concentration of 0.3 mg/dose to 1.5 mg/dose.

9. Pharmaceutical composition according to claims 1 to 5, **characterized by** incorporating to the pharmaceutical combinations of selected proteins or peptides a vehicle for intravenous administration consisting of a 0.9 % NaCl solution to achieve the concentration of proteins in said pharmaceutical composition from 0.2 mg/mL to 3 mg/mL.

10. Pharmaceutical composition according to claims 1 to 5, **characterized by** incorporating into the selected pharmaceutical protein or peptide combinations a vehicle for oral administration to achieve protein concentration in said pharmaceutical composition of 0.2 to 3 mg/g or 0.2 to 3 mg/mL, selected from:

   - emulsion with oils of *Plukenetia volubilis,* arachis, soybean, castor, sesame or preferably *Plukenetia volubilis* oil;
   - polyvinyl butyl ether or polyvinyl alcohol, in concentrations from 1% to 50%, preferably from 1% to 30%, more preferably from 10% to 20%, with respect to the volume of the pharmaceutical composition (v/v).

11. Pharmaceutical composition according to claims 1 to 5, **characterized by** incorporating into the selected pharmaceutical combinations of proteins or peptides a vehicle for sublingual administration selected from:

   - polyvinyl butyl ether or polyvinyl alcohol;
   - potassium monobasic and potassium dibasic phosphate buffer solution at 0.9% concentration, potassium monobasic and potassium dibasic phosphate 0.9% concentration, 0.9% sodium phosphate solution;
   - emulsion with oils of Plukenetia volubilis, arachis, soybean, castor, sesame or preferably Plukenetia volubilis.

12. Pharmaceutical composition according to claims 9 and 10, characterized because the emulsion of Plukenetia volubilis, arachis, soybean, castor, or sesame, used as a vehicle for oral or sublingual administration, has a droplet size of from 5 to 200 nm, preferably from 50 to 200 nm and more preferably from 50 to 100 nm.

13. Composition based on proteins and their component peptides for therapeutic purposes according to claims 1 to 5, **characterized by** its application in the therapy of diseases of the central and peripheral nervous system selected from a group consisting of AD, Parkinson's disease, Leigh syndrome, epilepsy, cerebral ischemia, head trauma, multiple sclerosis, amyotrophic lateral sclerosis, Wilson's disease, Menkes disease, retrovulbar neuritis, encephalopathies and polyneuropathies.

14. Composition based on proteins and their component peptides for therapeutic purposes, according to claims 1 to 5, **characterized by** its application in the therapy of diseases with autoimmune and inflammatory component selected from a group consisting of systemic lupus erythematosus, rheumatoid arthritis, juvenile idiopathic arthritis, reactive arthritis, fibromyalgia, ankylosing spondylitis, generalized osteoarthrosis, rheumatic fever, myasthenia gravis, sarcoidosis, Sjogren's syndrome, Behcet's disease, anterior uveitis, psoriasis, dermatitis, chronic urticaria, colitis, Crohn's disease, irritable bowel syndrome.

15. Composition based on proteins and their component peptides for therapeutic purposes according to claims 1 to 5, **characterized by** its application in adjuvant therapy in acute, chronic or recurrent infectious diseases, selected from a group consisting of septic shock, HIV, Hepatitis B, Hepatitis C, systemic or deep fungal infections, recurrent respiratory infections, recurrent herpetic or mucocutaneous herpetic or fungal infection.

16. Composition based on proteins and their component peptides for therapeutic purposes according to claims 1 to 5 **characterized by** its application in therapy as immunomodulator in primary or secondary immunodeficiency states such as aplasia, thymic hypoplasia, severe combined immunodeficiency, common variable immunodeficiency, IgA deficiency, ataxia-telangectasia, Wiskott-Aldrich syndrome, immunodeficiencies secondary to infections, immunosenescence.

17. Composition based on proteins and their component peptides for therapeutic purposes according to claims 1 to 5 **characterized by** its application in therapy as an immunomodulator in inflammatory diseases such as rhinosinusitis, atopic dermatitis.

18. Composition based on proteins and their component peptides for therapeutic purposes according to claims 1 to 5 for its application as an adjuvant treatment in cancer therapy.

19. Composition according to claims 1 to 5, **characterized by** its intranasal administration in one or more doses of 0.1 mg to 3 mg, in volumes of 0.5 mL to 1 mL per nostril, preferably 0.2 mL to 0.3 mL, with a few minutes between doses; with a frequency of 1, 2, 3 times per week, alternate days or daily, during a period of 4 weeks to 1 year.

20. Composition according to claims 1 to 5, **characterized by** its intramuscular administration in doses of 1.5 mg to 15 mg, with a frequency of 1, 2, 3 times per week, alternate days or daily, in one or more cycles with rest periods between cycles of 4 to 24 weeks, during a period for each cycle of 4 to 12 weeks.

21. Composition according to claims 1 to 5, **characterized by** its intravenous administration, in doses of 3 mg to 80 mg, with a frequency of 1, 2, 3 times per week, alternate days or daily for a period of 4 weeks to 1 year.

22. Composition according to claims 1 to 5, **characterized by** its oral administration, in doses of 60 mg to 120 mg, daily or every other day, for a period of 4 weeks to 1 year.

23. Composition according to claims 1 to 5, **characterized by** its sublingual administration, in doses of 0.5 mg to 3 mg, in volumes of 0.1 to 0.5 mL, with a frequency of twice a day, daily or every other day, for a period of 6 months to 1 year.

Figure 1. Percentage of alternation in the Y-maze test

Figure 2. Number of entries of experimental animals in the Y-maze

Figure 3. Morris Water Maze Test Results

Figure 4. Pain sensitivity test

# Figure 5. Influence of pharmaceutical composition on thymus CD3- and CD3+ lymphocyte population

## Inside the CD3-

**Group 1**

[T CD3-] CD4 FITC/CD8 ECD

CD8+:4.06% | CD4+CD8+:90.33%

CD4-CD8- | CD4+: 2.01%

**Group 2**

[T CD3-] CD4 FITC/CD8 ECD

CD8+:4.89% | CD4+CD8+:91.36%

CD4-CD8- | CD4+: 1.09%

**Group 3**

[T CD3-] CD4 FITC/CD8 ECD

CD8+:9.36% | CD4+CD8+:83.39%

CD4-CD8- | CD4+: 1.09%

**Group 4**

[T CD3-] CD4 FITC/CD8 ECD

CD8+: 18.42% | CD4+CD8+:64.12%

CD4-CD8- | CD4+: 1.96%

## Inside the CD3+

**Group 1**

[T CD3+] CD4 FITC/CD8 ECD

T CD8+: 11.76% | T CD4+CD8+: 11.71%

T CD4-CD8- | T CD4+: 67.47%

**Group 2**

[T CD3+] CD4 FITC/CD8 ECD

T CD8+: 9.92% | T CD4+CD8+: 28.73%

T CD4-CD8- | T CD4+: 48.06%

**Group 3**

[T CD3+] CD4 FITC/CD8 ECD

T CD8+: 14.77% | T CD4+CD8+: 44.30%

T CD4-CD8- | T CD4+: 20.65%

**Group 4**

[T CD3+] CD4 FITC/CD8 ECD

T CD8+: 8.94% | T CD4+CD8+: 23.40%

T CD4-CD8- | T CD4+: 43.96%

EP 4 129 318 A1

Figure 6. Influence of pharmaceutical composition on spleen CD3- and CD3+
lymphocyte population

Figure 7. Influence of pharmaceutical composition on spleen T lymphocyte population

Figure 8. Influence of pharmaceutical composition on thymus B lymphocyte population

Figure 9. Influence of pharmaceutical composition on thymus B lymphocyte subpopulations

Figure 10. Influence of pharmaceutical composition on spleen B lymphocyte population

Figure 11. Influence of pharmaceutical composition on the T-cell population of total spleen CD45+ cells

Figure 12. Influence of pharmaceutical composition on spleen TCD4+ and TCD8+ cell population

Figure 13. Influence of pharmaceutical composition on the vitality of SH-SY5Y cells cultured "*in vitro*"

Figure 14. Demonstration of the *in vitro* antioxidant effect of the pharmaceutical composition of the present invention

# INTERNATIONAL SEARCH REPORT

International application No.

PCT/CU2020/050006

## A. CLASSIFICATION OF SUBJECT MATTER

**See extra sheet**

According to International Patent Classification (IPC) or to both national classification and IPC

## B. FIELDS SEARCHED

Minimum documentation searched (classification system followed by classification symbols)
A61K, A61P

Documentation searched other than minimum documentation to the extent that such documents are included in the fields searched

Electronic data base consulted during the international search (name of data base and, where practicable, search terms used)

EPODOC, WPI, MEDLINE, EMBASE, BIOSIS

## C. DOCUMENTS CONSIDERED TO BE RELEVANT

| Category* | Citation of document, with indication, where appropriate, of the relevant passages | Relevant to claim No. |
|---|---|---|
| A | WO 0193896 A2 (RAKEPOLL HOLDING B V   ET AL.) 13/12/2001, the whole document. | 1-23 (all partially) |
| A | WO 2008054635 A2 (VIRAL GENETICS INC   ET AL.) 08/05/2008, the whole document. | 1-23 (all partially) |
| A | EP 3305803 A1 (UNIV AJOU IND ACADEMIC COOP FOUND GENESEN CO LTD) 11/04/2018, the whole document. | 1-23 (all partially) |
| A | JP 2004010574 A (REDOX BIOSCIENCE INC ET AL.) 15/01/2004, the whole document. | 1-23 (all partially) |
| A | WO 2009151689 A2 (REGENERX BIOPHARMACEUTICALS ET AL.) 17/12/2009, the whole document. | 1-23 (all partially) |

☐ Further documents are listed in the continuation of Box C.       ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 05/04/2021 | **(07/04/2021)** |
| Name and mailing address of the ISA/ | Authorized officer |
| | M. Hernández Cuéllar |
| OFICINA ESPAÑOLA DE PATENTES Y MARCAS | |
| Paseo de la Castellana, 75 - 28071 Madrid (España) | |
| Facsimile No.: 91 349 53 04 | Telephone No. 91 3498409 |

Form PCT/ISA/210 (second sheet) (January 2015)

**INTERNATIONAL SEARCH REPORT**

| International application No. |
| --- |
| PCT/CU2020/050006 |

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 1-23 (all partially)
because they relate to parts of the international application that do not comply with the prescribed requirements to such an extent that no meaningful international search can be carried out, specifically:

See additional sheet

3. ☐ Claims Nos.:
because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims;  it is covered by claims Nos.:

**Remark on Protest**     ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

INTERNATIONAL SEARCH REPORT

| International application No. |
| --- |
| PCT/CU2020/050006 |

Form PCT/ISA/210 (extra sheet) (January 2015)

## INTERNATIONAL SEARCH REPORT

Information on patent family members

International application No.

PCT/CU2020/050006

| Patent document cited in the search report | Publication date | Patent family member(s) | Publication date |
|---|---|---|---|
| WO2009151689 A2 | 17.12.2009 | HK1220703 A1 | 12.05.2017 |
| | | CN105504043 A | 20.04.2016 |
| | | CN105504043B B | 31.01.2020 |
| | | US2015203561 A1 | 23.07.2015 |
| | | EP2811030 A2 | 10.12.2014 |
| | | EP2811030 A3 | 21.01.2015 |
| | | US2013196912 A1 | 01.08.2013 |
| | | MX2010010177 A | 23.08.2012 |
| | | US2011172155 A1 | 14.07.2011 |
| | | JP2011514383 A | 06.05.2011 |
| | | CN102037133 A | 27.04.2011 |
| | | CN102037133B B | 13.01.2016 |
| | | CA2718774 A1 | 17.12.2009 |
| | | AU2009258034 A1 | 17.12.2009 |
| | | AU2009258034B B2 | 16.07.2015 |
| | | EP2260106 A1 | 15.12.2010 |
| | | EP2260106 A4 | 20.04.2011 |
| JP2004010574 A | 15.01.2004 | JP4925155B B2 | 25.04.2012 |
| WO0193896 A2 | 13.12.2001 | US2003165492 A1 | 04.09.2003 |
| | | MXPA02012089 A | 19.08.2004 |
| | | JP2003535143 A | 25.11.2003 |
| | | EP1286688 A2 | 05.03.2003 |
| | | CA2411432 A1 | 13.12.2001 |
| | | AU7964301 A | 17.12.2001 |
| WO2008054635 A2 | 08.05.2008 | JP2010510174 A | 02.04.2010 |
| | | US2009291884 A1 | 26.11.2009 |
| | | CA2668284 A1 | 08.05.2008 |
| | | AU2007314456 A1 | 08.05.2008 |
| | | EP2089420 A2 | 19.08.2009 |
| | | EP2089420 A4 | 27.04.2011 |
| EP3305803 A1 | 11.04.2018 | ES2791954T T3 | 06.11.2020 |
| | | JP2018525326 A | 06.09.2018 |
| | | JP6576471B B2 | 18.09.2019 |
| | | US2018148487 A1 | 31.05.2018 |
| | | US10435443 B2 | 08.10.2019 |
| | | CN107922460 A | 17.04.2018 |
| | | KR20160140031 A | 07.12.2016 |
| | | KR101745520B B1 | 12.06.2017 |
| | | WO2016195159 A1 | 08.12.2016 |

Form PCT/ISA/210 (patent family annex) (January 2015)

# INTERNATIONAL SEARCH REPORT

| International application No. |
| --- |
| PCT/CU2020/050006 |

## CLASSIFICATION OF SUBJECT MATTER

*A61K38/00* (2006.01)
*A61P25/00* (2006.01)
*A61P31/00* (2006.01)
*A61P37/00* (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2479815 A1
- WO 2011051535 A1
- CN 101693104 A
- WO 2010011315 A2
- CN 1814276
- WO 201303048226 A1
- JP H06107553 A
- US 6211155 B1
- RU 2017115294

- US 9192654 B
- WO 2019018445 A
- WO 2019129315 A1
- US 7018797 B
- RU 2588143 C2
- CN 109718363 A
- US 2015283113 A
- US 9192654 B2
- WO 2010011315 A

**Non-patent literature cited in the description**

- **JACOBSEN J.S. ; REINHART P. ; PANGALOS MN.** Current concepts in therapeutic strategies targeting cognitive decline and disease modification in Alzheimer's disease. *NeuroRx,* 2005, vol. 2 (4), 612-626

- **LOVELL MA ; XIE C ; GABBITA SP.** Markesbery WR. Decreased thioredoxin and increased thioredoxin reductase levels in Alzheimer's disease brain. *Free Radic Biol Med.,* 01 February 2000, vol. 28 (3), 418-27

- **AKTERIN S. ; COWBURN R. F. ; MIRANDA-VI-ZUETE A. ; JIMENEZ A. ; BOGDANOVIC N. ; WIN-BLAD B. ; CEDAZO-MINGUEZ A.** Involvement of glutaredoxin-1 and thioredoxin-1 in β-amyloid toxicity and Alzheimer's disease. *Cell Death & Differentiation,* 2006, vol. 13, 1454-1465

- **HERNANDEZ-ZIMBRON LF ; LUNA-MUÑOZ J ; MENA R ; VAZQUEZ-RAMIREZ R ; KUBLI-GARFI-AS C et al.** Amyloid-b Peptide Binds to Cytochrome C Oxidase Subunit 1. *PLoS ONE,* 19 September 2019, vol. 7 (8), e42344, https://www.ncbi.nlm.nih.gov/pmc/articles/PMC3424232

- **ADAV S.S. ; PARK J.E. ; SZE S.K.** Quantitative profiling brain proteomes revealed mitochondrial dysfunction in Alzheimer's disease. *Molecular Brain.,* 2019, vol. 12

- **LIVNEH I ; COHEN-KAPLAN V. ; COHEN-ROSEN-ZWEIG C ; AVNI N. ; CIECHANOVER A.** The life cycle of the 26S proteasome: from birth, through regulation and function, and onto its death. *Cell Research,* 2016, vol. 26, 869-885

- **TRAMUTOLA A. ; DI DOMENICO F. ; BARONE E. ; PERLUIGI M. ; BUTTERFIELD, D.A.** Oxid Med Cell Longev. It Is All about (U)biquitin: Role of Altered Ubiquitin-Proteasome System and UCHL1. *Alzheimer Disease,* 2016, vol. 2016, 2756068

- **HERRMANN J. ; LERMAN L.O. ; LERMAN A.** *Ubiquitin and Ubiquitin-Like Proteins in Protein Regulation,* 21 September 2019, https://doi.org/10.1161/01.RES.0000264500.11888.f0

- *Circulation Research,* 2007, vol. 100, 1276-1291

- **KOMATSU M ; CHIBA T ; TATSUMI K ; LEMURA S ; TANIDA I ; OKAZAKI N ; UENO T ; KOMINAMI E ; NATSUME T ; TANAKA K.** A novel protein-conjugating system for Ufml, a ubiquitin-fold modifier. *EMBO J.,* 08 April 2004, vol. 23 (9), 1977-86

- **IMYUNG-JIN et al.** Polymorphisms of small ubiquitin-related modifier genes are associated with risk of Alzheimer's disease in Korean: A case-control study. *Journal of the Neurological Sciences,* May 2016, vol. 364 (15), 122-127

- **ARANCIO O.** *Regulation of Synaptic Plasticity and Cognition by SUMO in Normal Physiology and Alzheimer's Disease,* 20 September 2019, http://columbianeuroresearch.org/taub/res-taub-connect-13-2014.html

- **BLAIR L.J. ; BAKER J.D. ; SABBAGH J.J. ; DICK-EY C.A.** The emerging role of peptidyl-prolyl isomerase chaperones in tau oligomerization, amyloid processing and Alzheimer's disease. *J Neurochem.,* April 2015, vol. 133 (1), 1-13

- **LXU L. ; REN Z. ; CHOW F.E. ; TSAI R. ; LIU T. ; RIZZOLIO F. ; BOFFO S. ; XU Y. ; HUANG S. ; LIP-PA C.F.** Pathological Role of Peptidyl-Prolyl Isomerase Pin1 in the Disruption of Synaptic Plasticity in Alzheimer's Disease. *J Neurochem.,* April 2015, vol. 133 (1), 1-13

- **AMIN F. ; BANO B.** *Putative Involvement of Thiol Protease Inhibitor in the Function of Alzheimer Drug*, 21 September 2019, https://www.intechopen.com/online-first/putative-involvement-of-thiol-protease-inhibitor-in-the-function-of-alzheimer-drug
- **WU Z. ; NI J ; LIU Y. ; TEELING JL. ; TAKAYAMA F. ; COLLCUTT A. ; IBBETT P. ; NAKANISHI H.** Cathepsin B plays a critical role in inducing Alzheimer's disease-like phenotypes following chronic systemic exposure to lipopolysaccharide from Porphyromonas gingivalis in mice. *Brain Behav. Immun.,* October 2017, vol. 65, 350-361
- **RICCIO M. ; DI GIAIMO R ; PIANETTI S. ; PALMIERI P.P ; MELLI M. ; SANTI S.** Nuclear Localization of Cystatin B, the Cathepsin Inhibitor Implicated in Myoclonus Epilepsy (EPM1). *Experimental Cell Research,* 15 January 2001, vol. 262 (2), 84-94
- **KENT Z. ; Q. WANG ; ERIN STEER ; P. ANTHONY OTERO ; NICHOLAS W. BATEMAN ; MARY HONGYING CHENG ; ANA LIGIA SCOTT ; CHRISTINE WU ; IVET BAHAR ; YU-TZU SHIH.** PINK1 Interacts with VCP/p97 and Activates PKA to Promote NSFL1C/p47 Phosphorylation and Dendritic Arborization in Neurons. *eNeuro,* 19 December 2018, vol. 5 (6, https://www.eneuro.org/content/5/6/ENEURO.0466-18.2018
- **VAITES L.P. ; HARPER W.** *Protein aggregates caught stalling,* 20 September 2019, https://www.savalnet.cl/cienciaymedicina/progresosmedicos/ agregados-proteicos-en-la-neurodegeneracion.html
- **DANTUMA N.P. ; HEINEN C. ; HOOGSTRATEN D.** The ubiquitin receptor Rad23: At the crossroads of nucleotide excision repair and proteasomal degradation. *DNA Repair,* 2009, vol. 8 (4), 449-460
- **MARTIN L.J.** DNA Damage and Repair: Relevance to Mechanisms of Neurodegeneration. *J Neuropathol Exp Neurol.,* 2008, vol. 67 (5
- **LUNIN S.M. ; NOVOSELOVA E.G.** Thymus hormones as prospective anti-inflammatory agents. *Expert Opinion on Therapeutic Targets,* 2010, vol. 14 (8), 775-86
- **D.MOIR R. ; LATHE R. ; TANZIR.T.** The antimicrobial protection hypothesis of Alzheimer's disease. *Alzheimer's & Dementia,* December 2018, vol. 14 (12), 1602-1614
- **SOSCIA SJ ; KIRBY JE ; WASHICOSKY KJ ; TUCKER SM ; INGELSSON M et al.** The Alzheimer's Disease-Associated Amyloid b-Protein Is an Antimicrobial Peptide. *PLoS ONE,* 2010, vol. 5 (3), e9505
- **FÜLÖP T ; ITZHAKI RF ; BALIN BJ ; MIKLOSSY J ; BARRON AE.** Role of Microbes in the Development of Alzheimer's Disease: State of the Art - An International Symposium. *2017 IAGG Congress in San Francisco. Front. Genet.,* 10 September 2018, https://doi.org/10.3389/fgene.2018.00362
- **HUNG Y.H. ; BUSH A.I. ; LA FONTAINE S.** Links between copper and cholesterol in Alzheimer's disease. *Front. Physiol.,* 16 May 2013, https://doi.org/10.3389/fphys.2013.00111
- **KELNE G.S. ; LEE M. ; CLARK M.E. ; MACIEJEWSKI D. ; MCGRATH D. ; RABIZADEH S. ; LYONS T. ; BREDESEN D. ; JENNER P. ; MAKI R.A.** The Copper Transport Protein Atox1 Promotes Neuronal Survival. *The Journal of Biological Chemistry,* 2000, vol. 275, 580-584
- **HATORI Y ; LUTSENKO S.** The Role of Copper Chaperone Atox1 in Coupling Redox Homeostasis to Intracellular Copper Distribution. *Antioxidants (Basel),* 27 July 2016, vol. 5 (3), 25
- **GREENOUGH M. A. ; A. RAMIREZ MUNOZ ; BUSHA A. I. ; OPAZO C. M.** Metallo-pathways to Alzheimer's disease: lessons from genetic disorders of copper trafficking. *Metallomics,* 2016, vol. 8, 831-839
- **CARLOS M.G.** *Elongation,* 27 September 2019, https://www.sebbm.es/ BioROM/contenido/av_bma/apuntes/T11/elonga.htm
- **FONTAINE, S.N. et al.** Cellular factors modulating the mechanism of tau protein aggregation. *Cell. Mol. LifeSci.,* 2015, vol. 72, 1863-1879
- **WIND M ; REINES D.** Transcription elongation factor SII. *Bioessays,* April 2000, vol. 22 (4), 327-336
- **TAN W. ; WANG W. ; MA Q.** Physiological and Pathological Function of Serine/Arginine-Rich Splicing Factor 4 and Related Diseases. *Biomed Res Int.,* 12 February 2018, vol. 2018, 3819719
- **XIAOMIN YIN X. ; JIN N. ; GU J. ; SHI J. ; ZHOU J. ; ZHOU J. ; GONG C-X. ; IQBAL K. ; GRUNDKE-IQBAL I. ; LIU F.** Phosphorylation-regulated Kinase 1A (Dyrk1A) Modulates Serine/Arginine-rich Protein 55 (SRp55)-promoted Tau Exon 10 Inclusion. *2012 The Journal of Biological Chemistry,* vol. 287, 30497-30506
- **CHARLEINE Z ; ANTHONY B ; BRICE D ; STEPHANE M ; EMELINE K ; GUY I ; GAELLE N ; JOHANN M ; NATHALIE C ; TANGUI M.** Time-Course and Regional Analyses of the Physiopathological Changes Induced after Cerebral Injection of an Amyloid _ Fragment in Rats. *The American Journal of Pathology,* 2011, vol. 179 (1), 315-334
- **TAL D.R. ; RÜB U ; ORANTES M ; BRAAK H.** Phases of A_-deposition in the human brain and its relevance for the development of AD. *NEUROLOGY,* 2002, vol. 58, 1791-1800
- **NEHA, RUPINDER ; K. SODHI ; AMTESHWAR S. JAGGI ; NIRMAL SINGH.** *Animal models of dementia and cognitive dysfunction,* 2014, http://dx.doi.org/10.1016/j.lfs.2014.05.017

- **OKAWA, O. ; N. OHISHI ; K. YAGI.** Assay of lipid peroxides in animal tissues by the thiobarbituric acid reaction. *Anal Biochem.,* 1979, vol. 95 (2), 351-58
- **TAL D.R. ; RÜB U ; ORANTES M ; BRAAK H.** Phases of A_-deposition in the human brain and its relevance for the development of AD. *NEUROLOGY,* 2002, vol. 58, 1791-1800
- **HIRAMITSU, M. ; O. TAKIGUCHI ; A. NISHIYAMA ; M. HIROMASA.** Cilostazol prevents amyloid β peptide induced memory impairment and oxidative stress in mice. *BJP,* 2010, vol. 161, 1899-1912
- **UWE, M. ; J. VON HAGEN.** Isolation of subcellular organelles and structures. *Methods Enz.,* 2009, vol. 463, 305-326
- **OKAWA, O. ; N. OHISHI ; K. YAGI.** *Assay of lipid peroxides in animal tissues by the thiobarbituric acid reaction,* 1979
- **YAGI.** Assay of lipid peroxides in animal tissues by the thiobarbituric acid reaction. *Anal Biochem.,* 1979, vol. 95 (2), 351-58
- **RESNICK, A.Z. ; L. PACKER.** Oxidative damage to proteins: Spectrophometric method for carbonyl assay. *Methods Enz.,* 1994, vol. 233, 357-63
- **MYHRE, O. ; H. UTKILEN ; N. DUALE ; G. BRUNBORG ; T. HOFER.** *Metal dyshomeostasis and Inflammation in Alzheimer's and Parkinson's diseases: Possible impact of environmental exposure,* 2013, http://dx.doi.org/10.1155/2013/726954
- **RAMIREZ-RAMIREZ, D. ; J. VALENTI-PEREZ ; M. GARCIA ; Z. BATISTA ; J. ESTRADA.** Oxidative stress in aged rats. *Finlay Journal,* 2013, vol. 3 (4
- **ARAKI, S. ; M. OKAZAKI ; S. GOTO.** Impaired lipid metabolism in aged mice as revealed by fasting-induced expression of apolipoprotein mRNAs in the liver and changes in serum lipids. *Gerontology,* 2004, vol. 50 (4), 206-215
- **D'HOOGE R. ; DE DEYN P.P.** Applications of the Morris water maze in the study of learning and memory. *Brain Research Reviews,* 2001, vol. 36, 60-90
- **KING-HIMMELREICH TS. ; MÖSER CV ; WOLTERS MC ; OLBRICH K ; GEISSLINGER G ; NIEDERBERGER E.** Age-Dependent Changes in the Inflammatory Nociceptive Behavior of Mice. *Int. J. Mol. Sci.,* 2015, vol. 16, 27508-27519
- **BERTOLLO CM ; OLIVEIRA AC ; ROCHA LT ; COSTA KA ; NASCIMENTO EB, JR. ; COELHO MM.** Characterization of the antinociceptive and anti-inflammatory activities of riboflavin in different experimental models. *Eur J Pharmacol,* 2006, vol. 547 (1-3), 184-191, https://www.ncbi.nlm.nih. gov/biosystems/1457777?Sel=geneid:820#show=genes2007
- **SWINGLE KF ; SHIDEMAN FE.** Phases of the inflammatory response to subcutaneous implantation of a cotton pellet and their modification by certain anti-inflammatory agents. *J PharmacolExpTher,* 1972, vol. 183 (1), 226-234
- **SPECTOR WG ; HEESOM N.** The production of granulomata by antigen-antibody complexes. *J Pathol,* 1969, vol. 98 (1), 31-39
- **SEN M. A ; LIEBERMAN M ; ALPERT S ; WEISSMAN IL ; SMALL M.** Differentiation of CD3 - 4- 8- Thymocytes in Short-Term Thymic Stromal Cell Culture. *J. Exp. Med,* 1992, vol. 176, 543-55
- **GALY A ; VERMA S ; BÁRCENA A ; SPITS H.** Precursors of CD3 + CD4 + CD8 + Cells in the Human Thymus Are Defined by Expression of CD34. Delineation of Early Events in Human Thymic Development. *J. Exp. Med,* 1992, vol. 178, 391-401
- **SCOTT HALE J ; BOURSALIAN TE ; TURK GL ; FINK PJ.** Thymic output in aged mice. *PNAS,* 2006, vol. 103 (2), 8447-8452
- **WEINBERGER B ; LAZUARDI L ; WEISKIRCHNER I ; KELLER M ; NEUNER C ; FISCHER KH et al.** Healthy aging and latentinfection with CMV lead to distinct changes in CD8+ and CD4+ T-cell subsets in the elderly. *Hum Immunol,* 2007, vol. 68 (2), 86-90
- **GARCIA B ; SAAVEDRA D ; LORENZO-LUACES P ; BADIA TJ ; LEONARD I ; MAZORRA Z et al.** Immunosenescence and gender: a study in healthy Cubans. *Immunity & Ageing,* 2013, vol. 10, 16
- **OVERGAARD N.H ; JUNF JW ; STEPTOE RJ ; WELLS JW.** CD4+/CD8+ doble-posiive T cells: more than just a development stage. *J Leucocyte Biol,* 2015, vol. 97, 31-37
- Desarrollo del linfocito y reordenamiento del gen del receptor para el antigeno. Inmunologia Celular y Molecular. ELSEVIER, 2018
- **NISHIMURA H ; HATTORI S ; ABE M ; UEDA G ; OKAMOTO H ; TSURUI H ; HIROSE S ; SHIRAI T.** Differential expression of three CD45 alternative structures on murine T cells: exon 6-dependent epitope as a marker for functional heterogeneity of CD4+ T cells. *Int Immunol.,* August 1992, vol. 4 (8), 923-30
- **GORONZY JJ ; LEE WW ; WEYAND CM.** Aging and T-cell diversity. *Exp. Gerontol,* 2007, vol. 42 (5), 400-406
- **LARBI A ; PAWELEC G ; WITKOWSKI JM ; SCHIPPER HM ; DERHOVANESSIAN E ; GOLDECK D.** Dramatic shifts in circulating CD4 but not CD8 T cell subsets in mild Alzheimer's disease. *J. Alzheimers Dis,* 2009, vol. 17 (1), 91-103
- **YAN J ; GREER JM ; HULL R ; O'SULLIVAN JD ; HENDERSON RD ; READ SJ ; MCCOMBE PA.** The effect of aging on human lymphocyte subsets: comparison of males and females. *Immun Ageing,* 2010, vol. 7, 4

- **WIKBY A ; JOHANSSON B ; OLSSON J ; LOFGREN S ; NILSSON BO ; FERGUSON F.** Expansions of peripheral blood CD8 T-lymphocyte subpopulations and an association with cytomegalovirus seropositivity in the elderly: the Swedish NONA immune study. *Exp Gerontol,* 2002, vol. 37 (2-3), 445-453
- **RODIG SJ1 ; SHAHSAFAEI A ; LI B ; DORFMAN DM.** The CD45 isoform B220 identifies select subsets of human B cells and B-cell lymphoproliferative disorders. *Hum Pathol.,* January 2005, vol. 36 (1), 51-7
- **OKA S ; MORI N ; MATSUYAMA S ; TAKAMORI Y ; KUBO K.** Presence of B220 within thymocytes and its expression on the cell surface during apoptosis. *Immunology,* 2000, vol. 100, 417-423
- **SANTOS-ARGUMEDO L.** Phenotypic and functional diversity of B lymphocytes. *Revista Alergia Mexico,* 2015, vol. 62, 302-311
- **MERINO MC ; GRUPPI A.** Origin and development of B1 lymphocytes A cell population involved in defense and autoimmunity. *MEDICINA (Buenos Aires),* 2006, vol. 66, 165-172